(19) **Europäisches Patentamt**
**European Patent Office**
**Office européen des brevets**

(11) **EP 2 062 982 A1**

(12) **DEMANDE DE BREVET EUROPEEN**

(43) Date de publication:
**27.05.2009 Bulletin 2009/22**

(51) Int Cl.:
*C12Q 1/68* (2006.01)

(21) Numéro de dépôt: **07291401.3**

(22) Date de dépôt: **26.11.2007**

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK RS**

(71) Demandeurs:
• **ImmunID**
**38100 Grenoble (FR)**
• **COMMISSARIAT A L'ENERGIE ATOMIQUE**
**75015 Paris (FR)**

(72) Inventeurs:
• **Pasqual, Nicolas**
**38100 Grenoble (FR)**
• **Weisbuch, Sébastien**
**38090 Villefontaine (FR)**

(74) Mandataire: **Marcadé, Véronique et al**
**Cabinet Ores**
**36, rue de St Pétersbourg**
**75008 Paris (FR)**

(54) **Procédé d'étude de la diversité combinatoire V(D)J**

(57) La présente invention est relative à une méthode d'analyse *in vitro* de la diversité du répertoire des lymphocytes T et/ou B d'un individu, par l'utilisation de PCR multiplexes, dont l'une au moins est une PCR multi-n-plexe avec n≥2, de telle manière que les amplicons correspondants au différents réarrangements uissent être distingués les uns des autres et que chaque réarrangement puisse être identifié. L'invention concerne également les applications de cette méthode, notamment dans le suivi d'un traitement ou dans le diagnostic et/ou le pronostic de certaines pathologies.

EP 2 062 982 A1

SEQUENCE LISTING

<110> IMMUNID
        COMMISSARIAT A L'ENERGIE ATOMIQUE
        PASQUAL, Nicolas
        WEISBUCH, Sébastien

<120> PROCEDE D'ETUDE DE LA DIVERSITE COMBINATOIRE V(D)J.

<130> VMAahF2110/1

<160> 7

<170> PatentIn version 3.3

<210> 1
<211> 21
<212> DNA
<213> Artificial

<220>
<223> amorce (antisens)

<400> 1
gtctcctctc ccgtgcagtc a                                               21

<210> 2
<211> 25
<212> DNA
<213> Artificial

<220>
<223> amorce (antisens)

<400> 2
ctgtctctgc aatgatgaaa tggcc                                           25

<210> 3
<211> 24
<212> DNA
<213> Artificial

<220>
<223> amorce (sens)

<400> 3
ctggatgcag acacaaagca aagc                                            24

<210> 4
<211> 27
<212> DNA
<213> Artificial

<220>
<223> amorce (sens)

```
<400>  4
aatgaagatg gaaggtttac agcacag                                    27


<210>  5
<211>  28
<212>  DNA
<213>  Artificial

<220>
<223>  amorce (sens)                                              .

<400>  5
acaaagaaga tggaaggttt acagcaca                                   28


<210>  6
<211>  27
<212>  DNA
<213>  Artificial

<220>
<223>  amorce (sens)

<400>  6
ctagagagag catcaaaggc ttcactg                                    27


<210>  7
<211>  27
<212>  DNA
<213>  Artificial

<220>
<223>  amorce (sens)

<400>  7
tggtgacagt agttacgggt ggagaag                                    27
```

**Description**

La présente invention concerne le domaine de l'immunologie. Plus précisément, la présente invention est relative à une méthode d'analyse *in vitro* de la diversité du répertoire des lymphocytes T et/ou B d'un individu et à ses applications, notamment dans le suivi d'un traitement ou dans le diagnostic et/ou le pronostic de certaines pathologies.

Un lymphocyte, pour être fonctionnel, doit avoir un système de reconnaissance spécifique de l'antigène. Ce paramètre est essentiel : il définit la fonction même de la cellule T ou B. Au cours des étapes précoces de la différenciation de lymphocytes T, les loci codant les chaînes clonotypiques du récepteur TCR subissent des réarrangements pour permettre l'expression d'un récepteur fonctionnel. De même, dans les lymphocytes B, les loci codant les chaînes des immunoglobulines (IG) subissent des réarrangements pour permettre l'expression d'une IG fonctionnelle.

Le mécanisme de réarrangement V(D)J est spécifique aux lymphocytes T et B. Les gènes V, D et J codant pour le TCR sont répartis sur de longues portions germinales au sein des différents loci du TCR. Pour donner une protéine, ces gènes doivent être associés en un exon par un processus de réarrangement des gènes, appelé recombinaison V(D)J. Le principe de la recombinaison est basé sur la reconnaissance de séquences RSS spécifiques des gènes V(D)J et l'excision de la région chromosomique intercalée entre les deux gènes réarrangés. Chaque gène V et J possède, à une de ses extrémités, une séquence signal de recombinaison (RSS). Quant aux gènes D, ils en possèdent aux deux extrémités. Les RSS sont des séquences reconnues par les enzymes spécifiques de la recombinase, RAG I et RAG II, exprimées spécifiquement dans les lymphocytes. Ces protéines sont les actrices principales du réarrangement. Une fois associées aux protéines HMG (*High mobility group*), les enzymes RAG reconnaissent le nonamère du RSS grâce à leur homéodomaine et induisent une coupure entre le segment de gène V, D, J et l'heptamère, de façon à générer une extrémité codante et une extrémité signal. L'achèvement d'un réarrangement est obtenu après ligation des deux extrémités codantes V et J. Cette étape est précédée par l'action de l'enzyme TdT et d'une nucléase au niveau de la jonction V-J. Une fois réarrangé, le gène néoformé est transcrit puis épissé en ARNm avant d'être traduit en protéine membranaire.

Un TCR donné reconnaît de façon spécifique un nombre limité de peptides antigéniques différents. De ce fait, un vaste répertoire de récepteurs est requis pour assurer la défense de l'individu contre les multiples infections qu'il est susceptible de rencontrer dans son environnement. Pour cela, le système immunitaire a développé un mécanisme d'assemblage d'un grand nombre de segments de gènes V, D, J positionnés de façon discontinue dans le génome. Ce mécanisme « d'assemblage » appelé recombinaison V(D)J est indépendant d'une cellule à l'autre et permet d'obtenir un seul « fragment » de gène codant pour le TCR. Ce système permet, avec un nombre modeste de gènes, de générer un grand nombre de récepteurs différents. Chaque cellule utilise une combinaison de segments géniques selon des règles précises et obtient une chaîne TCR potentiellement unique.

Quatre mécanismes majeurs contribuent à générer la diversité du répertoire : 1) une diversité combinatoire qui correspond à la première étape de réarrangement entre un segment V (un segment D) et un segment J ; 2) une diversité jonctionnelle, générée au niveau de la jonction entre les segments de gènes réarrangés ; 3) des hypermutations somatiques dans les gènes réarrangés V-J et V-D-J ; 4) une diversité d'appariement des hétérodimères protéiques TCR$\alpha$ x TCR$\beta$ ou TCR$\gamma$ x TCR$\delta$.

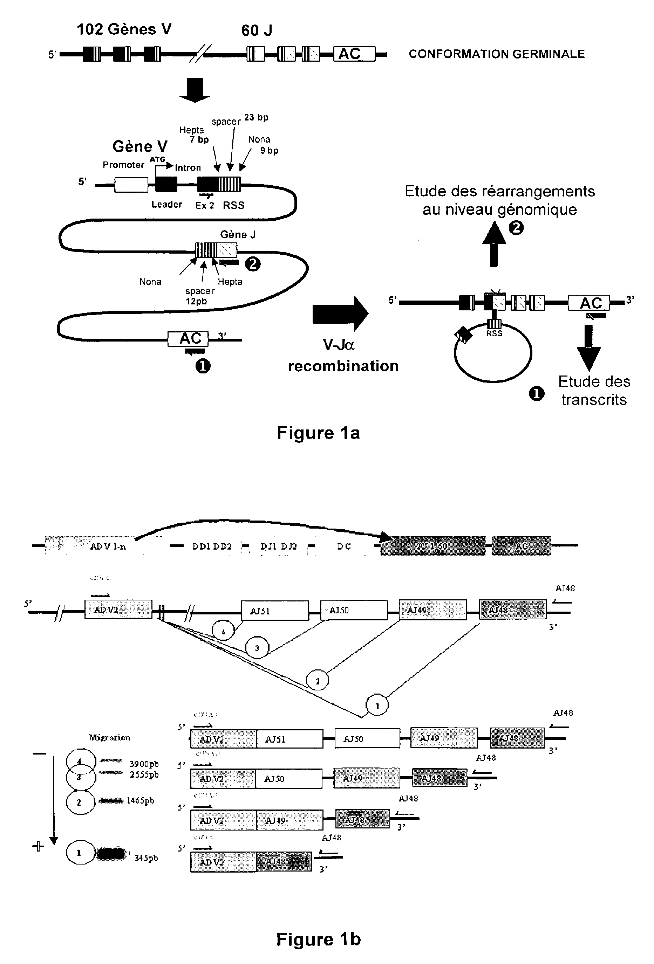

La première étape de génération de diversité est basée sur le principe du réarrangement des gènes V(D)J (figure 1). Les combinaisons résultantes de l'appariement d'un nombre fixe de gènes V, D et J forment la diversité combinatoire. Le calcul de cette diversité consiste à estimer le nombre de combinaisons mV x nD x pJ possibles. Le mécanisme régulant la recombinaison V(D)J n'est pas aléatoire : il est régulé de façon spatio-temporelle au cours de l'ontogénie (Aude-Garcia et al., 2001; Jouvin-Marche et al., 1998; Pasqual et al., 2002; Rytkonen et al., 1996). Une simple multiplication ne suffit donc pas à estimer le nombre total de combinaisons de gènes attendus. Cette première étape de génération de diversité définit l'ordre de grandeur du répertoire. En effet, même si cette étape ne génère qu'une modeste variabilité de combinaison (de l'ordre de quelques milliers de combinaisons possibles par rapport au répertoire maximal théorique estimé à $10^{15}$ (Davis and Bjorkman, 1988), la diversité combinatoire maximale est directement liée au nombre de gènes V, D et J initialement disponibles : les deux autres étapes de génération de la diversité amplifient de manière exponentielle la diversité du répertoire primaire.

La diversité de jonction permet de générer une très grande variabilité au niveau de la région CDR3 du récepteur en contact avec le peptide antigénique. Deux mécanismes contribuent à augmenter la diversité jonctionnelle : 1) le premier mécanisme est dû à l'ajout de nucléotides P (pour palindromiques), provenant de la résolution de l'épingle à cheveux des segments réarrangés (Fugmann et al., 2000). La diversité générée n'est pas aussi grande que celle provenant du deuxième mécanisme faisant intervenir l'enzyme terminal desoxynucléotidyl transférase ; 2) la TdT produit une importante diversité, en ajoutant de façon aléatoire des nucléotides N à l'extrémité 3' de chaque segment codant, sans besoin de matrice génomique (Bogue et al., 1992). Des études sur la souris TdT -/- ont permis d'estimer que, chez ces animaux, le répertoire TCR$\alpha\beta$ ne représentait que 5 à 10% du répertoire normal et donc que la TdT contribuait à hauteur de 90% à la génération de la diversité totale du TCR$\alpha\beta$. De plus, ces résultats ont montré que la longueur des CDR3 dans les transcrits TCR$\beta$ était nettement diminuée contrairement au CDR3 des transcrits TCR$\alpha$. Cette observation

vérifie, comme attendu, une contribution plus importante de la TdT sur les recombinaisons V-D-J que sur les réarrangements V-J (Cabaniols et al., 2001).

Le mécanisme des réarrangements secondaires contribue à « conserver » de la diversité : la diversité jonctionnelle représente le plus grand facteur d'amplification de la diversité du répertoire, mais si il n'y avait pas le mécanisme de réarrangement secondaire pour sauver 2/3 des thymocytes ayant interrompu leur cadre de lecture, ce bénéfice en terme de diversité représenterait un coût important pour l'organisme, avant même l'étape de sélection positive. Ces réarrangements non productifs ne peuvent donner une protéine TCR fonctionnelle. La cellule a alors la possibilité de tenter un deuxième réarrangement avec les gènes V(D)J encore disponibles sur le locus. La propriété d'ouverture concentrique du locus TRAD favorise ce processus en laissant le plus de chances possibles à la cellule, puisque les premiers réarrangements effectués par la cellule ont lieu entre un couple de gène V-J proches l'un de l'autre (Huang and Kanagawa, 2001; Pasqual et al., 2002; Wang et al., 1998). Si ces premiers réarrangements ne sont pas productifs, la cellule a la possibilité de tenter des réarrangements sur son second chromosome, ou encore d'utiliser les gènes V et J disponibles de part et d'autre du premier réarrangement. Ainsi, les réarrangements secondaires permettent la survie d'un grand nombre de cellules qui, à l'issue d'un premier réarrangement non productif, auraient dû être éliminées.

Les hypermutations somatiques (HMS) ont lieu pendant la différenciation des lymphocytes B dans les ganglions lymphatiques, lors de la rencontre avec un antigène (Berek et al., 1985). Les HMS sont situées dans des *"hot spot motifs"* des gènes réarrangés V-J et V(D)J des Ig (Chaudhuri et al., 2003; Oprea and Kepler, 1999) mais aussi dans certains cas, dans des gènes réarrangés V-J et V(D)J des TCR (Kotani et al., 2005). Le TCR peut être la cible d'HMS au niveau des gènes variables, si le lymphocyte surexprime l'enzyme AID *(Activation-induced cytidine deaminase)* qui est normalement spécifique des LB. En temps normal le TCR ne subit pas d'HMS car le LT ne synthétise tout simplement pas l'AID Néanmoins, si le lymphocyte T se met à l'exprimer, le TCR est aussi sensible à cette enzyme que le BCR car il possède tous la séquence sur laquelle elle agit. Au total, il est décrit dans la littérature que ce mécanisme induit une diversité supplémentaire d'un facteur 1000 dans le but d'augmenter les chances de reconnaître un antigène.

L'estimation de la diversité issue de l'appariement entre une chaîne TCRα et une chaîne TCRβ est obtenue en multipliant le nombre de combinaisons différentes d'une chaîne TCRα, par le nombre de combinaisons possibles pour la chaîne TCRβ. La diversité générée par ce mécanisme est directement dépendante du nombre de combinaisons primaires obtenu lors du réarrangement. En effet, si on examine le nombre de combinaisons primaires TCRγδ chez la souris, sans prendre en compte la diversité jonctionnelle, le résultat est seulement de 40 TCRδ (=10V*2D*2J) x 28 TCRγ (=7V*4J) = 1120 combinaisons différentes, alors que le même calcul conduit à $5,6 \cdot 10^6$ combinaisons pour TCRαβ (calculé comme suit: 102Vα*60Jα*33Vβ*2Dβ*14Jβ).

Le séquençage complet du génome humain et de la souris a récemment permis d'obtenir les cartes définitives de chacun des loci du TCR et rend dès lors possible de nouvelles approches génétiques pour découvrir les mécanismes de régulation de la recombinaison. Chaque cellule possède 4 loci capables de réarranger des gènes du TCR. Chez l'homme et la souris, les chaînes TCRα et TCRδsont réarrangées dans deux loci associés sur le même chromosome 14. Les loci TCRγ et TCRβ humains sont sur les chromosomes 7 (ou 13 chez la souris) et 7 (ou 6 chez la souris), respectivement (voir tableau 1).

Tableau 1 : Tableau comparatif des principales caractéristiques connues des 4 loci chez l'homme et la souris BALB/c. (D'après les données IMGT Lefranc MP).

| Chaîne | TCRα | | TCRδ | | TCRβ | | TCRγ | |
|---|---|---|---|---|---|---|---|---|
| Espèce | Humain | Souris | Humain | Souris | Humain | Souris | Humain | Souris |
| Chromosome | 14q11.2 | 14cl | 14q11.2 | 14cl | 7q34 | 6A-C | 7p14 | 13A2-3 |
| Taille du locus | 1000Kb | 1500Kb | 1000Kb | 1500Kb | 620Kb | 700Kb | 160Kb | 205Kb |
| Nombre de gènes **V** | 54 | 102 | 6+nVa | 10+ nVa | 63-67 | 33 | 12-15 | 7 |
| Nombre de familles **V** | 41 | 23 | 6+nVa | 10+ nVa | 30 | 30 | 6 | 7 |
| Nombre de gènes **D** | - | - | 3 | 2 | 2 | 2 | - | - |
| Nombre de gènes **J** | 61 | 60 | 4 | 2 | 6+7 | 7+7 | 5 | 4 |
| Nombre de gènes **C** | 1 | 1 | 1 | 1 | 2 | 2 | 2 | 4 |
| Nombre de pseudo V *transcription réarrangement* | 8 <br> - | 15 <br> - | 0 <br> 0 | 0 <br> 0 | 12-13 <br> - | 13 <br> - | | - <br> - |
| Nombre de pseudo J *transcription réarrangement (P-R)* | 8 <br> 8 | 6 <br> 16 | 0 <br> 0 | 0 <br> 0 | 2 <br> 2 | - <br> 2 | 0 <br> 0 | - <br> - |
| Combinatoire V (D)J maximale Sans les P-R | 54V*(61J -8 PR)= **2915** | 102V *(60J-16PR) = **4488** | 6V*3D*4 J= **72** | 10V*2D*2J= **40** | (67V*D1*6J1*2C )+(67*D1* (8J2-2PR) *C1+67*D2* (8J2-2PR)*C2= 804+402+402 = **1608** | (33V*D1*7J1-PR*2C)+(33*D1*(7 J2-1PR) *C1+33*D2*(7 J2-1PR)*C2= 396+198+198=**792** | 15*5= **75** | 7*4= **28** |

EP 2 062 982 A1

Remarque #1 : les tirés (-) indiquent l'absence d'études exhaustives permettant de dénombrer avec certitude le nombre de pseudogènes de réarrangement.

Remarque #2 : estimation des combinaisons TCRβ. D'après la structure du locus TCRβ chez l'homme, les gènes réarrangés sur le segment D1 peuvent se réarranger sur les segments J1.1-J1.6 puis sont épissés avec BC1 ou BC2. Ils peuvent également se réarranger sur le second set de segments J2.1-J2.8 mais sont alors uniquement épissés sur BC2. Si c'est le segment D2 qui est utilisé, seul le set J2.1 à J2.8 peut être utilisé et l'épissage se fera sur la chaîne BC2. Il en découle une combinatoire fractionnée en fonction du segment D1 ou D2 choisi lors du réarrangement D-J : (67V*1D1*6J1*2BC)+(67*1D1*(8J2-2PR)*1BC1 +67*1D2*(8J2-2PR)*1BC2= 1608 combinaisons possibles.

La nomenclature des gènes V(D)J a changé à plusieurs reprises pendant ces dernières années. Tout d'abord attribué selon leur ordre de découverte, le nom des gènes est maintenant défini en fonction de leur position dans le locus. La dernière nomenclature en date est celle d'IMGT (http://imgt.cines.fr), qui ordonne logiquement les familles V les unes par rapport aux autres et classe de manière intuitive les membres d'une famille sur le locus. Cette nomenclature IMGT sous entend que tous les gènes V soient connus. Le gène TRAV1 est le plus loin des segments AJ. C'est le gène le plus en amont (du coté 5') de la région V. Plus on se rapproche de la région J et plus le chiffre des familles V augmente : ainsi la famille TRAV41 humaine est la plus proche de la région AJ. Les membres d'une même famille ont un nom composé par le numéro de la famille, puis le numéro du membre. Par exemple TRAV8.1 est le membre le plus en amont (5') de la famille TRAV8 alors que le membre TRAV8.6 est le plus en aval (3') sur la région V. Dans ce qui suit, la nomenclature IMGT est utilisée.

La diversité du répertoire des immunoglobulines produites par les lymphocytes B résulte des mêmes mécanismes que ceux décrits ci-dessus pour les lymphocytes T.

La mesure de la diversité immunologique permet entre autres d'étudier les mécanismes de mise en place du répertoire immunitaire, l'homéostasie, les lymphocytes T ou B impliqués dans une réponse immunitaire, dans une leucémie ou encore d'évaluer l'immunodéficience induite par un traitement ou à l'inverse l'activation du système immunitaire spécifique. Cette liste n'est pas exhaustive.

L'étude du répertoire immunitaire d'une population lymphocytaire a conduit au développement de plusieurs approches multiparamétriques, permettant à la fois de mesurer le niveau de diversité et d'identifier la présence de certains clones T ou B spécifiques. Certaines approches mises au point par les immunologistes pour évaluer ces différents niveaux de diversité sont listées ci-dessous selon le principe et le « niveau » de diversité mesurée.

- **Mesure de la diversité V**
- Par cytométrie (Van den Beemd, van Dongen et al. 2000)
- Par Q-PCR au niveau génomique et transcriptomique (Fuschiotti et al., 2007; Lang et al., 1997; Pasqual et al., 2002).
- **Mesure de la diversité jonctionnelle CDR3 :**
- Par Immunoscope® (Cochet et al., 1992; Pannetier et al., 1995).
- Q-PCR couplé à l'immunoscope (TcLandscape®)
- Par séquençage
- Par la méthode Amplicot au niveau génomique (Baum and McCune, 2006).
- Par puce à ADN (Bonarius et al., 2006).
- **Etude des hypermutations somatiques (HMS) :**
- PCR / séquençage (Hamblin et al., 1999).
- **Mesure indirecte *via* la décroissance des cercles d'excisions TREC's**
- Par PCR (Douek et al., 1998).
- Par Q-PCR (Pham et al., 2003).

Si certaines de ces approches ont déjà fait leurs preuves en recherche fondamentale, notamment l'Immunoscope® (Pannetier, C., J. Even, et al., 1995) ou la cytométrie en flux (Van den Beemd, van Dongen et al., 2000), il reste encore un certain nombre de validations scientifiques et techniques à apporter pour évaluer la pertinence de son utilisation comme bio-marqueur médical. Face à la complexité du système immunitaire, le scientifique aurait besoin de coupler des approches technologiques complémentaires pour décrypter l'ensemble des informations contenues dans le répertoire immunitaire et relevantes d'une pathologie donnée.

D'autres méthodes, basées sur l'utilisation de PCR amplifiant spécifiquement des segments d'acides nucléiques caractéristiques de certains réarrangements, ont été décrites.

Par exemple, les brevets US 5,296,351 et US 5,418,134 présentent une méthode de détection des leucémies lymphoïdes ou des lymphomes B ou T, basée sur l'amplifications de séquences codant pour des immunoglobuline et/ou des récepteurs T, utilisant des amorces "consensus" pour amplifier simultanément plusieurs réarrangements V-J.

La demande WO2005/056828 décrit une méthode basée sur l'utilisation de PCR multiplexes, qui sont des réactions d'amplification en chaîne par polymérase (ACP, ou *Polymerase Chain Reaction, PCR)* dans lesquelles plusieurs fragments différents, correspondant à des réarrangements différents, sont amplifiés avec un même couple d'amorces, à partir d'ADN génomique.

Toutefois, aucune des méthodes décrites ci-dessus ne permet d'obtenir dans un temps raisonnable la résolution des réarrangements V(D)J du répertoire immunitaire, ni même sur l'ensemble des réarrangements intervenus dans un locus donné. En effet, le nombre de PCR multiplexes à effectuer pour cela serait trop élevé pour qu'une utilisation en routine de ces méthodes soit envisageable.

La présente invention est basée sur plusieurs perfectionnements de la méthode décrite dans la demande WO2005/056828, pour permettre l'analyse d'un plus grand nombre de réarrangements V(D)J de façon fiable, facile et rapide. De ce fait, les méthodes de la présente invention constituent des outils de choix pour analyser la diversité du répertoire des lymphocytes B et/ou T dans un échantillon dans de nombreuses applications, telles que le suivi immunitaire *(immunomonitoring)* en phases précliniques ou cliniques, pour étudier l'effet d'un traitement sur la (re)constitution du système immunitaire, le diagnostic personnalisé, le pronostic notamment en onco-hématologie, *etc.*

La présente invention porte donc en premier lieu sur une méthode d'analyse *in vitro* de la diversité du répertoire des lymphocytes T et/ou B d'un individu humain ou animal, à partir d'ADN génomique provenant d'un échantillon biologique dudit individu, comprenant les étapes suivantes :

A) amplification de fragments dudit ADN génomique par des PCR multiplexes, dont l'une au moins est une PCR multi-n-plexe avec n=2, effectuée avec une combinaison d'au moins 3 amorces, constituant au moins 2 couples d'amorces présentant les caractéristiques suivantes :

(i) chaque couple d'amorces est constitué d'une amorce s'hybridant spécifiquement en amont et/ou dans un gène V ou D donné et d'une amorce s'hybridant spécifiquement en aval et/ou dans un gène J donné, de façon à permettre l'amplification d'au moins deux fragments caractéristiques de deux réarrangements V-J ou D-J distincts;

(ii) les amorces sont thermodynamiquement compatibles ;

(iii) les amorces sont choisies de telle façon que les fragments amplifiés avec le premier couple d'amorces peuvent être distingués des fragments amplifiés avec le second couple d'amorces ;

B) détection des produits d'amplification obtenus à l'étape A;

C) interprétation des résultats.

Pour la mise en oeuvre de cette méthode, l'ADN génomique est de préférence purifié. Toutefois, l'homme du métier peut, en fonction de l'évolution des technologies, choisir de travailler sur des échantillons bruts. Tout échantillon biologique susceptible de contenir des lymphocytes peut être utilisé ; à titre d'exemples non limitatifs d'échantillons utilisables, on peut citer des échantillons de sang, thymus, ganglion, rate, PBMC, foie, peau, urine...

La mise en ouvre de la méthode ci-dessus nécessite une enzyme Taq polymérase de haute performance. Il est notamment préférable d'utiliser une enzyme ayant la capacité d'amplifier des fragments de haute taille à une vitesse élevée et pouvant "passer" sur des zones riches en GC. De façon encore préférée, l'homme du métier choisira, pour effectuer des réactions de PCR multi-n-plexes dans le cadre de la présente invention, une polymérase présentant les caractéristiques suivantes :

(i) elle est capable d'amplifier des fragments de plusieurs dizaines de kb ;

(ii) sa vitesse d'élongation est d'au moins 1 kb/minute ;

(iii) sa robustesse est telle qu'elle n'introduit pas plus d'une erreur par kb, en moyenne. A titre d'exemples non limitatifs d'enzymes utilisables pour mettre en oeuvre cette méthode, on peut citer les polymérases *Herculasell* de Stratagène et ou *Iproof de* Biorad

Dans le présent texte, on appelle "PCR multi-n-plexe" une réaction d'amplification en chaîne par polymérase présentant n degrés de multiplexage, c'est-à-dire mettant en oeuvre, dans une même réaction, n couples d'amorces différents, permettant chacun l'amplification d'au moins deux fragments d'ADN, caractéristiques d'au moins deux réarrangements chromosomiques différents. Par exemple, dans le locus TRA, une réaction de PCR multi-2-plexe peut être réalisée en utilisant une amorce "sens" s'hybridant spécifiquement en amont et/ou dans un gène $V_x$ donné et deux amorces "antisens" s'hybridant spécifiquement en aval et/ou dans deux gènes $J_y$ et $J_z$ distincts, permettant au moins l'amplification, en une seule réaction, de fragments caractéristiques des réarrangements $V_xJ_y$, $V_xJ_{y+1}$, $V_xJ_z$ et $V_xJ_{z+1}$. Bien entendu, une réaction de PCR multi-n-plexe n'est possible que si les amorces des n couples utilisés sont thermodynamiquement compatibles. La compatibilité thermodynamique des amorces est une notion bien connue de l'homme du métier, qui dispose d'algorithmes pour la vérifier. Elle implique notamment que les différentes amorces aient des températures de fusion (Tm) identiques ou proches. En outre, les différentes amorces ne doivent pas s'hybrider entre elles, ni former des épingles à cheveux. Dans la suite de ce texte, les contraintes liées à la compatibilité des amorces pour leur utilisation simultanée dans une PCR multi-n-plexe ne seront pas systématiquement rappelées, étant donné qu'elles font partie des connaissances générales de tout biologiste moléculaire.

Dans le présent texte, une "amorce s'hybridant spécifiquement en aval et/ou dans un gène J donné" pourra être appelée, par abus de langage, "amorce spécifique du gène J". De même, une "amorce s'hybridant spécifiquement en amont et/ou dans un gène V donné" pourra être appelée "amorce spécifique du gène V". S'agissant des gènes V, il est important de noter qu'ils sont, pour la plupart des loci, regroupés en familles, suivant leur degré d'homologie. Dans ce cas, une "amorce spécifique d'un gène V donné" peut en réalité désigner une amorce reconnaissant spécifiquement tous les membres ou certains membres d'une famille de gènes V. Des exemples d'amorces spécifiques d'une famille de gènes V sont présentés ci-après. Par ailleurs, lorsqu'un couple d'amorces permet de détecter les réarrangements de tous les membres ou de certains membres d'une même famille, ces réarrangements pourront être appelés par abus de langage "le réarrangement", "l'amplicon", ou "le produit de PCR".

La méthode ci-dessus peut en particulier être appliquée à l'analyse de la diversité combinatoire des réarrangements V-J d'au moins un locus génétique choisi parmi les loci TRA, TRB, TRG, TRD, IGH, IGK, IGL ...

Dans une mise en oeuvre particulière de la méthode selon l'invention, au moins une amorce de chaque couple d'amorces est marquée. Lorsqu'une amorce est commune à plusieurs couples d'amorces utilisés dans une même PCR multi-n-plexe, l'autre amorce de chaque couple sera préférentiellement marquée. Des marquages différents, permettant de distinguer les produits d'amplification par chacun des couples, peuvent être avantageusement utilisés. L'homme du métier dispose d'une grande variété de marqueurs utilisables pour marquer les amorces, parmi lesquels on peut citer les marqueurs colorimétriques, fluorescents, enzymatiques, radioactifs, la biotine, la streptavidine, etc.

Selon une mise en oeuvre particulière de la méthode décrite ci-dessus, l'étape B) comporte une étape de mesure en temps réel de l'amplification des fragments d'ADN ; l'interprétation des courbes d'amplification obtenues est alors effectuée de la façon suivante :

(i) si une ou quelques courbes, en nombre inférieur à la moitié des courbes et en particulier en nombre égal à 1, 2 ou 3, présentent un décalage par rapport aux autres courbes, tel que les autres courbes présentent un point d'inflexion au moins 2 cycles après le point d'inflexion de la première courbe, de préférence au moins 3 ou 4 cycles, ou ne montrent aucune amplification, le résultat indique la présence d'une lymphoprolifération clonale ou oligoclonale ;
(ii) si, au contraire, toutes les courbes présentent un point d'inflexion au même cycle, ou dans un décalage maximum de 2 ou 3 cycles d'amplification, le résultat permet d'écarter l'hypothèse d'une lymphoprolifération d'un clone résultant d'un des réarrangements correspondant aux fragments amplifiés.

Dans cette mise en oeuvre, et suivant la technologie utilisée pour réaliser la PCR quantitative (TaqMan®, mesure de l'incorporation d'un intercalant fluorescent tel que le SYBR-green®, ...), l'amplification mesurée est soit la somme des amplifications réalisées avec tous les couples d'amorces utilisés dans une même réaction (cas de l'utilisation du SYBR-green®), soit, au contraire, l'amplification par chacun des couples d'amorces, séparément (cas de la technologie TaqMan®, en utilisant des amorces marquées différemment pour chacun des couples). Quoi qu'il en soit, les inventeurs ont observé que lorsqu'une population lymphocytaire donnée est sur-représentée dans un échantillon, l'amplification du fragment correspondant au réarrangement intervenu dans ces lymphocytes est efficace, alors que l'amplification des fragments correspondant aux autres réarrangements conduit à une courbe décalée, voire à un défaut d'amplification (courbe plate).

Lorsque la mesure de l'amplification est effectuée avec un intercalant fluorescent, la méthode décrite ci-dessus peut également comporter, une étape (optionnelle) de confirmation d'une lymphoprolifération, par la mesure continue de la fluorescence dans chaque tube au cours d'une montée en température entre 40°C et 95°C, l'observation d'un pic majoritaire étant indicative de la présence d'un amplicon majoritaire et donc d'une lymphoprolifération, alors que l'observation de plusieurs pics de tailles similaires indique au contraire une diversité lymphocytaire.

Cette méthode permet également d'effectuer une étape de mesure de la diversité moléculaire des réarrangements observés au niveau de l'ADN génomique, par mesure de la diversité moléculaire des amplicons. Cette étape optionnelle ajoute un degré d'information supplémentaire, car la "diversité moléculaire" résulte de la combinaison de la diversité de jonction (CDR3), la diversité combinatoire (V-J) et la diversité issue des hypermutations somatiques. Elle est mesurée, selon l'invention, de la façon suivante :

(i) après déshybridation des amplicons à 95°C, la température des produits d'amplification est rapidement ramenée en dessous de 40°C, de préférence à 30°C ou en dessous ; cette baisse de température peut être effectuée dans le thermocycleur, ou en mettant le tube dans la glace pendant quelques minutes ; la chute de température doit être effectuée en un temps réduit, de préférence inférieur à 2 minutes, de façon encore préférée inférieur à 30 secondes.
(ii) la fluorescence est mesurée régulièrement et de préférence en continu au cours de la réhybridation ;
(iii) une réhybridation rapide (de l'ordre de la seconde) est indicative de la présence d'un amplicon majoritaire, et donc d'une lymphoprolifération clonale, alors qu'une réhybridation plus lente (de l'ordre de plusieurs dizaines de secondes, voire minutes) est indicative d'une bonne diversité moléculaire (au moins plusieurs dizaines de molécules, voire plusieurs milliers).

Selon une autre mise en oeuvre préférée de la méthode de l'invention, l'étape B) de détection des produits d'amplification comprend une étape de séparation desdits produits suivant leur taille. L'homme du métier dispose de plusieurs techniques permettant la séparation des amplicons suivant leur taille. A titre d'exemples non limitatifs, on peut citer l'électrophorèse sur gel d'agarose ou de polyacrylamide, ou l'électrophorèse capillaire, qui présente l'avantage d'être plus facilement automatisable. Les amplicons séparés suivant leur taille peuvent être détectés par tout moyen connu de l'homme du métier, notamment grâce à des marqueurs liés aux amorces, ou en utilisant des intercalants fluorescents tels que le bromure d'éthydium, le SYBR-green®, etc. La résolution des amplicons et leur détection permettent d'identifier les différents réarrangements chromosomiques ayant donné lieu à amplification. L'utilisation d'une technique d'amplification semiquantitative ou quantitative permet en outre de déterminer la fréquence, dans l'échantillon testé, des lymphocytes ayant subi le réarrangement V(D)J correspondant à chacun des amplicons observés. Cette mise en oeuvre de l'invention permet donc d'évaluer finement la diversité combinatoire d'une partie du répertoire immunitaire, cette partie étant d'autant plus grande que le nombre de réarrangements susceptibles de donner lieu à amplification est important, ce dernier paramètre dépendant du nombre de PCR multiplexes réalisées, de leur degré de multiplexage, et du choix des amorces.

L'homme du métier pourra décider de mettre en oeuvre la méthode de l'invention en effectuant une première évaluation de la diversité du répertoire lymphocytaire par simple mesure en temps réel de l'amplification des fragments d'ADN, comme mentionné plus haut, et d'effectuer ensuite une séparation des amplicons suivant leur taille pour obtenir davantage d'informations sur la distribution des différents réarrangements dans la population lymphocytaire. Dans le cas où une lymphoprolifération est détectée par PCR multi-n-plexe quantitative, la deuxième étape de détection, par séparation des amplicons suivant leur taille, permettrait en particulier d'identifier le réarrangement V(D)J présent dans le clone ou les quelques clones prolifératif(s). Il est important de noter que cette démarche ne nécessite pas d'effectuer des PCR supplémentaires, puisqu'il suffit alors d'utiliser les produits des PCR déjà effectuées, pour en séparer les amplicons suivant leur taille. Alternativement, l'homme du métier peut choisir de ne pas effectuer la première étape de recherche d'une lymphoprolifération par PCR en temps réel, et d'effectuer directement la séparation des produits d'amplification. Il obtiendra alors directement le nom et la fréquence (intensité) des différents réarrangements V(D)J ayant eu lieu dans la population lymphocytaire testée.

Dans cette mise en oeuvre de l'invention, les couples d'amorces utilisés en combinaison dans chaque réaction de PCR multi-n-plexe avec n≥2 sont de préférence choisis de façon à ce que la majorité des amplicons obtenus puissent être résolus suivant leur taille. Par "résolus", on entend ici que chacun des amplicons puisse être observé de façon univoque, suite à leur séparation par la taille par une méthode d'électrophorèse ou toute autre méthode. Lorsque des tailles d'amplicons sont trop proches, il n'est pas toujours possible de les discerner distinctement avec des conditions de séparation compatibles avec les conditions permettant de "résoudre" l'ensemble des autres tailles d'amplicons. Les différents amplicons obtenus avec un couple d'amorces donné correspondent à des réarrangements différents et sont donc de tailles significativement différentes. Il faut donc veiller à ce qu'au moins certains des produits de l'amplification par un couple d'amorces présentent des tailles différentes des produits de l'amplification par l'autre ou les autres couple(s) d'amorces. Bien entendu, les différences de tailles pour obtenir une bonne résolution des bandes dépendent de la technologie utilisée. A titre indicatif, des différences de taille de 10% permettent en général d'obtenir une bonne résolution. Idéalement, les amorces sont choisies pour permettre la résolution de tous les amplicons. Toutefois, si certaines bandes, correspondant à des amplicons obtenus avec différents couples d'amorces dans une même PCR multi-n-plexe, sont trop proches ou se chevauchent, d'autres moyens peuvent être mis en oeuvre pour identifier et quantifier les réarrangements correspondant. Par exemple, des marqueurs distincts peuvent être liés aux amorces correspondantes (au moins à une amorce de chaque couple concerné).

Dans une mise en oeuvre particulière de l'invention, au moins une PCR multi-n-plexe (avec n≥2) est effectuée en utilisant une combinaison d'au moins 3 amorces constituant au moins 2 couples d'amorces comportant une amorce "sens" commune spécifique d'un gène V donné, chaque couple d'amorces comportant en outre une amorce "antisens" spécifique d'un gène J donné. Plus particulièrement, cette méthode peut être avantageusement mise en oeuvre en effectuant plusieurs PCR multi-2-plexes avec des triplets d'amorces constitués chacun d'une amorce sens s'hybridant spécifiquement en amont et/ou dans un gène V donné et de deux amorces antisens s'hybridant spécifiquement en aval et/ou dans deux gènes J distincts.

Une manière alternative de combiner les amorces pour effectuer les PCR multi-n-plexes selon l'invention est de combiner au moins 3 amorces constituant au moins 2 couples d'amorces comportant une amorce antisens commune spécifique d'un gène J donné, chaque couple d'amorces comportant en outre une amorce sens spécifique d'un gène V donné.

Le locus TRB présente une configuration particulière, puisque les gènes J sont disposés en deux groupes (ou *clusters*) éloignés l'un de l'autre, le premier groupe (dans le sens 5' -> 3') comportant les gènes BJ1.1 à BJ1.6, et le second groupe comportant les gènes BJ2.1 à BJ2.7. Tirant parti de cette configuration particulière, les inventeurs ont déterminé des paramètres de choix des amorces pour obtenir une excellente résolution des amplicons correspondant aux réarrangement d'un gène V donné avec l'ensemble ou une partie des gènes J. Selon cette mise en oeuvre particulière de l'invention, au moins une réaction de PCR multi-n-plexe avec $n \geq 2$ est effectuée pour analyser certains réarrangements du locus TRB, en utilisant une combinaison d'au moins 3 amorces constituant au moins 2 couples d'amorces présentant les caractéristiques suivantes :

(i) les deux couples d'amorces comportent une amorce sens commune s'hybridant spécifiquement en amont et/ou dans un gène V donné et comportent chacun une amorce antisens s'hybridant spécifiquement en aval et/ou dans un gène J donné ;

(ii) les deux amorces antisens s'hybrident spécifiquement en aval et/ou dans deux gènes $J_y$ et $J_z$ appartenant à deux groupes distincts de gènes J du locus TRB ; et

(iii) la distance entre la région d'hybridation de l'amorce antisens spécifique du gène $J_y$ et le début dudit gène $J_y$ est supérieure à la distance entre la région d'hybridation de l'amorce antisens spécifique du gène $J_z$ et le début du premier gène J du groupe de gènes dudit gène $J_z$.

Dans ce qui précède, la "distance entre la région d'hybridation de l'amorce antisens spécifique du gène $J_y$ et le début dudit gène $J_y$" désigne la distance entre l'extrémité 3' de ladite région d'hybridation (correspondant donc, dans l'amplicon, à l'extrémité 5' de l'amorce antisens spécifique du gène $J_y$) et la première base codante du gène $J_y$ (située immédiatement après la séquence de recombinaison RSS).

Dans une mise en oeuvre préférée de cet aspect de l'invention, $J_y = J_{1.6}$ et $J_z = J_{2.7}$. Si $V = V_x$, on verra donc, dans l'ordre croissant de taille, les bandes caractéristiques des réarrangements $V_x J_{2.7}$, $V_x J_{2.6}$, $V_x J_{2.5}$, $V_x J_{2.4}$, $V_x J_{2.3}$, $V_x J_{2.2}$, $V_x J_{2.1}$, $V_x J_{1.6}$, $V_x J_{1.5}$, $V_x J_{1.4}$, $V_x J_{1.3}$, $V_x J_{1.2}$ et $V_x J_{1.1}$. Le cas échéant, si la polymérase utilisée est particulièrement performante, on observera également une bande de haut poids moléculaire correspondant aux réarrangements $V_x J_{1.n}$ amplifiés avec l'amorce s'hybridant en aval du gène $J_{2.7}$. Alternativement, la méthode peut être mise en oeuvre avec $J_y = J_{2.7}$ et $J_z = J_{1.6}$ ce qui conduira à observer, dans l'ordre croissant, les bandes caractéristiques des réarrangements $V_x J_{1.6}$, $V_x J_{1.5}$, $V_x J_{1.4}$, $V_x J_{1.3}$, $V_x J_{1.2}$, $V_x J_{1.1}$, $V_x J_{2.7}$, $V_x J_{2.6}$, $V_x J_{2.5}$, $V_x J_{2.4}$, $V_x J_{2.3}$, $V_x J_{2.2}$ et $V_x J_{2.1}$.

Selon une mise en oeuvre préférée de la méthode décrite ci-dessus pour analyser certains réarrangements du locus TRB, au moins une réaction de PCR multi-n-plexe avec $n = 2$ est effectuée en utilisant une combinaison d'au moins 3 amorces comprenant les amorces hTRBJ1.6 et hTRJB2.7 définies de la façon suivante :

- hTRBJ1.6 est un oligonucléotide antisens de 25 nucléotides s'hybridant entre les nucléotides 2341 et 2365 du gène J1.6 du locus TCRB ; et
- hTRBJ2.7 est un oligonucléotide antisens de 22 nucléotides s'hybridant entre les nucléotides 214 et 235 du gène J2.7 du locus TCRB.

Une méthode pour déterminer la séquence des différentes amorces décrites dans le présent texte par leur position par rapport à différents gènes des loci TCR ou IgH est expliquée dans l'exemple 1 ci-après. En particulier, les séquences génomiques divulguées dans la base de données *"Ensembl Genome Browser"* peuvent être utilisées pour identifier les amorces utilisables dans le cadre de la présente invention.

Les inventeurs ont identifié, dans le locus TRB, 24 familles de gènes V fonctionnels. L'invention concerne donc plus particulièrement une méthode permettant l'analyse des réarrangements V(D)J du locus TRB en effectuant 24 PCR multi-n-plexes avec $n = 2$ à l'aide de combinaisons d'au moins 3 amorces, chaque combinaison d'amorces comprenant les amorces hTRBJ1.6, hTRBJ2.7 définies à la revendication 13 et au moins une amorce hTRBV choisie parmi les amorces définies dans le tableau ci-dessous :

Tableau 2

| Nom du gène | Nom de l'amorce | Taille (nt) | Distance entre l'extrémité 5' de l'amorce et la fin du gène V (pb) |
|---|---|---|---|
| TRBV2 | hTRBV2up2 | 26 | 255 |
| TRBV3 | hTRBV3up2 | 23 | 57 |
| TRBV4 | hTRBV4up_ex | 23 | 100 |
| TRBV5.1, 3, 4, 5, 6, | hTRBV5up_ex1/2 8 | 25 | 256 |
| TRBV5.7 | hTRBV5up_ex2/2 | 25 | 256 |

(suite)

| Nom du gène | Nom de l'amorce | Taille (nt) | Distance entre l'extrémité 5' de l'amorce et la fin du gène V (pb) |
|---|---|---|---|
| TRBV6.1, 2, 3, 5, 6,7,8,9 | hTRBV6up_ex1/2 | 25 | 281 |
| TRBV6.4 | hTRBV6up_ex2/2 | 23 | 279 |
| TRBV7.1,3,4,5,6,7,8 | hTRBV7up_ex1/3 | 25 | 301 |
| TRBV7.2 | hTRBV7up_ex2/3 | 25 | 301 |
| TRBV7.9 | hTRBV7up_ex3/3 | 27 | 303 |
| TRBV9 | hTRBV9up_ex | 23 | 92 |
| TRBV10.1,3 | hTRBV10.1,3up1 | 26 | 162 |
| TCRBV10.2 | hTRBV10.2up1 | 27 | 162 |
| TRBV11 | hTRBV11up_ex | 27 | 120 |
| TRBV12.3,4,5 | hTRBV12.3,4,5up1 | 26 | 195 |
| TRBV12.1 | hTRBV12.1up 1 | 27 | 196 |
| TRBV12.2 | hTRBV12.2up1 | 27 | 196 |
| TRBV13 | hTRBV13up1 | 25 | 356 |
| TRBV14 | hTRBV14up_ex | 24 | 271 |
| TRBV15 | hTRBV15up_ex | 24 | 163 |
| TRBV16 | hTRBV16up1 | 22 | 295 |
| TRBV18 | hTRBV18up1 | 22 | 46 |
| TRBV19 | hTRBV19 up 2 | 24 | 217 |
| TRBV20 | hTRBV20-1up_ex | 24 | 91 |
| TRBV24 | hTRBV24up_ex | 24 | 96 |
| TRBV25 | hTRBV25up_int | 23 | 273 |
| TRBV27 | hTRBV27up2 | 22 | 312 |
| TRBV28 | hTRBV28up_G | 24 | 71 |
| TRBV29 | hTRBV29up_G | 21 | 91 |
| TRBV30 | hTRBV30up1 | 26 | 148 |

Cette mise en oeuvre de l'invention permet un analyse "complète" des réarrangements V(D)J du locus TRB, c'est-à-dire la détermination de la fréquence d'utilisation des gènes de chaque famille V fonctionnelle avec chaque famille J fonctionnelle de ce locus (sans information sur la nature du gène D utilisé dans ces réarrangements, ni sur l'utilisation de chaque membre d'une famille V donnée, ni sur la diversité jonctionnelle des réarrangements, etc.). Le couplage avec une mesure en temps réel de l'amplificationpar PCR multi-n-plexe permet en outre d'estimer la diversité moléculaire.

Selon une mise en oeuvre particulière de la méthode de l'invention, cette méthode permet la détection *in vitro* de réarrangements incomplets D-J dans un locus génétique choisi parmi les loci TRB, et IGH. La détection des réarrangements incomplets est importante, car même s'ils sont non fonctionnels, ils constituent dans certains cas la seule signature d'une population lymphoproliférative.

Cette méthode peut en particulier être adaptée pour analyser des réarrangements incomplets DJ du locus TRB humain, en effectuant au moins une réaction de PCR multi-n-plexe avec n≥2 avec une combinaison d'au moins 3 amorces constituant au moins 2 couples d'amorces présentant les caractéristiques suivantes :

(i) les deux couples d'amorces comportent une amorce sens commune s'hybridant spécifiquement en amont et/ou dans un gène D donné et comportent chacun une amorce antisens s'hybridant spécifiquement en aval et/ou dans un gène J donné ;

(ii) les deux amorces antisens s'hybrident spécifiquement en aval et/ou dans deux gènes $J_y$ et $J_z$ appartenant à deux groupes distincts de gènes J du locus TRB ; et

(iii) la distance entre la région d'hybridation de l'amorce antisens spécifique du gène $J_y$ et le début dudit gène $J_y$ est supérieure à la distance entre la région d'hybridation de l'amorce antisens spécifique du gène $J_z$ et le début du premier gène J du groupe de gènes dudit gène $J_z$.

En particulier, les amorces hTRBJ1.6 et hTRJB2.7 définies plus haut peuvent être combinées à une amorce sens s'hybridant spécifiquement en amont et/ou dans un gène D donné pour effectuer une réaction de PCR multi-n-plexe avec n≥2.

Du fait de la configuration du locus TRB, il est possible d'analyser tous les réarrangements incomplets DJ de ce locus avec seulement 2 PCR multi-2-plexes.

Ainsi, il est possible d'analyser tous les réarrangements incomplets du locus TRB humain en effectuant (i) une PCR multi-2-plexe à l'aide d'un triplet d'amorces constitué des amorces hTRBJ1.6, hTRJB2.7 et d'une amorce hTRDB1, et (ii) une PCR multiplexe simple à l'aide du couple d'amorces constitué des amorces hTRJB2.7 et d'une amorce hTRDB2, en choisissant par exemple les amorces hTRDB1 et hTRDB2 parmi les amorces définies dans le tableau ci-dessous :

Tableau 3

| nom du gène | nom oligonucléotide | taille (nt) | Distance entre l'extrémité 5' de l'amorce et la fin du gène D (pb) |
|---|---|---|---|
| TRBD1 | hTRBD1up1 | 25 | 325 |
| TRBD1 | hTRBD1up2 | 23 | 289 |
| TRBD2 | hTRBD2up1 | 26 | 322 |
| TRBD2 | hTRBD2up2 | 21 | 290 |

Lorsque l'analyse des réarrangements incomplets du locus TRB est couplée à l'analyse d'autres réarrangements, les PCR multiplexes décrites ci-dessus, en particulier la PCR multiplexe simple, peuvent être combinées à d'autres réactions d'amplification, effectuées dans un même tube (augmentant ainsi le degré de multiplexage et diminuant le nombre de réactions nécessaires pour analyser un nombre de réarrangements donné.

Selon une mise en oeuvre préférée de l'invention, la méthode combine l'analyse des réarrangements V(D)J du locus TRB et celle des réarrangements incomplets de ce locus, par la mise en oeuvre des variantes appropriées décrites plus haut. Les amorces décrites plus haut pour cette analyse sont adaptées à l'analyse des réarrangements de ce locus chez l'humain, mais cette méthode peut être transposée, sans aucune difficulté, chez l'animal, par exemple chez la souris. Des amorces utilisables chez la souris sont décrites, à titre d'exemple, dans la partie expérimentale qui suit.

Selon un autre aspect de l'invention, la méthode permet l'analyse des réarrangements de 95% des gènes J du locus TRA humain avec un gène V donné du même locus, en effectuant, à l'étape A), entre 3 et 6 PCR multi-n-plexes avec n≥2, avec des combinaisons d'amorces constituées chacune d'une amorce s'hybridant en amont et/ou dans ledit gène V et d'un ou deux couple(s) d'amorces antisens choisi(s) parmi les couples (hTRAJ56, hTRAJ41), (hTRAJ37, hTRAJ33), (hTRAJ48, hTRAJ29), (hTRAJ24, hTRAJ18), (hTRAJ53, hTRAJ11) et (hTRAJ7, hTRAJ3), lesdites amorces étant définies dans le tableau ci-dessous :

Tableau 4

| nom du gène | nom oligonucléotide | taille (nt) | distance avec le début du gène J en pb |
|---|---|---|---|
| hTRAJ56 | hTRAJ56do | 24 | 883 |
| hTRAJ41 | hTRAJ41 do | 25 | 443 |
| hTRAJ37 | hTRAJ37do | 28 | 351 |
| hTRAJ33 | hTRAJ33do | 24 | 98 |
| hTRAJ48 | hTRAJ48do | 28 | 43 |
| hTRAJ29 | hTRAJ29do | 24 | 300 |
| hTRAJ24 | hTRAJ24do | 28 | 227 |
| hTRAJ18 | hTRAJ18do | 29 | 147 |

(suite)

| nom du gène | nom oligonucléotide | taille (nt) | distance avec le début du gène J en pb |
|---|---|---|---|
| hTRAJ53 | hTRAJ53do | 24 | 200 |
| hTRAJ11 | hTRAJ11do | 20 | 88 |
| hTRAJ7 | hTRAJ7do | 20 | 478 |
| hTRAJ3 | hTRAJ3do | 25 | 329 |

Cette analyse peut en particulier être réalisée à partir de 6 PCR multi-2-plexes. Alternativement, elle peut être réalisée à partir de 3 PCR multi-4-plexes effectuées avec des combinaisons d'amorces comportant chacune une amorce sens spécifique d'un gène V et un quadruplet d'amorces choisi parmi les quadruplets suivants: (hTRAJ56, hTRAJ41, hTRAJ37, hTRAJ33), (hTRAJ48, hTRAJ29, hTRAJ24, hTRAJ18) et (hTRAJ53, hTRAJ11, hTRAJ7, hTRAJ3). Bien entendu, les situations intermédiaires (4 PCR multi-2-plexes et 1 PCR multi-4-plexe ; 2 PCR multi-2-plexes et 2 PCR multi-4-plexes) sont également envisagées.

Afin d'avoir une information plus globale sur les réarrangements du locus TRA, il est proposé d'effectuer la méthode décrite ci-dessus avec au moins trois, mais de préférence 4, 5, 6 ou davantage d'amorces s'hybridant en amont et/ou dans des gènes V distincts, situés chacun dans une région distincte du locus. Il est important que les gènes TRAV visés par ces amorces soient bien répartis dans le locus, afin que la diversité combinatoire observée soit effectivement représentative de l'ensemble des réarrangements de ce locus. Des amorces utilisables pour cela sont définies dans le tableau ci-dessous :

Tableau 5

| nom du gène nom de | l'oligonucléotide | taille (pb) | distance avec la fin du gène V (pb) |
|---|---|---|---|
| TRAV1 | hTRAV1up_ex | 26 | 104 |
| TRAV2 | hTRAV2up_ex | 24 | 70 |
| TRAV3 | hTRAV3 | 22 | 377 |
| TRAV4 | hTRAV4up_ex_testAn1 | 23 | 96 |
| TRAV8 | hTRAV8-2,4,6 up | 22 | 298 |
| TRAV9 | hTRAV9-1up_ex_2/2_testAn2 | 25 | 113 |
| TRAV10 | hTRAV10 up n3 | 24 | 85 |
| TRAV12.2,3 | hTRAV12.2,3up1 | 27 | 114 |
| TRAV12.1 | hTRAV12.1up1 | 28 | 112 |
| TRAV14 | hTRAV14 up n2 | 22 | 69 |
| TRAV16 | hTRAV16 up n5 | 27 | 118 |
| TRAV17 | hTRAV17 up n2 | 22 | 40 |
| TRAV19 | hTRAV19 up | 24 | 144 |
| TRAV21 | hTRAV21 up | 24 | 91 |
| TRAV22 | hTRAV22 up | 21 | 42 |
| TRAV23 | hTRAV23 up n2 | 28 | 130 |
| TRAV25 | hTRAV25 up n3 | 27 | 154 |
| TRAV27 | hTRAV27 up | 27 | 138 |
| TRAV29 | hTRAV29 up | 24 | 267 |
| TRAV30 | hTRAV30 up n2 | 24 | 139 |
| TRAV35 | hTRAV35_int up | 27 | 377 |
| TRAV36 | hTRAV36_int up n2 | 27 | 304 |

(suite)

| nom du gène nom de | l'oligonucléotide | taille (pb) | distance avec la fin du gène V (pb) |
|---|---|---|---|
| TRAV38 | hTRAV38-1,2 up n3 | 24 | 170/169 |
| TRAV40 | hTRAV40 up | 28 | 76 |
| TRAV41 | hTRAV41_int up | 28 | 368 |

La mise en oeuvre de la méthode en effectuant, pour chacune des 25 amorces TRAV décrites dans ce tableau, 6 PCR multi-2-plexes (ou 3 PCR multi-4-plexes etc.) avec les combinaisons décrites plus haut pour observer les réarrangements de 95% des gènes TRAJ avec un gène V, permet d'observer entre 80 et 85% de la totalité des réarrangements VJ du locus TRA.

Bien entendu, l'homme du métier peut transposer cette méthode à l'analyse du locus TRA d'un animal, par exemple au locus TRA murin.

Selon un autre aspect, l'invention concerne une méthode permettant l'analyse des réarrangements de tous les gènes J du locus TRG humain avec au moins 2 gènes $V_x$ et $V_y$ donnés du même locus, en effectuant, à l'étape A), au moins une PCR multi-2-plexe avec un triplet d'amorces constitué de 2 amorces sens s'hybridant en amont et/ou dans lesdits gènes $V_x$ et $V_y$ et de l'amorce antisens hTRGJdo2 de 26 nucléotides s'hybridant entre les nucléotides 266 et 291 du gène J2 du locus TRG humain.

A titre d'amorces s'hybridant en amont et/ou dans un gène V du locus TRG humain utilisables pour la mise en oeuvre de cet aspect de l'invention, on peut citer les amorces définies dans le tableau ci-dessous :

Tableau 6

| nom du gène | nom oligonucléotide | taille (nt) | distance avec la fin du gène V en pb |
|---|---|---|---|
| TRGV1.2 | hTRGV1.2up1 | 30 | 99 |
| TRGV1.3 | hTRGV1.3up1 | 26 | 79 |
| TRGV1.4 | hTRGV1.4up1 | 28 | 158 |
| TRGV1.5 | hTRGV1.5up1 | 23 | 287 |
| TRGV1.8 | hTRGV1.8up1 | 26 | 129 |
| TRGV2.9 | hTRGV2.9up1 | 27 | 178 |

Cette analyse peut notamment être effectuée en ne faisant que des PCR multiplexes simples, ou des PCR multi-n-plexes avec n≥2 avec, dans une même réaction, un couple d'amorces spécifiques du locus TRG et un couple d'amorces spécifiques d'un autre locus.

La diversité des réarrangements intervenus dans le locus TRD peut également être étudiée par une méthode selon l'invention, en effectuant, à l'étape A), une PCR multi-2-plexe avec un triplet d'amorces constitué d'une amorce s'hybridant en amont et/ou dans un gène V de ce locus et des amorces antisens hTRDJ1do5 et hTRDJ3do2, définies comme suit :

- hTRDJ1do5 est un oligonucléotide antisens de 23 nucléotides s'hybridant entre les nucléotides 90 et 112 du gène J1 du locus TRD ; et
- hTRDJ3do2 est un oligonucléotide antisens de 26 nucléotides s'hybridant entre les nucléotides 448 et 473 du gène J3 du locus TRD.

Avec cette combinaison d'amorces, il est possible d'analyser, à partir d'une seule PCR multi-2-plexe, les réarrangements de tous les gènes J du locus TRD humain avec un gène V donné. L'analyse complète de tous les réarrangements VJ du locus TRD peut donc être obtenue en faisant une PCR multi-n-plexe avec n≥2 par famille TRDV. A titre d'exemples d'amorces sépcifiques de gènes TRDV utilisables avec les amorces antisens hTRDJ1do5 et hTRDJ3do2 pour mettre en oeuvre cet aspect de l'invention, on peut citer les amorces définies dans le tableau ci-dessous:

Tableau 7

| Nom du gène | Nom de l'amorce | Taille (nt) | Distance entre l'extrémité 5' de l'amorce et la fin du gène V (pb) |
|---|---|---|---|
| TRAV9 | hTRAV9-1up_ex_2/2_testAn2 | 25 | 113 |
| TRAV12.2,3 | hTRAV12.2,3up1 | 27 | 114 |
| TRAV12.1 | hTRAV12.1up1 | 28 | 112 |
| TRAV14 (TRDV4) | hTRAV14 up n2 | 22 | 69 |
| TRAV16 | hTRAV16 up n5 | 27 | 118 |
| TRAV17 | hTRAV17 up n2 | 22 | 40 |
| TRAV19 | hTRAV19 up | 24 | 144 |
| TRAV21 | hTRAV21 up | 24 | 91 |
| TRAV22 | hTRAV22up2 | 21 | 232 |
| TRAV23 (TRDV6) | hTRAV23 up n2 | 28 | 130 |
| TRAV25 | hTRAV25 up n3 | 27 | 154 |
| TRAV27 | hTRAV27 up | 27 | 138 |
| TRAV29 (TRDV5) | hTRAV29 up | 24 | 267 |
| TRAV30 | hTRAV30 up n2 | 24 | 139 |
| TRAV35 | hTRAV35_int up | 27 | 377 |
| TRAV36 (TRDV7) | hTRAV36up1 | 26 | 280 |
| TRAV38 (TRDV8) | hTRAV38-1,2 up n3 | 24 | 170/169 |
| TRAV39 | hTRAV39up1 | 26 | 352 |
| TRAV40 | hTRAV40 up | 28 | 76 |
| TRAV41 | hTRAV41_int up | 28 | 368 |
| TRDV1 | hTRDV1up1 | 25 | 259 |
| TRDV2 | hTRDV2up1 | 24 | 260 |
| TRDV3 | hTRDV3up1 | 24 | 287 |

Selon une autre variante, l'invention permet également l'analyse des réarrangements de tous les gènes J du locus IgH humain avec au moins 2 gènes $V_x$ et $V_y$ donnés du même locus, en effectuant au moins une PCR multi-n-plexe avec n≥2 (et en particulier avec n=2), avec une combinaison d'amorces comprenant 2 amorces sens s'hybridant en amont et/ou dans lesdits gènes $V_x$ et $V_y$ et une amorce antisens s'hybridant en aval et/ou dans le gène IgHJ6, par exemple l'amorce hIgHJ6do2 s'hybridant entre la 368ième et la 392ième base à compter du début du gène IgHJ6.

A titre d'amorces s'hybridant en amont et/ou dans un gène V du locus IgH humain utilisables selon cet aspect de l'invention, on peut citer les amorces définies dans le tableau ci-dessous :

Tableau 8

| nom du gène | nom oligonucléotide | taille (nt) | distance avec la fin du gène V en pb |
|---|---|---|---|
| IGHV1.2, 8, 18, 46, 69 | hIGHV1Aup1 | 25 | 172 |
| IGHV1.3, 8, 24, 45, 58, 69, f | hIGHV1Bup2 | 22 | 62 |
| IGHV2 | hIGHV2up1 | 22 | 44 |
| IGHV3.7, 13, 15, 20, 21, 23, 48, 53, 64, 66, 72, 73, 74 | hIGHV3Aup1 | 24 | 315 de V3.7 |
| IGHV3.7, 9, 11, 13, 15, 20, 21, 30, 33, 43, 48, 49, 72 | hIGHV3Bup1 | 21 | 288 de V3.7 |

(suite)

| nom du gène | nom oligonucléotide | taille (nt) | distance avec la fin du gène V en pb |
|---|---|---|---|
| IGHV4 | hIGHV4up1 | 25 | 69 |
| IGHV5 | hIGHV5up1 | 21 | 55 |
| IGHV6 | hIGHV6up1 | 23 | 371 |

Selon une mise en oeuvre particulière de cette méthode, les amorces spécifiques des gènes $V_x$ et $V_y$ du locus IgH sont choisies de telle sorte que la somme de la distance entre l'extrémité 5' de la zone d'hybridation de l'amorce spécifique de $V_x$ et la fin dudit gène $V_x$ et de la distance entre l'extrémité 5' de la séquence codante du gène IgHJ1 et l'extrémité 3' de la zone d'hybridation de l'amorce antisens hIgHJ soit supérieure à la somme de la distance entre l'extrémité 5' de la zone d'hybridation de l'amorce spécifique de $V_y$ et la fin dudit gène $V_y$ et de la distance entre l'extrémité 5' de la séquence codante du gène IgHJ6 et l'extrémité 3' de la zone d'hybridation de l'amorce antisens hIgHJ6. Ceci permet que l'amplicon correspondant à $V_x$J6 soit plus grand que celui correspondant à $V_y$J1, pour avoir une résolution des amplicons dans l'ordre $V_y$J6, $V_y$J5,..., $V_y$J1, $V_x$J6, ..., $V_x$J1.

Toutefois, étant donné la taille du cluster de gènes IgHJ, les amorces spécifiques des gènes $V_x$ et $V_y$ du locus IgH seront de préférence choisies de façon à obtenir une disposition "enchâssée" des amplicons, c'est-à-dire telle qu'au moins un amplicon obtenu avec un premier couple d'amorces soit encadré par 2 amplicons obtenus avec un deuxième couple d'amorces.

La méthode de l'invention permet également l'analyse des réarrangements incomplets du locus IgH humain, en effectuant au moins une réaction de PCR multi-n-plexe avec n=2 avec une combinaison d'au moins 3 amorces constituant au moins 2 couples d'amorces comportant une amorce antisens commune s'hybridant spécifiquement en aval et/ou dans un gène J donné telle que, par exemple, l'amorce hIgHJ6do2 décrite plus haut, chaque couple d'amorces comportant en outre une amorce sens s'hybridant spécifiquement en amont et/ou dans un gène D donné.

A titre d'amorces s'hybridant en amont et/ou dans un gène D du locus IgH humain utilisables selon cet aspect de l'invention, on peu citer les amorces définies dans le tableau ci-dessous :

Tableau 9

| nom du gène | (pb) | taille | Distance entre l'extrémité 5' de l'amorce et la fin du gène D (Pb) |
|---|---|---|---|
| hIGHD1 | hIGHD1up1 | 23 | 44 |
| hIGHD2 | hIGHD2up1 | 22 | 67 |
| hIGHD3 | hIGHD3up1 | 20 | 102 |
| hIGHD4 | hIGHD4up1 | 19 | 132 |
| hIGHD5 | hIGHD5up2 | 20 | 85 |
| hIGHD6 | hIGHD6up1 | 21 | 160 |
| hIGHD7 | hIGHD7up2 | 20 | 90 |

Cette analyse peut notamment être effectuée en ne faisant que des PCR multiplexes simples, ou des PCR multi-n-plexes avec n=2 avec, dans une même réaction, un couple d'amorces spécifiques du locus IgH et un couple d'amorces spécifiques d'un autre locus.

Selon une mise en oeuvre préférée de l'invention, la méthode combine l'analyse des réarrangements V(D)J du locus IgH et celle des réarrangements incomplets de ce locus, par la mise en oeuvre des variantes appropriées décrites ci-dessus. Les amorces décrites plus haut pour cette analyse sont adaptées à l'analyse des réarrangements de ce locus chez l'humain, mais cette méthode peut être transposée, sans aucune difficulté, chez l'animal, par exemple chez la souris.

Selon une autre mise en oeuvre de l'invention, la méthode est adaptée pour analyser la diversité combinatoire des réarrangements V(D)J d'au moins deux locus génétiques choisis parmi les loci TRA, TRB, TRG, TRD, IGH, IGK et IGL, en combinant les variantes appropriées décrites ci-dessus. L'analyse des différents loci peut se faire de façon simultanée ou séquentielle, en faisant le cas échéant des PCR multi-n-plexes avec, dans la même réaction, au moins un couple spécifique d'un locus et un autre couple spécifique d'un autre locus.

En particulier, l'analyse combinée de la diversité combinatoire des réarrangements V(D)J du locus TRB et de la diversité combinatoire des réarrangements VJ du locus TRG ou du locus TRD permet d'avoir une vision représentative du répertoire de lymphocytes T. En ajoutant l'analyse de la diversité combinatoire des réarrangements V(D)J du locus IgH, on obtient une information sur l'ensemble des lymphocytes (B et T).

Un aspect important de l'invention, illustré dans les exemples ci-après, est la possibilité d'identifier par leur nom les différents réarrangements observés. Selon une mise en oeuvre préférée, l'étape C) de la méthode comporte une étape de traitement des données obtenues par la séparation des amplicons suivant leur taille, ledit traitement étant effectué par un ordinateur et permettant d'attribuer à chaque amplicon observé le nom du réarrangement V(D)J correspondant. De manière encore préférée, le traitement des données intègre également l'intensité du signal de chacun des amplicons observés, pour quantifier la fréquence relative du réarrangement V(D)J correspondant.

Ceci permet de décrire une signature d'une diversité immunitaire en classant les réarrangements VDJ par ordre d'intensité ou par ordre de contribution au sein du répertoire immunitaire observé. Ce classement des réarrangements correspond à une signature du répertoire immunitaire à un instant "t" dans un échantillon.

En particulier, la méthode de l'invention peut être telle que l'étape B) comprend l'acquisition des données concernant la taille des amplicons et, pour chacun, l'intensité du signal, et l'étape C) comprend les étapes suivantes :

(i) identification de chaque amplicon, en déterminant à quel réarrangement V(D)J il correspond, en fonction de sa taille ;
(ii) à partir de l'intensité du signal de chaque amplicon, détermination de la proportion de l'ADN génomique de départ présentant le réarrangement V(D)J correspondant ;
(iii) présentation des résultats sous forme d'un graphique tridimensionnel présentant les gènes ou les familles de gènes $V_x$ suivant un axe, les gènes $J_y$ suivant un autre axe, et la fréquence des réarrangements $V_xJ_y$ suivant le troisième axe.

Si on mesure en outre la diversité moléculaire, par exemple en effectuant une mesure en temps réel des amplifications par PCR multi-n-plexe, la méthode permet de mesurer une diversité immunitaire globale par la prise en compte de la mesure de la diversité combinatoire et de la diversité moléculaire.

La présente invention porte également sur une méthode pour déterminer *in vitro* le niveau d'immunodéficience d'un individu, comportant les étapes suivantes :

A) à partir d'un échantillon biologique dudit individu, faire une numération lymphocytaire ;
B) à partir du même échantillon ou d'un autre échantillon provenant du même individu au même moment, déterminer le niveau de diversité combinatoire du répertoire des lymphocytes dudit individu, par la mise en oeuvre d'une méthode telle que décrite plus haut ;
C) combiner les données obtenues aux étapes A) et B).

Cette méthode peut comporter une étape additionnelle d'interprétation de la combinaison obtenue à l'étape C), au regard d'un graphique attribuant un niveau de risque au moins aux zones (i) à (iv), et de préférence aux zones (i) à (vi) ci-après :

(i) numération faible (<1000 Ly/μL) et diversité combinatoire V-J faible (<40%) : risque infectieux élevé ;
(ii) numération faible (<1000 Ly/μL) mais diversité combinatoire V-J normale (>65%) : risque infectieux faible ;
(iii) numération normale (1000-3200 Ly/μL) et diversité combinatoire V-J faible (<40%) : risque infectieux moyen ;
(iv) numération normale (1000-3200 Ly/μL) et diversité combinatoire V-J normale (>65%) : le répertoire immunitaire est sain.
(v) numération au dessus de la normale (> 3200Ly/μL), et diversité combinatoire V-J faible (<40%) : risque lympho-prolihératif élevé
(vi) numération au dessus de la normale (> 3200Ly/μL), et diversité combinatoire V-J normale (>65%) : risque lymphoprolifératif moyen.

Dans une mise en oeuvre préférée d'une méthode pour déterminer *in vitro* le niveau d'immunodéficience d'un individu telle que décrite ci-dessus, l'étape B) comprend la détermination du niveau de diversité combinatoire du répertoire des lymphocytes T et des lymphocytes B dudit individu. Dans ce cas de figure, on peut avantageusement examiner les données obtenues à l'aide d'un graphique tridimensionnel présentant le niveau de diversité des immunoglobulines sur un axe, le niveau de diversité des TCR sur un autre axe, et la numération lymphocytaire sur un troisième axe.

Un autre aspect de l'invention concerne une méthode de suivi de l'évolution de la diversité du répertoire des lymphocytes T et/ou B d'un individu, comportant les étapes suivantes :

A) Mesure de la diversité du répertoire des lymphocytes dudit individu, par la mise en oeuvre d'une méthode telle que décrite ci-dessus, à partir de deux échantillons dudit individu prélevés à deux dates différentes ;

B) comparaison des deux échantillons en évaluant :

(i) le nombre S de réarrangements observés dans les deux échantillons ;
(ii) le nombre A de réarrangements observés dans l'échantillon le plus récent mais pas dans le plus ancien ;
(iii) le nombre D de réarrangements observés dans l'échantillon le plus ancien mais pas dans le plus récent ;
(iv) le nombre Z de réarrangements qui ne sont observés dans aucun des échantillons.

Un exemple d'interprétation de ce graphique est présenté à l'exemple 11 ci-après. Cette méthode peut également servir aussi à comparer 2 échantillons d'individus différents, par exemple pour comparer un donneur et un receveur en cas de greffe.

La présente invention porte également sur une trousse, ou *"kit",* pour la mise en oeuvre d'une des méthodes décrites plus haut, comprenant au moins une combinaison d'amorces telles que définies dans ce texte, et des réactifs pour effectuer des PCR.

Parmi les réactifs pour effectuer des PCR, une trousse selon l'invention comprendra de préférence une polymérase présentant les caractéristiques suivantes :

(i) elle est capable d'amplifier des fragments de plusieurs dizaines de kb ;
(ii) sa vitesse d'élongation est d'au moins 1 kb/minute ;
(iii) sa robustesse est telle qu'elle n'introduit pas plus d'une erreur par kb, en moyenne.

De façon avantageuse, une trousse de l'invention comprendra une plaque multipuits dans laquelle chaque puit contient une combinaison différente d'amorces, sous forme lyophilisée ou en phase liquide. De préférence, cette plaque multipuits comprend toutes les combinaisons d'amorces nécessaires à l'amplification d'au moins 50, 60, 70, 80 voire 95% des réarrangements V-J d'au moins un locus choisi parmi les loci TRA, TRB, TRG, TRD et IGH.

Un autre aspect de l'invention concerne l'utilisation d'une méthode ou d'une trousse telle que décrites plus haut, pour étudier la mise en place et/ou la qualité du répertoire TCR et/ou IgH d'un animal transgénique humanisé et/ou d'une culture de lymphocytes. Ceci permet notamment de vérifier la qualité d'un répertoire immunitaire suite à une culture cellulaire, par exemple pour vérifier que la culture cellulaire reste appropriée pour tester des molécules ou pour étudier des mécanismes biologiques. Dans le cas de lignées T ou B monoclonales ou oligoclonales, cela permet de vérifier qu'il s'agisse bien du ou des clones préalablement identifiés, et ainsi de détecter toute contamination ou erreur d'étiquetage sur un tube avec de lancer une expérience. Une autre application importante est de contrôler la qualité lors de la production de cultures de Lymphocytes (T régulateurs par exemple...) avant réinjection (à visée thérapeutique).

La présente invention porte également sur l'utilisation d'une méthode ou d'une trousse telle que décrite plus haut, pour cribler des molécules thérapeutiques *in vitro.* Des exemples d'applications sont décrits à l'exemple 18 ci-après.

Outre les dispositions qui précèdent, l'invention comprend encore d'autres dispositions, qui ressortiront des exemples expérimentaux ci-dessous, et des figures annexées.

**Exemple 1 : Choix de la position des amorces utilisables dans le cadre de l'invention**

*Critères de sélection des oligonucléotides*

Les amorces utilisables pour mettre en oeuvre le procédé de l'invention sont choisies en fonction 1) de leurs propriétés thermodynamiques (déterminées à partir d'algorithmes classiquement utilisés par l'homme du métier pour identifier la capacité des oligonucléotides à se fixer sur leur séquence cible, notamment en fonction du nombre de liaisons hydrogènes) ; 2) de leur compatibilité avec les autres amorces utilisées dans le même tube, tant sur le plan thermodynamique que sur l'incapacité des différentes amorces à s'hybrider entre elles ; et 3) de leur position respective qui permet d'obtenir des tailles d'amplicons pouvant être résolus.

Par "résolu", il doit être compris que les amplicons peuvent être observés de façon univoque, suite à leur séparation par la taille par une méthode d'électrophorèse ou tout autre méthode. Lorsque les tailles de certains amplicons sont trop proches, il n'est pas possible de les discerner distinctement avec des conditions de séparation compatibles avec la séparation permettant de "résoudre" l'ensemble des autres tailles d'amplicons. Ce cas est minoritaire et identifié.

Les oligonucléotides sont décrits ici en donnant leur position dans le locus ainsi que leur taille, en nombre de bases.

Pour les gènes V, les oligonucléotides sont orientés dans le sens de transcription, ils sont dénommés "SENS" ; ils sont complémentaires au brin d'ADN non codant.

Pour les gènes J, les oligonucléotides sont dénommés "ANTISENS" : ils sont complémentaires au brin d'ADN codant et inversés (on dit aussi qu'ils sont dans le sens 3'→5').

***Amorces spécifiques des <u>gènes V</u>***

Pour les oligonucléotides spécifiques des gènes V, la position est donnée en fonction de <u>la fin du gène V</u>, c'est-à-dire la dernière base avant le RSS. Cette position correspond à la distance (incluant l'oligonucléotide) entre la 1<sup>ère</sup> base de l'oligonucléotide et la dernière base du gène V.

o Exemple de position de l'oligonucléotide pour la famille de gène TRBV : si la distance est de n base de la fin du gène V et la taille de l'oligonucléotide est de *t* bases, l'oligonucléotide débute à n bases en amont de la fin du gène (en comptant la dernière base) et se termine à *n-t+1* bases de la fin du gène V.

Les oligonucléotides ont été sélectionnés de manière à s'hybrider au plus grand nombre de membres possible d'une même famille V. Deux cas de figures peuvent être décrits concernant le nombre d'oligonucléotides V nécessaires pour suivre toute une famille V :

o *Cas 1 : existence d'une zone d'homologie de 100% entre tous les membres d'une même famille.* dans ce cas il est possible de trouver, en faisant un alignement de séquences, une zone commune à 100% pour tous les membres de la famille V en question et répondant aux critères de sélection des oligonucléotides précisés précédemment. Dans ce cas, <u>1 seul oligonucléotide V</u> est nécessaire pour suivre tous les membres de la famille.

o Cas 2 : zone d'homologie <u>inférieure</u> à 100%. Dans ce cas, la plus grande zone (en nombre de bases) qui répond aux critères de sélection est sélectionnée, et <u>tous les oligonucléotides</u> correspondants à cette position nécessaires pour suivre tous les membres de la famille sont dessinés. Ex : pour une famille de 5 membres présentant une zone d'homologie de 100% pour 3 membres, les 2 autres membres étant différents entre eux dans cette zone. Dans ce cas, un total de 3 oligonucléotides différents correspondant à la même position sont dessinés pour suivre l'ensemble des membres de cette famille. Trois sous cas sont alors possibles :

• Les oligonucléotides V choisis à cette position sont compatibles entre eux thermodynamiquement. Dans ce cas, les n oligonucléotides V (dans l'exemple ci-dessus 3 oligonucléotides) sont regroupés dans un même tube de PCR. Puisque tous ces oligonucléotides, même si ils ont quelques bases différentes, sont dessinés à la même position, les amplicons seront donc de même taille.

• Les oligonucléotides V ne sont pas suffisamment compatibles thermodynamiquement ou ils ne peuvent être mis dans la même réaction de PCR car cela poserait des problèmes de dimères. Dans ce cas, 2 ou n PCR peuvent être réalisées dans des tubes différents, pour suivre spécifiquement les membres V pour lesquels les amorces sont incompatibles avec les autres.

• Il existe un cas particulier (rare) dans lequel les gènes V d'une même famille n'ont pas la même taille. Ceci est dû au fait que l'intron d'un ou de n membre(s) d'une famille V présente(nt) une taille différente de celui des autres membres de ladite famille V (rmq : il n'y a qu'un intron par gène V). Ce cas de figure n'est pas gênant si l'oligonucléotide V est dessiné en aval de cette "zone de taille différente". Si cela n'est pas possible, la solution mise en oeuvre consiste à séparer les 2 ou n oligonucléotides dans 2 ou n PCR.

*Amorces spécifiques des **gènes J***

La position d'une amorce spécifique d'un gène J est donnée en indiquant sa distance par rapport au <u>début du gène J,</u> c'est-à-dire la 1<sup>ère</sup> base du segment de gène J (séquence codante), après le RSS. Cette position ce situe en aval du début du J et correspond à la distance (incluant l'oligonucléotide) entre la 1<sup>ère</sup> base du gène J et la première base de l'oligonucléotide (c'est-à-dire la base à l'extrémité 5' de l'amorce).

o Exemple de position de l'oligonucléotide pour la famille de gène TRBJx : la distance est de n bases du début du J, la taille de l'oligonucléotide est de *t* bases. Donc la zone d'hybridation de l'amorce (sur le brin codant) se termine à la *n*<sup>ième</sup> base du gène J ou en aval du début du gène J et débute à *n-t+1* bases du début du J.

***Récupération des séquences des loci du TCR et IG***

Plusieurs méthodes sont possibles pour obtenir les séquences. Deux possibilités sont décrites ci-après.

**1<sup>e</sup> possibilité**

La 1<sup>e</sup> possibilité nécessite d'aller sur le site internet Européen *« Ensembl Genome Browser »* <u>http://www.en-sembi.org</u> et de rechercher le locus d'intérêt : après avoir choisi l'espèce (humain, souris, etc.) il faut cliquer sur le chromosome d'intérêt, exemple chromosome 14 pour le locus TRA. Il faut ensuite indiquer, dans la case prévue à cet effet, le chiffre indiquant le début *(start)* de la région chromosomique *(p. ex.* :21158000) et la fin de la région *(end) (p. ex.* :22125000). La base de données *Ensembl* fait ressortir graphiquement la disposition des gènes TCR et IG présents dans le locus et l'homme du métier peut exporter (par un clic gauche sur le contig concerné) la séquence d'ADN au format EMBL ou GenBANK avec toutes les annotations de gènes correspondantes. Il a ainsi à sa disposition la localisation de toutes les séquences des gènes TCR et IG, y compris les séquences en amont et aval de ces derniers.

Les régions chromosomiques pour les différents loci humains concernés par la présente invention sont indiquées ci-après.

*Homo sapiens* TRAITRD : locus à 14q11.2:

http://www.ensembl.org/Homo sapiens/contigview?region=14&vc start=2115 8000&vc end=22125000

*Homo sapiens* TRB : locus à 7q34:

http://www.ensembl.org/Homo_sapiens/contigview?region=7&vc start=14164 0000&vc end=142275000

*Homo sapiens* TRG : locus à 7p14:

http://www.ensembl.org/Homo sapiens/contigview?region=7&vc start=38242 000&vc end=38385000

*Homo sapiens* IGH: locus à 14q32.33:

IGHV :

http://www.ensembl.org/Homo sapiens/contigview?region=14&vc start=1054 76000&vc end=106368585

IGHD et IGHJ :

http://www.ensembl.org/Homo sapiens/contigview?region=14&vc start=1054 00000&vc end=105460000

IGHC:

http://www.ensembl.org/Homo sapiens/contigview?region=14&vc start=1051 20000&vc end=105400000

*Homo sapiens* IGK : locus à 2p11.2:

IGKV (proximal cluster), IGKJ et IGKC :

http://www.ensembl.org/Homo_sapiens/contigview?region=2&vc start=88920 000&vc end=89480000

IGKV (duplicated distal cluster) :

http://www.ensembl.org/Homo_sapiens/contigview?region=2&vc start=89550 000&vc end=89950000

*Homo sapiens* IGL : locus à 22q11.2:

http://www.ensembl.org/Homo sapiens/contigview?region=22&vc start=2070 0000&vc end=21650000

**2ème possibilité :**

La 2ème possibilité présentée ci après nécessite de regrouper tous les cosmides contenant les séquences d'un locus TCR ou IG. Pour ce faire, nous avons identifié la liste des numéros d'accession des cosmides pour les chaînes des IG et des TCR, chez l'homme et la souris en se basant sur la bibliographie (Lefrancs, *The Immunoglobulin Facts Book* 2001 et Lefrancs *The T cell receptor Facts Book* 2001), ou encore (Baum et al., 2006; Baum et al., 2004).

Tableau 10

| Humain : | |
|---|---|
| **Locus** | **Numéro d'accession EMBL-EBI** |
| TRAD* | AE000658 à AE000662 |
| TRB | L36092 |
| TRG | AF159056, X08084, M12950, M12960, M16016 et M12961 |
| IGH | voir Lefranc ., The Immunoglobulin FactsBook ISBN:012441351X |
| | |
| **SOURIS :** | |
| **Locus** | **Numéro d'accession EMBL-EBI** |
| **TRAD*** | AE008683 à AE008686 |
| **TRB** | AE00063 à AE00065 |
| **TRG** | AF037352 and AF021335 |

(suite)

| SOURIS : | |
|---|---|
| **Locus** | **Numéro d'accession EMBL-EBI** |
| **IGH** | voir Lefranc ., The Immunoglobulin FactsBook ISBN:012441351X |
| * Rappel : le locus TRD est situé dans le locus TRA. | |

A partir de ces numéros il est possible de récupérer l'ensemble des informations sur les séquences sources des loci dans le site internet de référence Européen « EMBL-EBI » (http://www.ebi.ac.uk), en faisant une « recherche », dans la rubrique « nucleotide sequence ». Les résultats de la « EMBL-BANK » (*Europe's primary nucleotide sequence resource)* sont ensuite téléchargeables au format EMBL.

L'étude des séquences peut être réalisée sur des logiciels tel que *NTI Vector®.* Les gènes étant annotés, leur position est indiquée précisément.

### Correspondance entre les différentes nomenclatures des gènes des loci TCR et IG

Il est important de noter que la nomenclature des gènes a évolué au court du temps. Afin de se repérer entre les nomenclatures, l'homme du métier dispose de tableaux de correspondances pour les TCR et IG que l'on peut retrouver dans les 2 livres [1] Lefranc, M.-P. and Lefranc, G., The Immunoglobulin FactsBook, Academic Press, 458 pages (2001) ISBN:012441351X [2] Lefranc, M.-P. and Lefranc, G., The T cell receptor FactsBook, Academic Press, 398 pages (2001) ISBN:0124413528. Ces informations peuvent aussi être retrouvées sur le site IMGT (http://imgt.ci-nes.fr).

### Exemples

Les tableaux ci-dessous montrent 2 exemples d'amorces (antisens) spécifiques de gènes J et 5 exemples d'amorces (sens) spécifiques de gènes V.

Tableau 11

| nom du gène | nom nucléotide | oligo- taille séquence | (nt) | distance avec le début du gène Jenpb | SEQ ID No : |
|---|---|---|---|---|---|
| TRAJ33 | hTRAJ33do | 5'-GTCTCCTCTCCCGTGCAGTCA-3' | 21 | 94 | 1 |
| TRAJ5 | hTRAJ5do | 5'-CTGTCTCTGCAATGATGAAATGGCC-3' | 25 | 275 | 2 |

Tableau 12

| nom du gene | nom de l'oligonucléotide | séquence | taille (nt) | distance avec la fin du gène V (pb) | SEQ ID No : |
|---|---|---|---|---|---|
| TRAV10 | hTRAV10 up n3 | CTGGATGCAGACACAAAGCAAAGC | 24 | 85 | 3 |
| TRAV12.2,3 | hTRAV12.2,3up1 | AATGAAGATGGAAGGTTTACAGCACAG | 27 | 114 | 4 |
| TRAV12.1 | hTRAV12.1up1 | ACAAAGAAGATGGAAGGTTTACAGCACA | 28 | 112 | 5 |
| TRAV16 | hTRAV16 up n5 | CTAGAGAGAGCATCAAAGGCTTCACTG | 27 | 118 | 6 |
| TRAV27 | hTRAV27 up | TGGTGACAGTAGTTACGGGTGGAGAAG | 27 | 138 | 7 |

**Exemple 2: Protocole pour l'utilisation *d'ImmunTraCkeR Kit Exemple sur le locus TRB.***

Suivant la nature de l'échantillon (cellules ; PBMC ; extrait de thymus ;...), les quantités nécessaires sont optimisées. Pour un échantillon cellulaire, la quantité nécessaire pour réaliser l'expérimentation est de $10^6$ cellules.

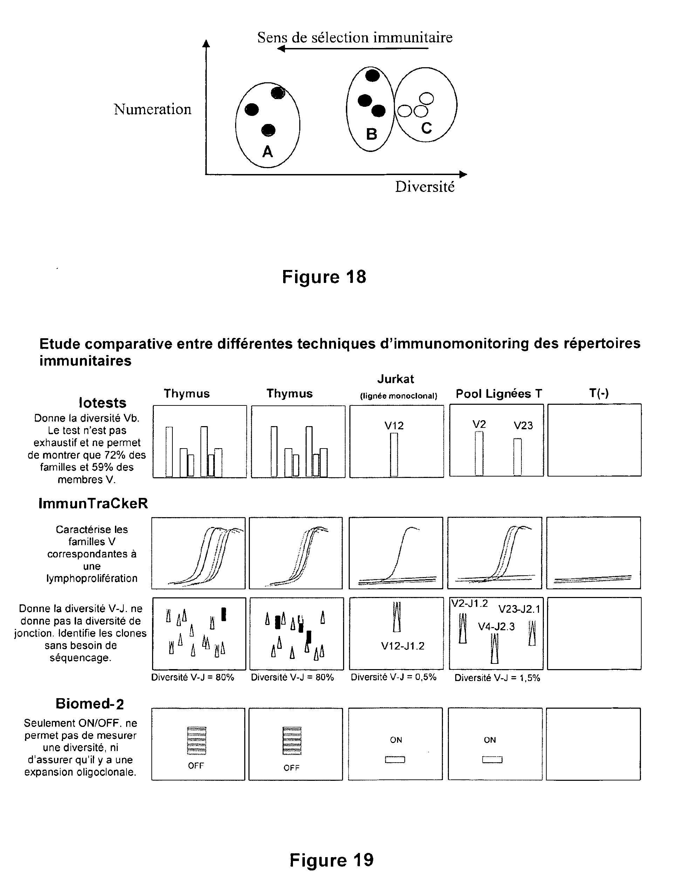

La succession des différentes étapes du protocole est schématisée sur la figure 19.

### 2-A. Extraction de l'ADN

Un ADN extrait d'une haute pureté est nécessaire pour la détection des réarrangements V-J en utilisant la trousse *ImmunTraCkeR Kit.* L'homme du métier sait quel procédé ou kit sont appropriés pour cela. En particulier, l'homme du métier sait que cette extraction doit être réalisée sans aucun EDTA ou autre produit pouvant inhiber la PCR. Les inventeurs recommandent d'extraire l'ADN en utilisant *High Pure PCR Preparation Template Kit* de Roche®. La concentration d'ADN recommandée est de 100 ng/µl.

### 2-B. Contrôle de la qualité de l'ADN et détermination de la quantité

Une mesure de l'absorbance de l'échantillon à 260 nm avec un spectrophotomètre (exemple *Amersham Gen-Quant Pro*) est réalisée. Cette mesure permet de calculer la concentration de l'ADN, le taux d'extraction et le rapport ADN/protéine qui donne une estimation de la qualité de l'ADN. En outre, l'état de dégradation de l'ADN est contrôlé sur un gel d'agarose et ensuite la concentration de l'ADN est normalisée par comparaison avec un contrôle d'actine.

### 2-C. Amplification par PCR

*L'immunTraCkeR Kit* contient les combinaisons d'amorces (déshydratées ou en phase liquide) déjà distribuées dans les tubes. Le mélange réactionnel est préparé et distribué dans lesdits tubes de réaction.

a) Protocole optimisé *Herculase®ll Fusion*

Préparation du mélange réactionnel

Tableau 13

| Composant | Quantité par réaction |
|---|---|
| Eau distillée | 12,89 µl |
| Tampon réactionnel Herculase® II 5X | 5µl |
| Mélange de dNTP (10 mM) | 0,62 µl |
| Matrice d'ADN (50ng/µl) | 1µl |
| ADN polymérase *Herculase® II Fusion* | 0.5µl |
| | Volume final : 20 µl |

Le mélange réactionnel est alors distribué dans des tubes ou des puits, à raison de 20 µl dans chacun.

La PCR est réalisée en utilisant des conditions cycliques optimisées. Des paramètres de cyclage suggérés pour effectuer des PCR avec l'ADN polymérase *Herculase® II Fusion,* en utilisant un dispositif *Primus* 96+ *(MWG)* sont indiqués ci-dessous.

Paramètres cycliques de la PCR:

Tableau 14

| Segment | Nombre de cycles | Température | Durée |
|---|---|---|---|
| 1 | 1 | 98°C | 3 minutes |
| 2 | 30 | 98°C | 20 secondes |
| | | 62°C | 20 secondes |
| | | 72°C | 3 minutes 30 secondes |
| 3 | 1 | 72°C | 3 minutes |

Durée de la PCR : env 4,5 heures.

### 2-D. Electrophorèse sur gel d'agarose

Préparer un gel d'agarose à 0,8% (p/v) dans un tampon TBE 1X. Charger les produits de la PCR mélangés auparavant avec un tampon de charge (0,25% du bromophénole bleu, 0,25% du xylène cyanol FF, 30% du Ficoll 400, dans de l'eau), à raison d'environ 10μl de produits PCR et 2μl du tampon de charge. Déposer un marqueur de taille d'ADN approprié sur chaque extrémité du gel. Mettre le gel sous tension dans un tampon TBE 1X, sans recirculation du tampon, pendant 1 heure et 30 minutes à 250V et 120mA. Colorer le gel avec 40μl de bromure d'éthydium dilué dans 150 ml de tampon TBE 1X pendant 30 minutes.

### 2-E. Acquisition et interprétation

Poser le gel sur un transilluminateur à UV et acquérir l'image, par photographie. Enregistrer la présence ou l'absence des produits spécifiques de la PCR.

### Exemple 3 : Analyse des réarrangements de tous les gènes J fonctionnels du locus béta avec 1 gène V donné, en 1 PCR multi-2-plexe résolutive

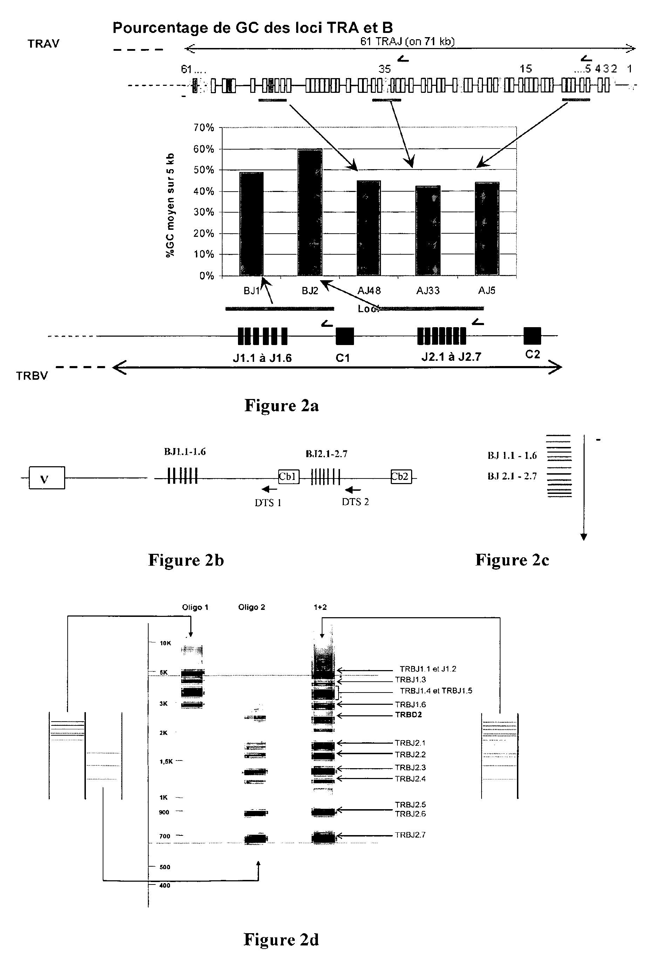

La figure 2a, montre l'analyse du taux de GC moyen des régions TRBJ et TRAJ humaine. La région J2 contient un taux très élevé de GC avec 60% contre 40-45% en moyenne.
La figure 2b montre la disposition particulière des gènes J du locus TRB (ne 2 clusters distincts).
La figure 2c illustre le principe de choix des amorces, avec 1 amorce antisens proche d'un cluster J et deuxième amorce antisen loin de l'autre cluster J, permettant de positionner l'intégralité du cluster J1 au dessus du cluster J2.
La figure 2d présente un exemple de résultat obtenu par cette méthode.
Cette configuration permet de diviser par 2 la quantité de matériel biologique nécessaire et réduire par la même occasion le prix de revient du test. Le temps pour réaliser le test est également diminué. En outre, ceci permet une lecture simplifiée de la piste du gel (ou d'un autre type de séparation) car les gènes J sont dans l'ordre du locus et sont donc facilement identifiables.

### Exemple 4 : Analyse de plusieurs loci dans une même réaction de PCR multi-n-plexe

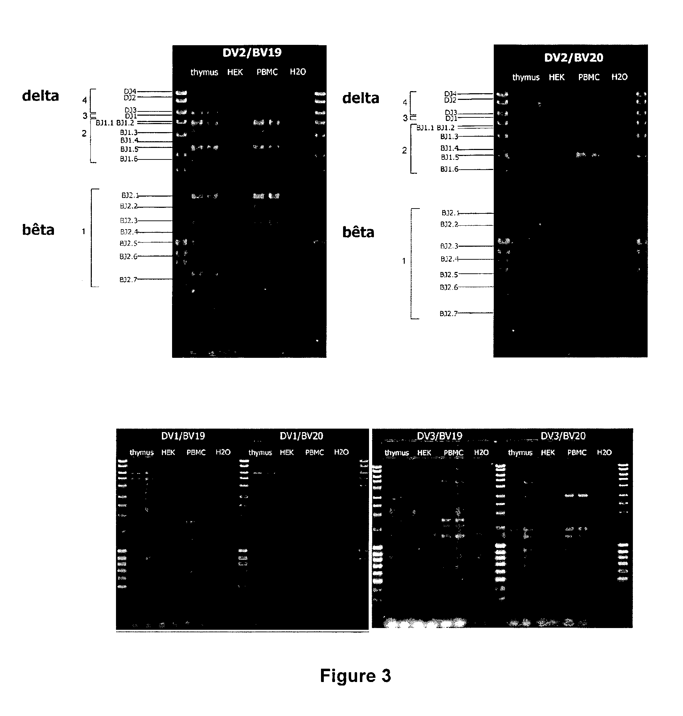

La figure 3 illustre la possibilité d'analyser des réarrangements de plusieurs loci dans une même réaction, de manière à diminuer la quantité de matériel biologique d'un facteur 3-4, nécessaire pour suivre l'ensemble du répertoire immunitaire. Dans cet exemple, des réarrangements des répertoires TCRB et TCRD sont observés en 1 seule étape. Ceci permet de suivre tous les lymphocytes T alpha/beta et Tgamma/delta.

### Exemple 5 : PCR multiplexe enchâssée.

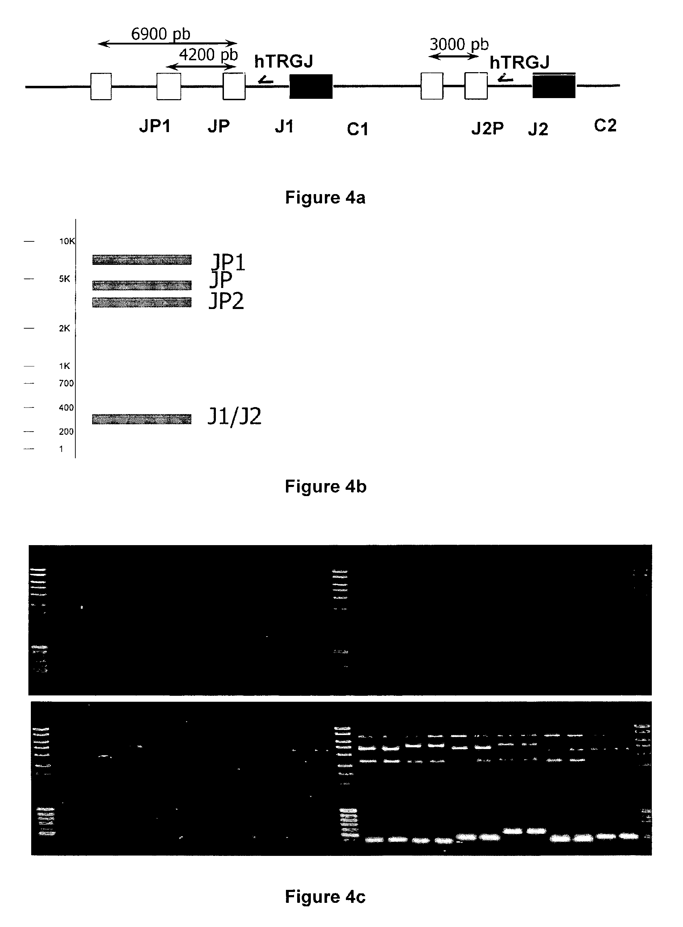

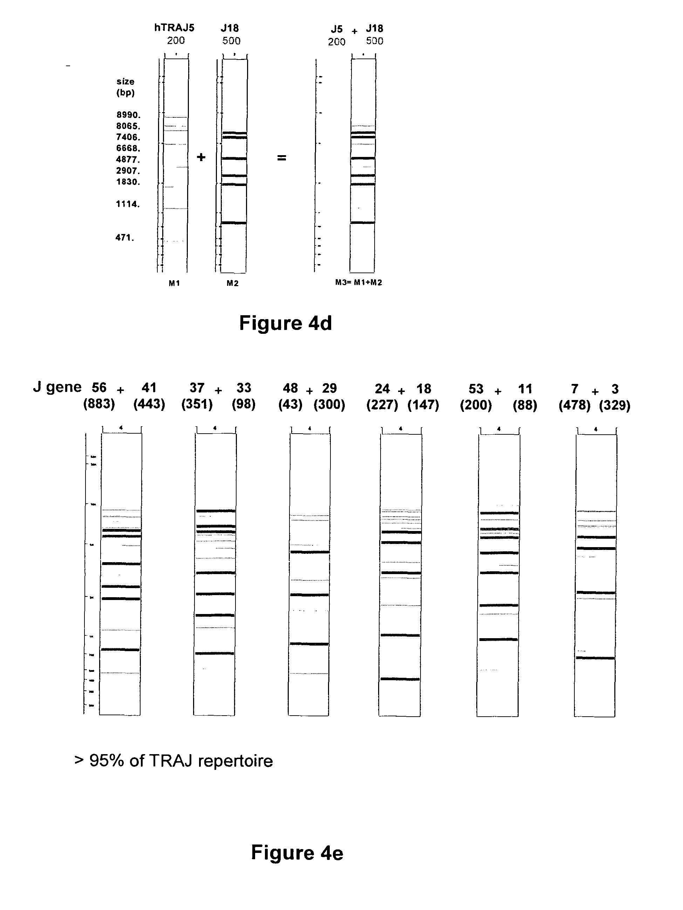

La figure 4 illustre le principe de PCR multi-n-plexe "enchâssée", c'est-à-dire dans laquelle les séries d'amplicons obtenues avec les différents couples d'amorces sont telles qu'un amplicon obtenu avec un premier couple d'amorces peut être encadré par 2 amplicons obtenus avec un deuxième couple d'amorces.
La figure 4a présente un schéma du locus TRG.
La figure 4b schématise le principe de la résolution avec une famille hTRGV.
La figure 4c montre le résultat d'une PCR multiplexe sur le locus hTRG, visant les 2 clusters J avec une seule amorce J. l'expérience a été réalisée sur des cellules HEK et CaCO comme contrôles négatifs et sur des PBMC et des cellules de thymus comme contrôles positifs.
A noter que pour le locus TRG, il est possible de suivre 2 clusters TRGJ avec 1 seul oligonucléotide J, du fait de l'homologie de séquence de 100% en aval des gènes J1 et J2.
La figure 4d montre un exemple de PCR multi-2-plexe enchâssée avec deux amorces TRAJ.
La figure 4e montre un exemple de résolution de 95% de la région AJ avec seulement 6 PCR multi-2-plexes. La position des primers est indiquée en dessous du nom du gène J des oligonucléotides en aval du (début) du gène J. Cette position est importante. Elle permet de s'assurer que les bandes attendues auront une taille leur permettant d'être bien résolues.

### Exemple 6 : Résultat de la trousse *ImmunTraCkeR* TRBV en PCR multi-2-plexe

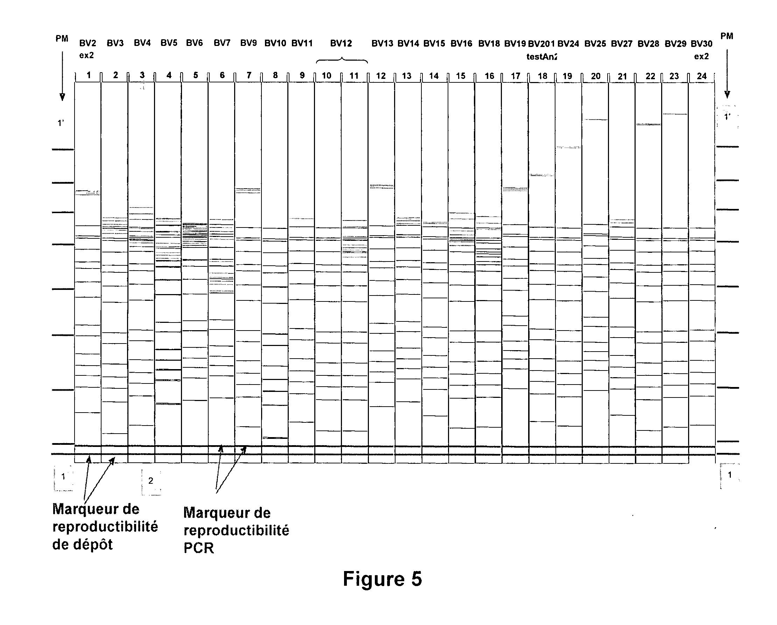

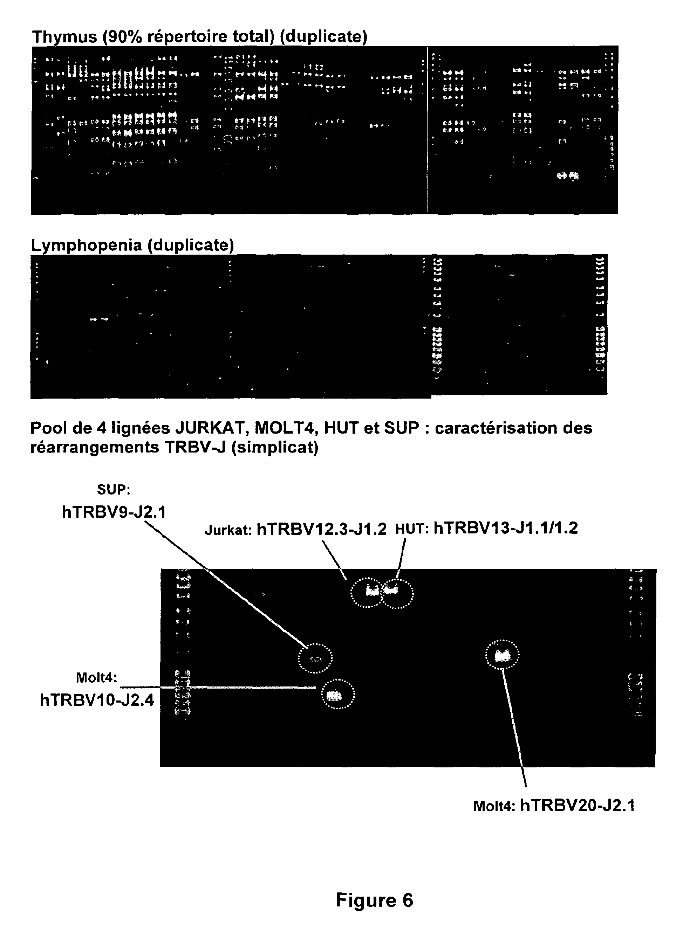

Les figures 5 et 6 montrent les différentes bandes obtenues après la migration sur gel d'agarose de tous les produits PCR obtenus par PCR multi-n-plexe. La figure 5 montre une représentation schématique du résultat théorique obtenu avec la trousse I*mmunTraCkeR* TRB humain (la représentation est similiaire chez d'autres espèces : le rat, la souris, le singe...). Chaque colonne correspond à 1 famille TRBV, chaque bande correspond à un réarrangement V-J donné. Les gènes TRBV ont été étudiés dans l'ordre suivant : BV2, BV3, BV4, BV5, BV6, BV7, BV9, BV10, BV11, BV12, BV13, BV14, BV15, BV16, BV18, BV19, BV20, BV24, BV25, BV27, BV28, BV29, BV30. La figure 6 montre les résultats expérimentaux correspondants obtenus en duplicat (déposé côte à côte), avec trois types d'échantillons : ADNg extrait de thymus, ADNg extrait de PBMC en condition lymphopénique et enfin un pool d'ADN de 4 lignées T comportant chacune un ou 2 réarrangements TRBV-J.

Le fait de disposer côte à côte l'ensemble des PCR des 24 TRBV permet une détection exhaustive des gènes TRBV sur l'ensemble des segments BJ.

On peut également rajouter (en option) un marqueur de reproductibilité de dépôt et/ou de PCR dans chacune des pistes (RMQ : ces 2 marqueurs de reproductibilité ne sont pas indispensables). Avantage recherché : améliorer la normalisation du signal entre les bandes.

Cette figure illustre le fait que l'invention permet d'évaluer la qualité d'un répertoire immunitaire, en mesurant à la fois la diversité combinatoire d'une image du répertoire immunitaire (calcul de la somme des bandes obtenues) ainsi que l'intensité du signal de toutes les bandes (calcul de la somme de tous les signaux des amplicons de l'image). En outre, le nom de chaque réarrangement V(D)J présent ou absent est identifié (en fonction de sa position dans l'image. La colonne donne le nom du V ; la taille de l'amplicon donne le nom du J. L'intensité du signal détecté donne la proportion respective de chaque réarrangement V(D)J. Au total cela permet donc en 1 seule étape d'avoir un outil qui mesure à la fois la diversité (utile pour mesurer le niveau d'immunodéficience précis d'un patient) et identifie le nom des réarrangements (donc le marqueur TCR ou IG d'un lymphocyte) impliqués dans une pathologie (leucémie, lymphome, GVHD...) ou ayant réagi à la hausse (multiplication cellulaire) ou à la baisse suite à un traitement.

**Exemple 7 : Etude des réarrangements D-J par PCR clustering**

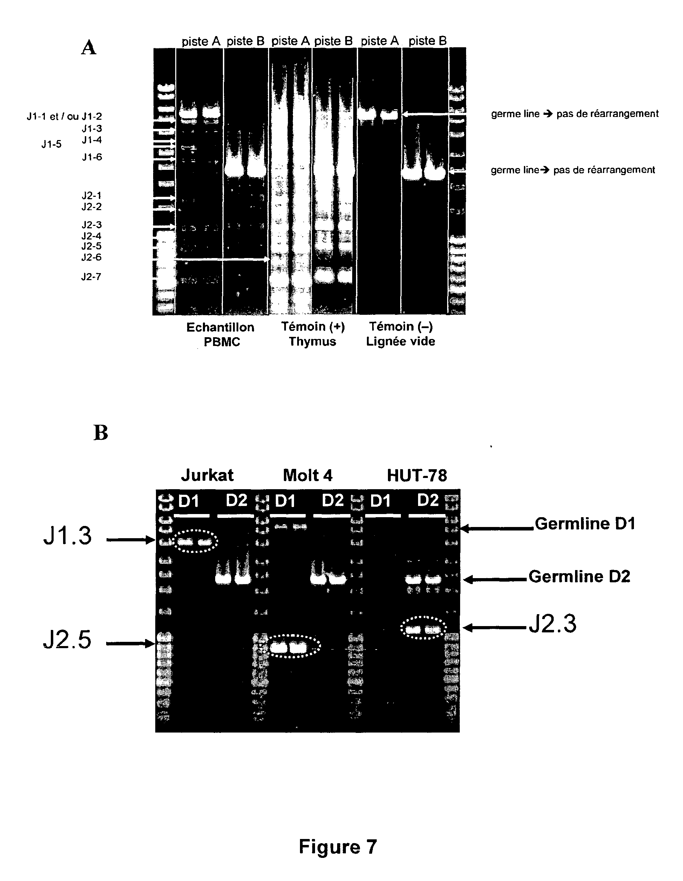

La figure 7 montre le résultat d'un test de détection des réarrangements incomplets D-Jβ à la fois exhaustif et résolutif (Pistes A : réarrangements D1 avec le cluster J1 et le cluster J2. Pistes B : réarrangements D2 avec le cluster J2). Ce test permet de caractériser un clone en précisant le nom des gènes D et J réarrangés.

En comparaison, Biomed-2 ne propose qu'un signal de type ON /OFF : la présence d'une population monoclonale est effectivement détectée, mais cette population n'est pas caractérisée au niveau combinatoire. Il n'est pas possible de faire la différence entre les gènes V et J réarrangés. En effet, la taille des produits PCR de ce test ne varie que de quelques bases, rendant impossible l'identification de la famille V en question, ni du gène J réarrangé. La méthode de la présente invention permet donc de fournir un niveau d'information supérieur.

Analyse des résultats :

Partie A : On observe bien le nombre de bandes attendues sur témoins positifs (PBMC et Thymus). Les bandes correspondant aux réarrangements du gène D1 avec les gènes J1.1 et J1.2 sont fusionnées car les limites résolutives de la technologie utilisée ne permettent pas la séparation de bandes dont la différence de taille n'excède pas 10% de la taille de la plus grande bande. Un séquençage de cette bande, ou l'utilisation d'amorces marquées, serait nécessaire à la validation de cette observation.

Deux bandes très fortes apparaissent sur le témoin négatif. Ces bandes ne sont pas non spécifiques, mais correspondent à l'amplification de l'ADN germinal : les caractéristiques du locus couplées à la capacité de la technologie à amplifier des fragments de grande taille, font qu'un réarrangement n'est pas nécessaire pour observer un produit dans ce cas précis. Ces deux bandes constituent un très beau témoin interne de la présence et de la qualité de l'ADN testé, ainsi que de l'efficacité de l'enzyme.

Partie B : le procédé permet de caractériser très rapidement le réarrangement intermédiaire des lignées de lymphocytes T (voire même B). Dans l'exemple ci-dessus, le réarrangement incomplet D1-J1.3 pour la lignée cellulaire T appelée JURKAT, le réarrangement incomplet D1-J2.5 pour la lignée T MOLT4, et le réarrangement incomplet D2-J2.3 pour la lignée T HUT-78 ont été caractérisés sans besoin de séquençage supplémentaire.

Il est important de noter que les réarrangements incomplets D-J et D1-D2 sont non fonctionnels, mais représentent dans certains cas le seul marqueur biologique pour identifier une lymphoprolifération (leucémie ou lymphome B ou T).

**Exemple 8: Exemples de représentation des résultats d'une cartographie**

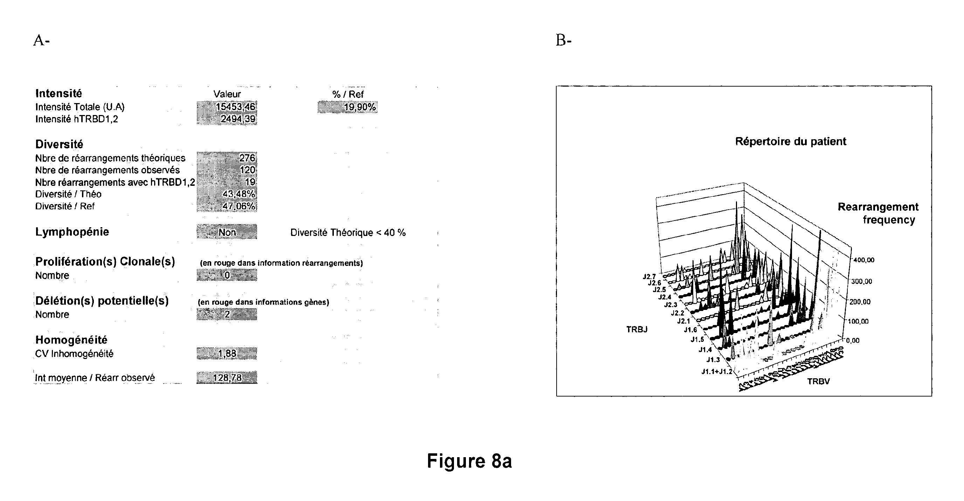

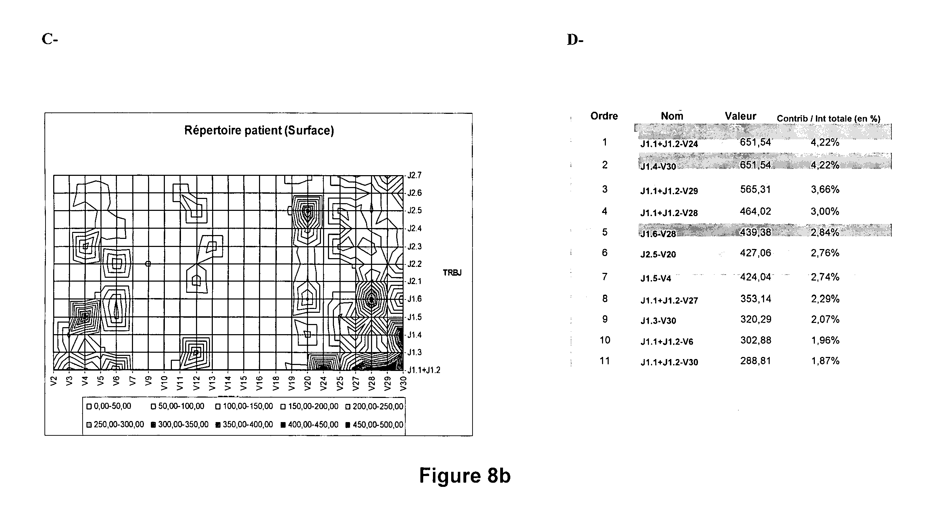

La figure 8 illustre un procédé permettant de regrouper toutes les données d'analyse du répertoire immunitaire sur 1 seule page, incluant notamment, la numération, et/ou la diversité de l'échantillon, et/ou l'intensité du signal, et/ou une comparaison avec une diversité de référence provenant d'un autre patient ou du même patient, une information permettant d'identifier que le patient a une lymphopénie en comparant le % de diversité obtenu avec un répertoire de référence, et/ou une information sur le nombre de clones détectés dans l'image, et/ou un graphique de représentation 2Dimensions, et/ou 3 dimensions, et/ou la liste de tous les réarrangements V-J classés par quantité décroissante en quantité de signal détecté, et/ou % de représentativité dans l'image. Le procédé dans ses étapes d'analyse permet ainsi

1- de compiler l'information collectée concernant l'historique clinique du patient et l'historique biologique de l'échantillon (résultats de numérations, cytométries, conditions de prélèvement...) ;
2- de fouiller les données par des méthodes statistiques et de corréler les résultats d'analyses du répertoire immunitaire (diversité combinatoire, ou autre approche) avec les données cliniques et biologiques. Il est notamment possible de classer tous les réarrangements V-D)J par ordre de fréquence de détection (intensité du signal de l'amplicon). Cet ordre varie d'un individu à l'autre en fonction des traitements et infections rencontrés par son répertoire de lymphocytes. Ceci permet d'avoir la signature du répertoire immunitaire d'une personne à un instant T. Cette signature peut être le marqueur biologique d'une pathologie, telle que les maladies auto-immunes, les allergies, les leucémies, les lymphomes, etc...

N.B. : il est important de noter que cette approche peut aussi être compatible avec d'autres approches d'analyses du répertoire immunitaire (toutes approches d'analyse de la diversité jonctionnelle, diversité d'appariement, diversité d'hypermutations somatiques, ...).

## Exemple 9 : Numération Diversité Lymphocytaire (NDL)

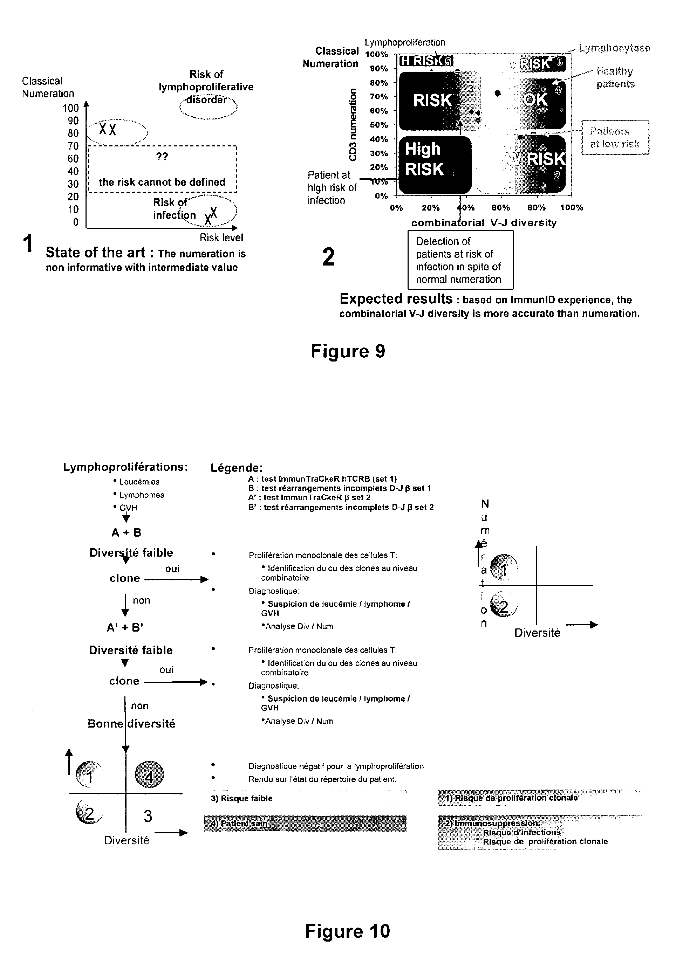

La numération lymphocytaire effectuée lors d'une NFS ou d'un marquage en cytométrie donne le nombre de lymphocytes dans un prélèvement. Ce nombre est utilisé pour vérifier que le patient ne présente pas d'immunodéficience. La fourchette de "normalité" est classiquement comprise entre 1000 et 3200 lymphocytes/$\mu$L de sang. En dessous de 1000, le patient est considéré comme légèrement immunodéprimé, au dessous de 450 il s'agit une immunosuppression sévère. A l'inverse, au dessus de 3200, le patient est considéré comme potentiellement à risque d'expansion lymphocytaire. Il existes 2 points faibles à cette approche : le premier est la taille de la fourchette elle-même, qui correspond à un facteur 3 entre les valeurs minimale et maximale. Le 2e point encore plus gênant est que cette numération ne préfigure pas de la diversité réelle du répertoire immunitaire du patient (figure 9, graphe de gauche). La présente invention permet de coupler une numération classique avec une mesure de la diversité combinatoire V-J (figure 9, graphe de droite).
Ceci peut notamment servir pour la sélection de patients dans le cadre d'essais cliniques, afin de tester des médicaments sur une population homogène, permettant d'interpréter les résultats.
Le graphique de la figure 9 distingue notamment les populations suivantes :

1. Numération faible (<1000 Lv/$\mu$L) et diversité combinatoire faible (<40%) : Il s'agit d'un patient immunosupprimé. Pour action : Ne pas inclure ce patient dans l'étude clinique. Faire suivre ce patient par un hématologue.
2. Numération faible (<1000 Ly/$\mu$L) mais diversité combinatoire V-J normale (>65%) : le patient a un faible taux de lymphocytes circulants par rapport aux autres populations de cellules immunitaires, mais la qualité de sa défense immunitaire spécifique n'est pas spécialement remise en cause.
Pour action: s'il s'agit d'une étude concernant les personnes âgées, il serait intéressant d'inclure un bras de patients ayant cette caractéristique.
3. Numération normale (1000-3200Ly/$\mu$L) et diversité combinatoire faible (<40%) : il existe des "trous" dans le répertoire immunitaire. D'apparence normale, cette numération lymphocytaire cache un trouble d'immunodéficience pouvant être associée à une ou plusieurs expansions clonales. L'efficacité vaccinale peut être remise en cause.
Pour action: Nous recommandons de ne pas inclure ce patient dans l'étude clinique. Faire suivre ce patient par un onco-hématologue.
4. Numération normale (1000-3200Ly/$\mu$L) et diversité normale (>65%) : le répertoire immunitaire est sain.
Pour action: le patient peut être inclus dans l'étude clinique.
5. Numération au dessus de la normale, et diversité faible : zone à fort risque, l'échantillon ne contient qu'un ou quelques clones de lymphocytes.
Pour action: Ne pas inclure ce patient dans l'étude clinique. Faire suivre ce patient par un onco-hématologue.
6. Numération au dessus de la normale, mais diversité normale : lymphocytose généralisée, l'ensemble du système immunitaire spécifique de l'individu est suractivé, mais aucun élément ne laisse entendre une expansion monoclonale pouvant être reliée à une leucémie ou un lymphome.

Pour action: Ne pas inclure ce patient dans l'étude clinique. Faire suivre ce patient par un onco-hématologue pour suivre l'évolution de la lymphocytose.

Cette nouvelle technique de numération, dite « Numération Diversité Lymphocytaire », couplant l'analyse du répertoire immunitaire à la numération (quelle que soit la méthode de comptage) du nombre de lymphocytes du patient, est donc largement plus informative que la simple numération des lymphocytes. Elle permet notamment d'éviter le paradoxe de la numération cellulaire qui par moment donne l'impression qu'un patient a un nombre de lymphocytes considéré comme normal, mais qui en réalité présente des clones T ou B et donc a une diversité immunitaire faible (zone 3). A l'inverse, cela permet de s'assurer que des patients ayant une faible numération, ont tout de même une diversité immunitaire « correcte » (zone 2) leur permettant de se défendre contre les infections et donc de ne pas leur imposer un suivi médical aussi lourd que pour des patients réellement immunodéprimés, c'est-à-dire avec une faible numération et une faible diversité (zone 1). En outre, cela permet de faire la distinction entre au moins 2 catégories de patients ayant une très grande numération de lymphocytes. La première catégorie (zone 5) a une diversité faible qui s'associe à la présence d'un ou plusieurs clones, pouvant être due à une leucémie, un lymphome, une GVHD, une maladie auto-immune, une allergie, une réponse à un vaccin ou tout autre thérapie et immunothérapie. La 2e catégorie (zone 6) correspond à une lymphocytose, c'est-à-dire une expansion de la majorité du répertoire immunitaire.

**Exemple 10 : stratégie de diagnostique personnalisé en onco-hématologie**

La figure 10 présente un arbre décisionnel en onco-hématologie par NDL.
Selon le schéma présenté, deux jeux d'amorces sont utilisés séquentiellement (le test avec le 2ème jeu étant optionnel) pour repérer les réarrangements VJ et DJ du TRB. Les amorces du 2ème jeu sont décalées par rapport à celles du 1er jeu, afin d'éviter de laisser échapper un clone à cause d'un polymorphisme apparu au niveau du site d'hybridation de l'amorce correspondante, ou d'une hypermutation somatique, etc.
Cette procédure permet de réaliser un diagnostic du niveau de risque de lymphoprolifération et en même temps du niveau de risque d'immunodéficence (à associer à un risque d'infection).

**Exemple 11 : comparaison de la diversité lymphocytaire de deux échantillons**

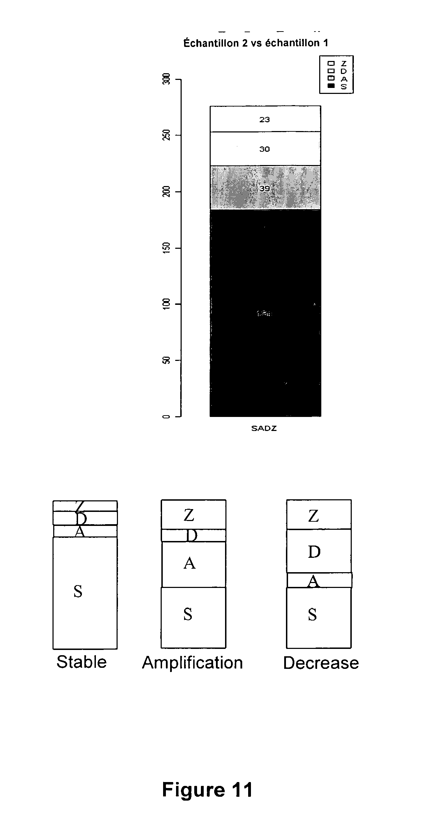

La figure 11 montre un graphique qui permet de comparer deux répertoires immunitaires afin d'identifier les ressemblances ou différences qui peuvent exister. Voici ci-dessous l'ordre de lecture et la description de chaque composante, ainsi qu'un exemple concret d'interprétation des résultats.
La région S pour « stable » représente le nombre de réarrangements observés dans les deux échantillons. La zone A pour « apparu » représente le nombre de réarrangements observés dans l'échantillon 2 mais pas dans l'échantillon 1. La zone D pour « disparu » représente le nombre de réarrangements qui sont observés dans l'échantillon 1 mais pas dans l'échantillon 2. Enfin, la zone Z pour « Jamais vu » représente le nombre de réarrangements qui ne sont observés ni dans l'échantillon 1 ni dans l'échantillon 2.
Interprétation : le nombre élevé de réarrangements observés en commun (184) dans les deux échantillons indique que les deux répertoires semblent identiques.
Le fait de représenter sur 1 graphique, la somme des apparitions, amplification A, disparition D, la non détection Z et la détection S (pour stable) de tous les réarrangements entre 2 cartographies immunitaire permet en 1 étape et de façon très visuelle de savoir si un patient est en cours de reconstitution (il aura beaucoup de A et un faible D) ou si il s'oriente vers une phase d'immunodéficience (il aura beaucoup de D et un faible A), enfin si le répertoire est stable entre les 2 cartographies (il y aura beaucoup de S).
Par ailleurs, le procédé décrit ici permet, en rapportant le nombre de réarrangements apparus ou disparus au temps écoulé entre les deux prélèvements d'échantillons, d'obtenir une indication sur la vitesse de reconstitution ou de diminution d'un répertoire. Ceci permet notamment de comparer l'effet stimulant ou à l'inverse immunosuppresseur d'un traitement.

**Exemple 12: détection précoce d'une lymphoprolifération (oligo)clonale, par PCR multi-n-plexe quantitative**

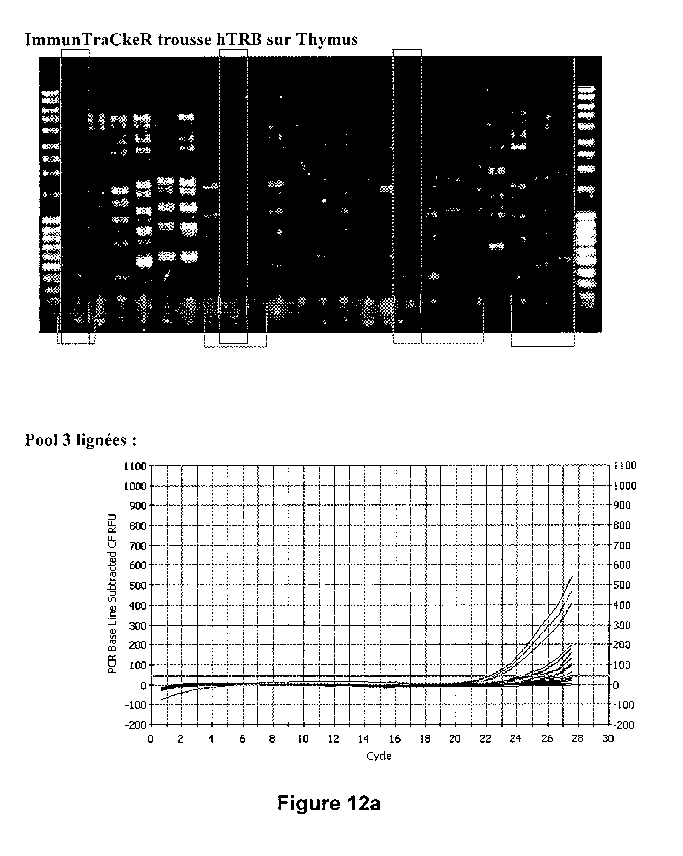

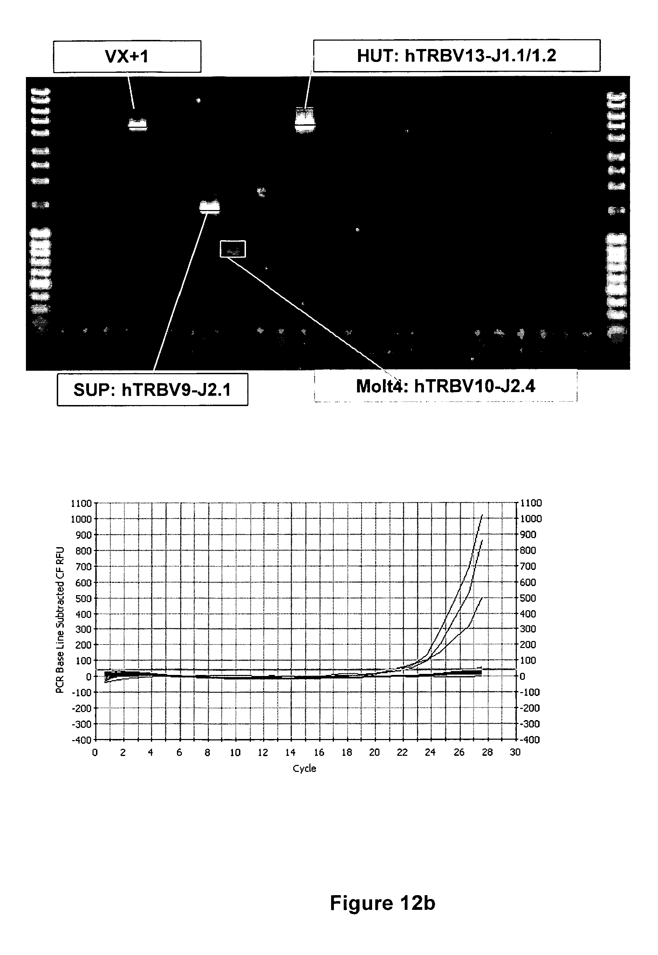

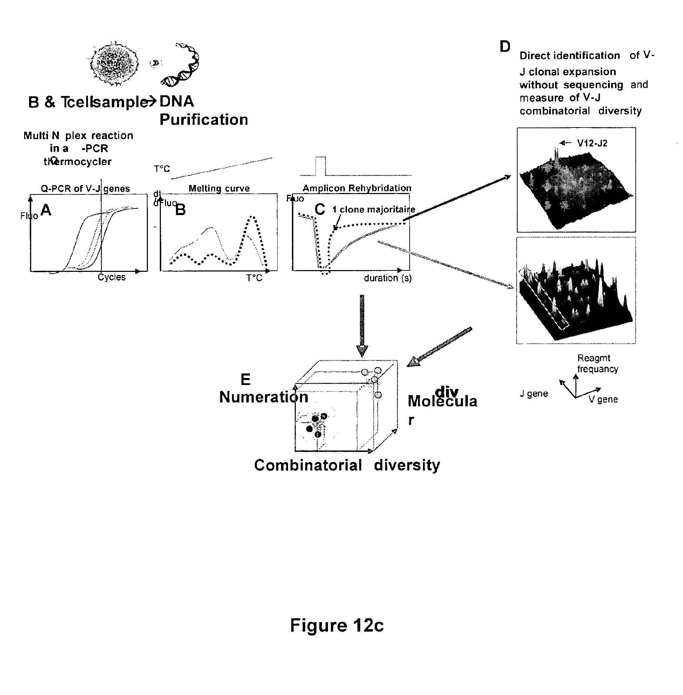

La figure 12 schématise un mode particulier de mise en oeuvre des procédés de l'invention, dans lequel les PCR multi-n-plexes sont suivies en temps réel, afin de détecter la présence d'une lymphoprolifération avant toute étape (optionnelle) de séparation des amplicons par électrophorèse.
L'amplification de PCR est effectuée dans un appareil de PCR quantitative en temps réelle, en utilisant par exemple le protocole décrit à l'exemple 2 ci-dessus, avec en plus une mesure de florescence à chaque cycle d'amplification.
Un mélange réactionnel spécifique, compatible à la fois avec la PCR quantitative et avec la PCR multi-n-plexe, a été mis au point pour cela. La polymérase utilisée est une enzyme de PCR longue telle que *Herculasell* ou *IProof.* Le problème majeur pour la mise au point de ce mélange réactionnel est que le *SYBR green* modifie la migration de l'ADNg de manière non linéaire. Le biais de migration est en partie proportionnel à la quantité de *SYBR green* utilisée, et en partie dépendant de la quantité de l'amplicon.

Dans le cas présent, le niveau de multiplexage important et les variations d'intensité (fréquence) entre les amplicons rendent difficile la mise au point de la PCR en temps réel.

Les inventeurs ont déterminé la quantité de *SYBR green* suffisante pour avoir assez de signal fluorescent pour la PCRq (figures 12a et 12b) : la réaction utilise comme base le même mélange réactionnel que celui présenté plus haut, avec en outre du *SYBR green* en quantité supérieure à0,4x final dans un volume réactionnel de 25μL, (soit 1μl à 10x initial) et inférieure à 2x final, avec une préférence pour 1x final. Cette quantité est considérée comme un maximum, puisque si cette quantité est trop importante, le *SYBR green* entraîne un biais dans la migration, sans compter l'inhibition de PCR multiplexe qui a été observée si la quantité est supérieure à 1,5x final.

Dans les cas où les PCR multi-n-plexes sont effectuées avec 1 amorce spécifique d'une famille V et au moins 2 amorces spécifiques des gènes J, cette étape, représentée au graphe A de la figure 12c, permet de détecter la présence d'une famille V majoritaire (le signal détecté correspond à la somme des tous les réarrangements V-J de la famille V étudiée de le tube de Q-PCR en question).

Il y a plusieurs types de résultats attendus :

- REPERTOIRE IMMUNITAIRE DIVERSIFIE : dans un échantillon de PBMC ou de thymus sain, en utilisant 50ng d'ADNg par Q-PCR, toutes les familles V sont généralement détectées à partir de 20 cycles et jusqu'à 27 cycles maximum.
  Dans un échantillon contenant aucun lymphocyte, aucun signal n'est détecté entre 20 et 27 cycles.

- REPERTOIRE COMPORTANT UN ou DES CLONE(S) T OU B MAJORITAIRE(S) : Dans un échantillon contenant majoritairement 1 clone V-(D)-J, la courbe correspondant à cette famille de gène V, sort entre 20 et 27 cycles, mais les courbes correspondant aux autres familles sont détectées au delà de 27 cycles. Ceci permet de distinguer une famille V majoritairement représentée dans un échantillon.
  De la même manière, si n familles V sont présentes en grande quantité dans l'échantillon, nous détectons n courbes dans la fenêtre 20-27 cycles, et les autres familles V sont détectées plus tardivement >27 cycles, voire pas du tout détectées.
  Ceci permet donc d'avoir, en temps réel et en moins de 2 heures, un diagnostic de lympho-prolifération sur 1 famille V au niveau génomique, à partir de la technique de PCR multi-n-plexe.
  Le graphe B de la figure 12c illustre l'étape optionnelle d'analyse de la courbe de fusion. Cette étape permet de confirmer la présence d'un amplicon majoritaire dans un tube de PCR en 1 phase de *melting curve* : montée en température de 40°C à 95°C (température de déshybridation totale de l'ADN). Durant cette phase, la fluorescence dans le tube est mesurée en continu. Si la courbe contient plusieurs pics de tailles similaires, c'est qu'il n'y a pas un amplicon majoritaire ; si à l'inverse 1 pic majoritaire est observé, cela vient appuyer le fait qu'un amplicon est majoritaire dans le tube de PCR en question.
  Le graphe C de la figure 12c schématise la mesure de la diversité moléculaire. Cette étape permet une confirmation supplémentaire de la présence d'un amplicon majoritaire dans 1 tube de PCR par la mesure de la diversité moléculaire, produite par la combinaison entre la diversité de jonction (CDR3), la diversité combinatoire (V-J) et la diversité issue des hypermutations somatiques.
  En bref, après avoir fait tomber la température très rapidement à 30° ou en-dessous, cette étape consiste à mesurer la vitesse de réhybridation des amplicons à température constante dans 1 tube de PCR, en mesurant la ré-émission de fluorescence.

- Dans le cas d'une grande diversité « moléculaire », le nombre d'amplicons différents dans 1 tube de PCR est grand et leur réhybridation est lente (de l'ordre de plusieurs couples de secondes, voir minutes) (courbe pleine). Si à l'inverse il n'y a qu'un amplicon majoritaire (avec 1 réarrangement V-J donné, 1 région de CDR3 donnée et des hypermutations somatiques données), la réhybridation de cet amplicon est rapide (de l'ordre de la seconde), ce qui produit alors une courbe plus verticale, comme celle représentée en pointillés.

- En résumé, cette étape de réhybridation peut être informative pour mesurer l'ordre de grandeur de la diversité « moléculaire » d'un échantillon, sans avoir besoin de faire migrer les produits de PCR. Plus la diversité moléculaire est grande et moins la somme des coefficients directeurs des courbes de chaque famille V est grande.

Au total, les 3 étapes A/B/C permettent d'utiliser les propriétés de la Q-PCR, pour identifier la présence d'une ou plusieurs familles V sur-représentés, sans faire migrer le produit de PCR multi-n-Plexe.

Néanmoins, il n'est pas possible de savoir quel est le nom du gène J sur-représenté, ni de mesurer la diversité combinatoire. Si le scientifique souhaite avoir ces informations supplémentaires, il doit réaliser une séparation des produits de PCR et analyser l'intensité des bandes correspondant à chacun des réarrangements (graphe D de la figure 12c).

Le graphe E de la figure 12c montre une synthèse graphique de la numération en fonction de la diversité combinatoire et moléculaire : connaissant la numération lymphocytaire de l'échantillon (mesurée indépendamment de cette expérience par comptage classique ou par cytométrie), il est possible de réaliser un graphique en 3 dimensions, avec la diversité combinatoire V-J, (en mesurant la somme des réarrangements V-J détectés), et la diversité moléculaire (en mesurant la somme des coefficients directeurs des pentes des familles de gènes V). Au final ce procédé permet de mieux caractériser un patient, en fonction de la diversité de son répertoire immunitaire.

En conclusion, ce procédé permet de suivre avec un niveau de finesse inégalé, l'évolution de la diversité immunitaire suite à un traitement. Il est notamment possible de diagnostiquer très précocement un niveau d'immunodéficience.

## Exemple 13 : mesure de l'efficacité d'un traitement

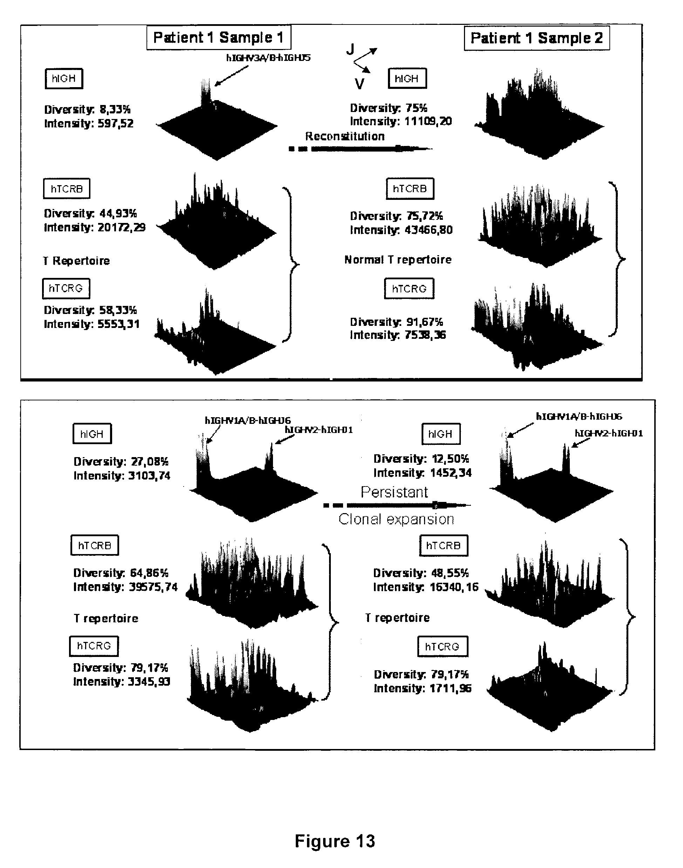

La figure 13 présente deux exemples d'analyse des répertoires IgH et TCR à différents temps, pour suivre l'évolution de ces répertoires chez des patients, en réponse à un traitement.

Dans le cas d'une leucémie lymphoïde chronique B (LLC-B), cette pathologie est diagnostiquée en identifiant un signal fort pour 1 ou seulement quelques clones B. L'approche résolutive développée par l'invention permet d'identifier le nom du gène V et J, sans avoir besoin de séquencer le produit de PCR. Ceci est particulièrement intéressant pour suivre la maladie résiduelle. La figure 13 présente 2 cas de figures : un pronostique positif (patient 1) et l'autre négatif (patient 2). En effet, si le traitement est efficace (patient répondant), le test mesure une augmentation de la diversité combinatoire IgH, ainsi qu'une stabilité, voire augmentation des chaînes du TCRb, TCRg. A l'inverse, pour les patients non répondant (NR), la diversité d'un, voir des 3 répertoires diminue. Le patient est plus immunodéprimé (immunosupprimé) après le traitement.

Dans le cas présent, les graphiques résultent des mesures de diversité combinatoire des chaines IgH, TCRb et TCRg.

Ces résultats montrent comment cette approche permet d'avoir le résultat de plusieurs tests en 1 seule étape à partir d'un seul prélèvement de moins de 1ml de sang : diagnostiquer la LLC, identifier de quelle population B ou T provient la lympho-prolifération, identifier le ou les clones impliqué(s) grâce à leur réarrangement V-J afin d'être capable de le suivre entre différents organes, et le cas échéant trouver l'origine de la pathologie. Par ailleurs, la caractérisation du ou des clone(s) majoritaire(s) rend possible un suivi longitudinal de la pathologie, permettant à terme de mesurer à la fois la présence et l'impact de la maladie résiduelle sur le système immunitaire. En parallèle s'ajoute la mesure fine du niveau de reconstitution du répertoire immunitaire en fonction du traitement du patient : grâce à ce type de test il est possible d'évaluer rationnellement le niveau d'immunodéficience global du patient et de le corréler au niveau de risque infectieux.

Cette méthode présente donc un intérêt à plusieurs titres :

- Faciliter le classement des patients
- Diagnostiquer et caractériser un clone B ou T, par le nom de son réarrangement V-J, notamment en analysant simultanément tous les répertoires lymphocytaires T & B,
- Pronostiquer l'évolution d'une pathologie en fonction du ou des clones V-J impliqués dans la pathologie.
- Suivre la maladie résiduelle et faire une comparaison avec la diversité combinatoire V-J.
- Affiner le suivi de l'évolution des patients ayant une lymphocytose.
- Comparer la pathologie entre différentes sources d'échantillon : sang, rate, ganglion et tâcher de préciser si possible «l'origine de la pathologie» en quantifiant la présence du clone V-J entre ces différentes populations.
- Avancer vers une corrélation du risque infectieux avec le niveau de Numération Diversité Lymphocytaire (NDL) de chaque patient.

## Exemple 14: répartition des patients en fonction de leur diversité TCT/IG et de leur numération

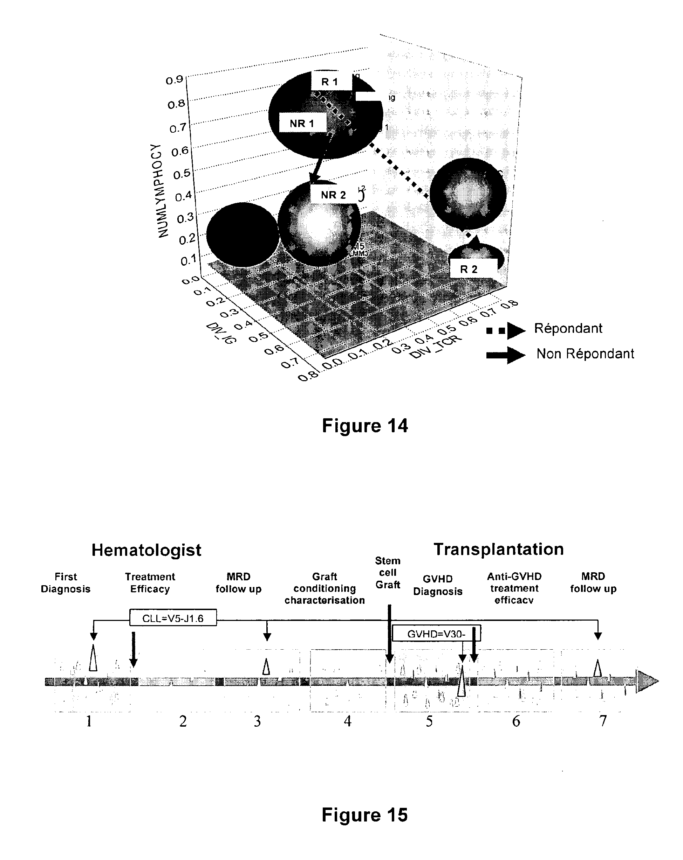

La figure 14 montre une représentation en 3 dimensions de la NDL (Numération/Diversité Lymphocytaire). Ce graphique regroupe la mesure de diversité des lymphocytes B et lymphocytes T, ainsi que la numération en % ou en valeur absolue du nombre de lymphocytes présents dans le prélèvement.

Le patient Répondant passe d'une zone à risque (avec une forte numération (env. 80%), une faible diversité IgH (8%) et une diversité moyenne voire faible pour le TCR (44%)) à une zone à moindre risque proche des prélèvements de PBMC (contrôle sain), avec une diminution de la numération (20%), une reconstitution de la diversité IgH (75%), TCRb (75%) et TCRg (90%).

A l'inverse, un patient Non Répondant (NR) reste dans la zone à risque et subit une immunosupression globale (diminution de la numération, qui ne résulte pas de l'efficacité du traitement, contrairement à ce que pourrait croire le clinicien s'il regardait seulement ce marqueur), une diminution du répertoire IgH, TCRb et TCRg.

Ceci illustre le paradoxe de la numération, dont l'utilisation seule peut aboutir à des erreurs d'interprétation. En effet, il est important de noter que la numération lymphocytaire entre ces deux prélèvements n'évolue pas dans le même sens que la diversité du répertoire combinatoire. Il est donc particulièrement intéressant de coupler les informations de numération et de diversité du répertoire afin de juger du réel état de santé du patient. En effet dans le cas présent, la numération leucocytaire passe de 78% à 21%, alors que la diversité combinatoire V-J passe de 8 à 75%. Cette approche permet de vérifier en une seule étape et à moindre coût que le patient en question a bénéficié d'un bon niveau de reconstitution du répertoire immunitaire et qu'il a moins de risque de déclarer une maladie infectieuse. Notons que ceci est cohérent avec la diminution de cellules CD19/CD5 indiquant la présence de la LCC qui passe de 84% à 4%.

### Exemple 15: immunomonitoring dynamique du diagnostic initial au suivi de greffe de cellules souches.

La figure 15 présente sous forme schématique l'application de la méthode de l'invention au suivi de la reconstitution du répertoire immunitaire chez un patient ayant subi une greffe de cellules souches (moelle).

Etape 1 : Mesure de la diversité immunitaire, diagnostic initial, détection du ou des clones de cellules T ou B (clones plus foncés).
Etape 2 : Evaluation de l'efficacité du traitement.
Etape 3 : Suivi de la maladie résiduelle.
Etape 4 : Evaluation de la préparation du répertoire du patient receveur (conditionnement de la greffe).
Etape 5 : Mesure de la reconstitution de la diversité V-J du patient et diagnostic précoce en cas de GVHD (maladie du greffon contre l'hôte).
Etape 6 : Evaluation de l'efficacité du traitement.
Etape 7 : Suivi de la maladie résiduelle.

L'étude longitudinale d'un patient par la méthode présentée ici permet un traitement personnalisé, adapté à chaque situation.
Le résultat du test présenté ici permet de mesurer le niveau de sévérité de la maladie et de s'assurer au maximum que le traitement est efficace, en évitant de traiter des patients qui seraient non répondants au traitement. Le répertoire immunitaire est ici utilisé comme un biomarqueur général de l'état de santé d'un individu. Il est utilisé à 2 niveaux : 1/ pour évaluer le risque infectieux du patient, 2/ tout en suivant de manière résolutive d'éventuels clones de lymphocytes T ou B pouvant être la signature d'une pathologie. Grâce à une étude longitudinale faite à partir de prises de sang successives chez un patient il est ainsi possible de suivre le niveau du répertoire immunitaire durant tout le traitement afin de s'assurer que ce dernier a bien été efficace et que le patient conserve un répertoire immunitaire diversifié pour se défendre contre les infections bactériennes ou virales. Ce diagnostic dynamique permet au clinicien d'adapter au mieux le traitement de son patient en lui proposant le médicament adéquat, à la bonne dose et au bon moment.
Un autre avantage du principe du test diagnostic préalable est d'éviter de trop traiter un patient « répondant » pour qui le traitement à la dose minimale est efficace.

### Exemple 16 : suivi d'un traitement *ex vivo* contre une GVHD

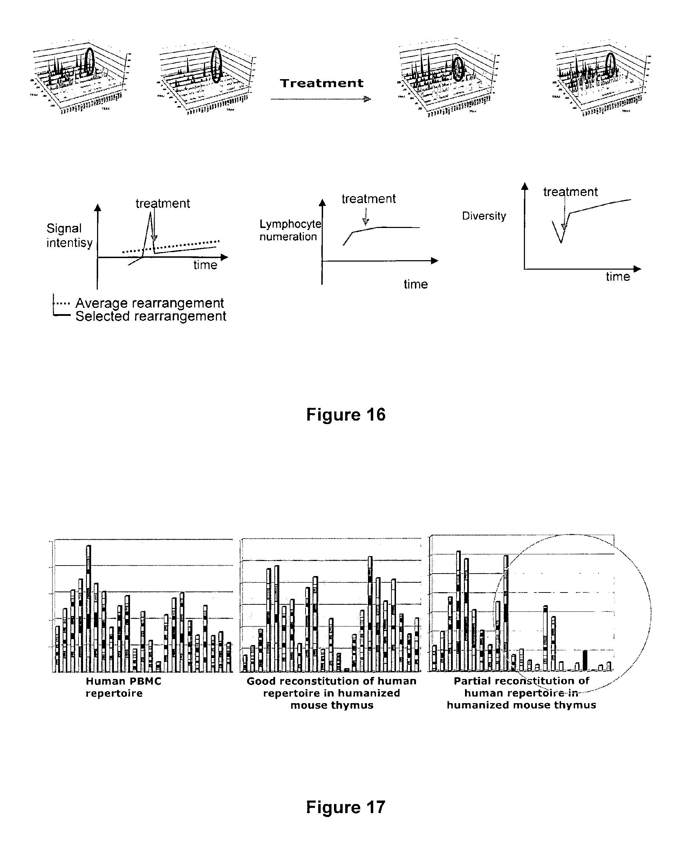

Les 4 cartographies de la figure 16 illustrent les étapes 5 et 6 de la figure précédente. Les 4 cartographies représentent la diversité du répertoire immunitaire mesuré à partir de prélèvements d'un même patient à 2 temps avant le traitement (environ 200, 250 jours), et deux temps post-traitement (environ 300 et 600 jours). Les résultats concernant ces 4 prélèvements sont représentés selon l'intensité, la numération et la diversité.

1. La diminution de la diversité du répertoire correspond bien à l'apparition d'un clone. Le traitement *ex vivo* semble avoir inhibé l'expansion clonale (qui représente près de 10% du répertoire combinatoire suivi). L'intensité correspondant à ce réarrangement est systématiquement plus forte que l'intensité moyenne des réarrangements.
2. La proportion de lymphocytes dans les PBMC (numération) atteint son maximum vers J300, puis diminue légèrement vers J600.
3. Le niveau de diversité combinatoire suit une tendance inverse. L'augmentation de la proportion de lymphocytes T ne correspond pas à une augmentation de la diversité. Contrairement à ce qui serait attendu, la diversité diminue entre le point 1 et le point 2.

Le traitement *ex-vivo* semble avoir inversé la tendance. La reconstitution se fait en 2 phases : le répertoire récupère rapidement un niveau de diversité de 35% vers J300, puis la reconstitution est plus lente et atteint environ 40% vers J600.
Ces résultats montrent donc un répertoire à la périphérie initialement restreint en comparaison des contrôles positifs sur un répertoire sain de thymus et sur 4 donneurs sains. L'utilisation de la méthode de l'invention permet de mesurer qu'après J600, le niveau de reconstitution du répertoire TCR est proche de celui des donneurs sains. Cette approche permet donc d'évaluer l'efficacité d'un traitement et de voir son impacte sur la cinétique de reconstitution immunitaire. Enfin, l'utilisation du procédé de la présente invention a permis de vérifier que le profil du répertoire combinatoire des donneurs pouvait être conservé durablement chez le receveur, d'où une attention plus systématique à accorder à l'analyse du répertoire des donneurs pour expliquer l'évolution d'une allogreffe au cours du temps.

La méthode présentée ici est donc un outil particulièrement intéressants pour les cliniciens pratiquant des greffes de moelle, puisqu'elle permet, entre autres, de décrire et suivre des expansions clonales correspondant à une *Graft Versus Host* (GVH) chez le receveur. Elle permet en outre de mieux suivre la reconstitution du répertoire post-greffe de moelle osseuse. Le clinicien sera alors en mesure d'adapter le traitement de manière personnalisée, en mesurant avec plus de finesse le rapport GVH/GVL grâce au suivi de la diversité combinatoire V-J.

## Exemple 17: Développement d'animaux transgéniques humanisés (ayant un répertoire immunitaire humain).

Les résultats sont présentés sur la figure 17.

Le présent procédé permet d'évaluer avec la trousse *ImmunTraCkeR* TRB humain, la qualité de reconstitution du répertoire immunitaire d'une souris transgénique « humanisée ». Dans cet exemple l'échantillon biologique étudié est issu de la rate d'une « souris immunodéficiente » (ne possédant pas de cellules immunitaires, ayant reçue une injection de cellules CD34+. Ces cellules ont la capacité de se diversifier et de reconstituer un système immunitaire. Le procédé permet de représenter la diversité du répertoire V-J dans un graphique 2 dimension. Chaque histogramme correspond à une famille V et au sein d'un histogramme, les sous-divisions correspondent à un gène J donné. La sous-division la plus basse de chaque histogramme correspond dans cet exemple à J2.7, et la plus haute correspond à J1.1. Il est possible de cribler les souris « humanisées », ayant un répertoire immunitaire pas totalement reconstitué (graphique de droite), des souris ayant un répertoire bien diversifié (graphique du centre), avec une répartition des réarrangements proche de celle observée dans un échantillon de PBMC humain (graphique de gauche).

## Exemple 18 : utilisation de la méthode comme outil de criblage *in vitro* de molécules

L'étude représentée par la figure 18 montre l'évaluation de l'efficacité d'un épitope (un antigène) dans le cas du développement d'un traitement (vaccin), en mesurant la décroissance de diversité du répertoire immunitaire d'un échantillon contenant des lymphocytes T. Il est montré que plus la diversité diminue, et plus la sélection des lymphocytes spécifiques à l'épitope est grande.

Sur cette figure, 3 cas sont représentés :

A : L'épitope testé conduit à une importante sélection lymphocytaire, comme indiqué par la décroissance de la diversité.

B : L'épitope testé est peu sélectif comme cela peut être observé en comparaison avec le contrôle négatif.

C : contrôle négatif

Ce test de criblage d'épitopes permet d'identifier les épitopes stimulant le répertoire immunitaire. Il est possible de corréler le nombre de pics (nb de clones T) avec l'efficacité de sélection et d'activation du répertoire immunitaire.

La figure 18 est issue d'une étude qualitative et quantitative du répertoire TCR Beta murin à partir de l'ADN génomique. En utilisant les amorces décrites à l'exemple 21 ci-dessous (trousse *ImmunTraCkeR* TRB souris), pour obtenir la mesure de la diversité combinatoire mTRBV-J et de l'intensité et de l'homogénéité du répertoire, il est possible d'évaluer sur ce modèle animal l'efficacité de différents protocoles vaccinaux

Dans un autre cas d'évaluation de l'efficacité d'un épitope, illustré par la figure 18, le modèle pour lequel le répertoire T est étudié correspond à une culture *in vitro* de lymphocytes. Dans ce cas, l'étude de la diversité du répertoire T sur des échantillons d'ADNg, provenant de culture de lymphocytes *in vitro,* permet de mesurer l'expansion clonale de certains gènes TCR alpha, beta, gamma, delta et hIgH suite à une présentation antigénique par des cellules présentatrices.

Bien sûr, à la sélection de l'antigène pour induire une réponse immunitaire sélective, s'ajoute la capacité d'identifier le meilleur mode d'injection (nombre et fréquence d'injection, site d'injection et dose).

La figure 18 illustre une autre utilisation, de la méthode présentée ici. En effet, la sélection d'épitope peut être un évènement non souhaité lors du développement de nouvelles approches thérapeutiques. Dans ce cas, il est important d'évaluer l'immunotoxicité (immunotoxicologie) pour éliminer les protéines induisant une activation du SI non attendue. Ceci permet de cribler des molécules, telles que les anticorps monoclonaux ou polyclonaux à visée thérapeutique, pour s'assurer qu'ils n'induisent pas une activation lymphocytaire inappropriée.

Ainsi, la méthode présentée ici est un outil particulièrement intéressant pour la recherche pré-clinique et clinique comme outils d'évaluation de nouvelles approches thérapeutiques et notamment vaccinales. Que ce soit sur des cellules en culture, chez l'animal modèle ou chez l'homme, l'outil ainsi décrit a pour objet de statuer sur la qualité de l'impact attendu sur le système immunitaire, soit par une activation spécifique ou par une absence d'activation.

Par extension, il est possible de faire le contrôle de la qualité du répertoire immunitaire en suivant un ou des clones caractérisé(s) pour des modèles d'études ou pour des productions de cultures de Lymphocytes avant réinjection (à visée thérapeutique) par exemple.

## Exemple 19: comparaison avec d'autres technologies existantes

La figure 19 montre la synthèse des résultats décrivant la comparaison de différentes techniques de suivi immunitaire (*immunomonitoring*) des répertoires des cellules immunitaires. Différents échantillons ont été étudiés par chacune des techniques. Les échantillons cellulaires ont été traités de façon contrôlée afin d'éviter tout biais dû à la préparation de l'échantillon.

Deux échantillons nommés « Thymus », correspondant à des cellules issues de thymus, ont été testés. Le Thymus est l'organe où a lieu la maturation et la sélection de certaines cellules immunitaires. Ces échantillons sont choisis car ils sont constitués d'un répertoire diversifié ne représentant pas de clonalité avec une présence des nombreuses cellules immunitaires ayant différents réarrangement (polyclonalité).

Deux autres échantillons ont été choisis pour représenter une population cellulaire beaucoup moins diverse que pour des échantillons de Thymus : l'échantillon « Jurkat » qui correspond à la lignée cellulaire jurkat (monoclonalité), ainsi que l'échantillon « Pool de Lignées T », qui correspond à un mélange dans des proportions connues de 3 lignées cellulaires (oligoclonalité). Enfin, un échantillon nommé « T(-) » correspond à témoin négatif, qui est un mélange cellulaire ne comportant pas de cellule immunitaire.

Ont été étudiées, en plus de la présente méthode avec la trousse « ImmunTraCkeR »» TRB humain conforme à l'invention, 2 techniques permettant d'apporter différentes informations sur la qualité du répertoire immunitaire. D'une part, le test cellulaire « *lotests* » (société Beckmann Coulter) qui est basé sur la reconnaissance d'antigène du répertoire Vbeta par cytométrie en flux, grâce à une trousse d'anticorps spécifiques. Ce test permet d'identifier la présence de 72% des familles et 59% des membres V. D'autre part, un test de biologie moléculaire « Biomed-2 » (consortium européen Biomed-2), qui est basé sur des approche de PCR multiplexées selon un principe différent de la présente invention, qui permet d'identifier si dans un échantillon donné il y a un clone très largement représenté.

L'analyse des résultats observés permet d'identifier que le test « ImmunTraCkeR » permet d'identifier lors de la mesure de la PCR en temps réel si l'échantillon est clonal (« Jurkat », oligoclonal (« Pool lignées T ») ou polyclonal (« Thymus »). L'analyse montre également que l'analyse des amplicons (réarrangements amplifiées selon la méthode « ImmunTraCkeR ») permet d'observer précisément quels clones sont présent en terme de réarrangement Vx-Jy, sans étape de séquençage, que se soit pour l'échantillon (« Jurkat », oligoclonal (« Pool lignées T ») ou polyclonal (« Thymus »). La représentativité de la diversité des combinaisons observées est donnée en pourcentage (par rapport à une diversité théorique où 100 % des réarrangements Vx-Jy seraient observés).

Le test « Biomed-2 » permet bien d'identifier la présence très majoritaire d'un clone dans l'échantillon. Le test « Biomed-2 » ne permet pas de discerner entre la présence d'un ou de quelques clones si le cas se présente, et ce test aura une signature montrant que l'échantillon est clonal même si il y a une oligoclonalité (« Pool lignées T ») : le test est positif (« On ») dans les deux cas.

Le test « lotests », qui est un test effectué à partir des cellules fraiches, permet de discerner la clonalité de l'oligoclonalité et de la polyclonalité. Cependant, à la différence du test « ImmunTraCkeR », seulement les segments V beta sont étudiés. En outre, l'exhaustivité des anticorps Vbeta n'étant pas atteinte dans la trousse d'anticorps de ce test, le réarrangement V4 n'est pas observé alors qu'il l'est avec le test « ImmunTraCkeR ».

Les résultats de cette étude présentés figure 19 montrent que le test « ImmunTraCkeR » a une capacité technique plus grande que les deux autres test évalués pour l'analyse des répertoires immunitaires et qu'en conséquence ce test permet une étude plus étendue des répertoires immunitaires.

## Exemple 20 : protocole de production de tests hTCRB

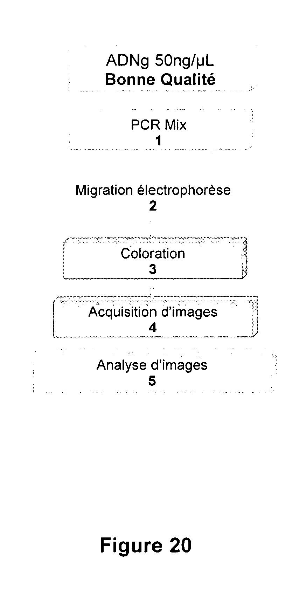

Pour utiliser la méthode qui est décrite dans ce document, et réaliser des analyses avec une des trousse « *ImmunTraCkeR* » dans de bonnes conditions, en plus de suivre les étapes de réalisation du test (présentés figure 20) il est préférable d'avoir produit le test en suivant un protocole de production et selon les bonnes pratiques de production.

Pour réaliser une production contrôlée de kits permettant de réaliser des tests hTRbeta (ou trousse « *immunTraCkeR* » TRbeta humain), il est nécessaire de s'assurer que la personne qui doit effectuer une production du test pour une utilisation à terme dans une étude sur des échantillons, respecte les différentes étapes énoncées ci-après en fonction de l'utilisation souhaitée.

Production 12 Tests hTRBeta

Préparation du « mix oligos »
Préparation du matériel
1 plaque Greiner (support)
3 barrettes de 8 puits + bouchons
microtubes de 0,5mL et 1,5mL
pipettes, embouts
$H_2O$ stérile
EB

Dilution des Vβ :

100μM à 20μM

$$\text{Calcul : } 20 \; x \; Vf \; / \; 100 = Vi$$

Donc, dans microtube 0,5mL: Vi μL d'oligo à 100μM + (Vf-Vi) d'EB.
20μM à 3,5μM
Calcul du volume nécessaire pour la production de 12kits + 10% :

$$12*1.67 = 20.04 + 10\% = 22μL$$

$$\text{Calcul : } 3,5 \; x \; 22 \; / \; 20 = 3,86μL$$

Donc, dans microtube 0.5mL: 3,85μL d'oligo à 20μM + 18,15μL d'$H_2O$

Dilution de bc1do2 & 2S7do1 :

100μM à 20μM

$$\text{Calcul : } 20 \; x \; Vf \; / \; 100 = Vi$$

Donc, dans microtube 0,5mL: Vi μL d'oligo à 100μM + (Vf-Vi) d'EB. 20μM à 3,5μM
Calcul du volume nécessaire pour la production de 12kits + 10% : 23*12*1,67= 461+10%= 507,1μL

$$\text{Calcul : } 3,5 \; x \; 507.1 \; / \; 20 = 88,74μL$$

Dans tube Eppendorf de 1,5mL: 88,74μL d'oligo à 20μM + 418,36μL d'$H_2O$

Répartition des oligos Bc1do2 & 2S7do1

Pool Bc1do2 & 2S7do1 dilués à 3,5μM dans un tube à hémolyse stérile
Distribuer 44.1μL de ce pool dans chaque microtube 0,5mL contenant les Vβ dilués
Mélanger par refoulement, vortexer intensément puis centrifuger brièvement.

Répartition des oligos Vβ-Bc1do2-2S7do1

Distribuer 66μL du mix « Vβ-Bcldo2-2S7dol » dans les barrettes de 8 puits en conservant l'ordre des Vβ suivant :

Barrette n°1 : Vβ2up2, Vβ3up2, Vβ4up_ex, Vβ5pool, Vβ6pool, Vβ7pool, Vβ9up_ex, Vβ10pool

Barrette n°2: Vβ11up_ex, Vβ2pool, Vβ13up1, Vβ14up_ex, Vβ15up_ex. Vβ16up1, Vβ18up1, Vβ19up2

Barrette n°3 : Vβ20-1up_ex, Vβ24up_ex, Vβ25up_int, Vβ27up2, Vβ28up_G, Vβ29up_G, Vβ30up1

Préparation des kits

Préparation du matériel

3 plaque Greiner (support)

3*12 barrettes de 8 puits + bouchons

pipettes, embouts

Production de 12 tests

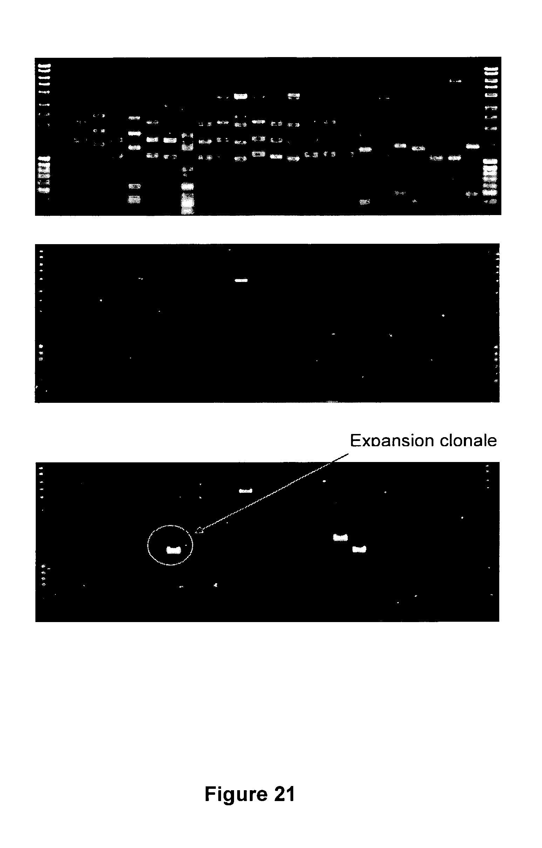

A l'aide de la pipette multicanal, distribuer 5μL de mix des barrettes 1 à 3 (vol/puits=64μL) dans chaque puits des 12 barrettes correspondants. Pour un lot de n tests, au moins deux tests seront utilisés pour contrôler la qualité et la conformité de la production. Parmi les différents contrôles effectués tout au long de la production pour permettre la traçabilité et la maîtrise de toutes dérives sur un lot, les contrôles fonctionnels qui permettent de vérifier la qualité avant validation du lot sont importants. A ce titre, la figure 21 propose des exemples sur trois échantillons de contrôle d'un test issu d'un lot de production. Ces trois échantillons sont ceux sur lesquels un contrôle systématique est réalisé au cours des productions. Les résultats sont comparés systématiquement aux précédents résultats pour identifier tout écart. Chaque nouveau résultat permet d'affiner la tendance et les limites hautes et basses acceptables pour que le test puisse être utilisé dans une étude du répertoire immunitaire.

Sur la figure 21, de haut en bas, est représenté le résultat attendu pour un répertoire immunitaire de thymus (polyclonalité), un répertoire immunitaire lorsque l'échantillon ne comporte qu'un clone très majoritaire (clonalité) et enfin un répertoire immunitaire lorsque l'échantillon comporte plusieurs clones (oligoclonalité).

Le protocole ci-dessus peut être adapté pour produire des tests TRB souris avec la trousse d'oligonucléotides tels que décrits dans l'exemple 21 ci-après, ainsi que pour toute autre production de test.

### Exemple 21 : Application de la méthode à l'analyse du locus TRB chez la souris

Les protocoles et méthodes décrits dans le présent texte peuvent être adaptés à l'étude du répertoire TRB murin, en utilisant les amorces décrites dans le tableau ci-dessous.

Tableau 15

| nom du gène du | nom oligonucléotide nom | taille (nt) | distance avec la fin du gène V en pb | Orientation OLIGONUCLÉOTIDE |
|---|---|---|---|---|
| TRBV1 | mTRBV1up1 | 23 | 153 | SENS |
| TRBV2 | mTRBV2up1 | 28 | 135 | SENS |
| TRBV3 | mTRBV3up1 | 26 | 292 | SENS |
| TRBV4 | mTRBV4up1 | 25 | 124 | SENS |
| TRBV5 | mTRBV5up1 | 24 | 227 | SENS |
| TRBV12 | mTRBV12upl | 25 | 105 | SENS |
| TRBV13 | mTRBV13up1 | 22 | 287 | SENS |
| TRBV14 | mTRBV14up1 | 28 | 130 | SENS |
| TRBV15 | mTRBV15up1 | 24 | 362 | SENS |
| TRBV16 | mTRBV16up1 | 23 | 62 | SENS |
| TRBV17 | mTRBV17up1 | 25 | 108 | SENS |
| TRBV19 | mTRBV19up1 | 27 | 113 | SENS |
| TRBV20 | mTRBV20up1 | 25 | 199 | SENS |
| TRBV23 | mTRBV23up1 | 27 | 376 | SENS |
| TRBV24 | mTRBV24up1 | 27 | 61 | SENS |
| TRBV26 | mTRBV26up1 | 24 | 395 | SENS |

(suite)

| nom du gène du | nom oligonucléotide nom | taille (nt) | distance avec la fin du gène V en pb | Orientation OLIGONUCLÉOTIDE |
|---|---|---|---|---|
| TRBV29 | mTRBV29up1 | 24 | 99 | SENS |
| TRBV30 | mTRBV30up2 | 23 | 155 | SENS |
| TRBV31 | mTRBV31up1 | 24 | 73 | SENS |
| nom du gène | taille nom oligonucléotide | (pb) | distance avec le début du gène J en pb | Orientation OLIGONUCLÉOTIDE |
| TRBJ1.7 | mTRBJ1.7do1 | 26 | 2315 | ANTI-SENS |
| TRBJ2.7 | mTRBJ2.7do1 | 22 | 241 | ANTI-SENS |

## REFERENCES

Aude-Garcia, C., Gallagher, M., Marche, P. N., and Jouvin-Marche, E. (2001). Preferential ADV-AJ association during recombination in the mouse T-cell receptor alpha/delta locus. Immunogenetics 52, 224-230.

Baum, P. D., and McCune, J. M. (2006). Direct measurement of T-cell receptor repertoire diversity with AmpliCot. Nat Methods 3, 895-901.

Baum, T. P., Hierle, V., Pasqual, N., Bellahcene, F., Chaume, D., Lefranc, M. P., Jouvin-Marche, E., Marche, P. N., and Demongeot, J. (2006). IMGT/GeneInfo: T cell receptor gamma TRG and delta TRD genes in database give access to all TR potential V(D)J recombinations. BMC Bioinformatics 7, 224.

Baum, T. P., Pasqual, N., Thuderoz, F., Hierle, V., Chaume, D., Lefranc, M. P., Jouvin-Marche, E., Marche, P. N., and Demongeot, J. (2004). IMGT/GeneInfo: enhancing V(D)J recombination database accessibility. Nucleic Acids Res 32, D51-54.

Berek, C., Griffiths, G. M., and Milstein, C. (1985). Molecular events during maturation of the immune response to oxazolone. Nature 316, 412-418.

Bogue, M., Gilfillan, S., Benoist, C., and Mathis, D. (1992). Regulation of N-region diversity in antigen receptors through thymocyte differentiation and thymus ontogeny. Proc Natl Acad Sci U S A 89, 11011-11015.

Bonarius, H. P., Baas, F., Remmerswaal, E. B., van Lier, R. A., ten Berge, 1. J., Tak, P. P., and de Vries, N. (2006). Monitoring the T-cell receptor repertoire at single-clone resolution. PLoS ONE 1, e55.

Cabaniols, J. P., Fazilleau, N., Casrouge, A., Kourilsky, P., and Kanellopoulos, J. M. (2001). Most alpha/beta T cell receptor diversity is due to "terminal deoxynucleotidyl transferase. J Exp Med 194, 1385-1390.

Chaudhuri, J., Tian, M., Khuong, C., Chua, K., Pinaud, E., and Alt, F. W. (2003). Transcription-targeted DNA deamination by the AID antibody diversification enzyme. Nature 422, 726-730.

Cochet, M., Pannetier, C., Regnault, A., Darche, S., Leclerc, C., and Kourilsky, P. (1992). Molecular détection and in vivo analysis of the specific T cell response to a protein antigen. Eur J Immunol 22, 2639-2647.

Davis, M. M., and Bjorkman, P. J. (1988). T-cell antigen receptor genes and T-cell recognition. Nature 334, 395-402.

Douek, D. C., McFarland, R. D., Keiser, P. H., Gage, E. A., Massey, J. M., Haynes, B. F., Polis, M. A., Haase, A. T., Feinberg, M. B., Sullivan, J. L., et al. (1998). Changes in thymic function with age and during the treatment of HIV infection. Nature 396, 690-695.

Fugmann, S. D., Lee, A. I., Shockett, P. E., Villey, I. J., and Schatz, D. G. (2000). The RAG proteins and V(D)J recombination: complexes, ends, and transposition. Annu Rev Immunol 18, 495-527.

Fuschiotti, P., Pasqual, N., Hierle, V., Borel, E., London, J., Marche, P. N., and Jouvin-Marche, E. (2007). Analysis of the TCR alpha-chain rearrangement profile in human T lymphocytes. Mol Immunol 44, 3380-3388.

Hamblin, T. J., Davis, Z., Gardiner, A., Oscier, D. G., and Stevenson, F. K. (1999). Unmutated Ig V(H) genes are associated with a more aggressive form of chronic lymphocytic leukemia. Blood 94, 1848-1854.

Huang, C., and Kanagawa, O. (2001). Ordered and coordinated rearrangement of the TCR alpha locus: role of secondary rearrangement in thymic selection. J Immunol 166, 2597-2601.

Jouvin-Marche, E., Aude-Garcia, C., Candeias, S., Borel, E., Hachemi-Rachedi, S., Gahery-Segard, H., Cazenave, P. A., and Marche, P. N. (1998). Differential chronology of TCRADV2 gene use by alpha and delta chains of the mouse TCR. Eur J Immunol 28, 818-827.

Kotani, A., Okazaki, I. M., Muramatsu, M., Kinoshita, K., Begum, N. A., Nakajima, T., Saito, H., and Honjo, T. (2005). A target selection of somatic hypermutations is regulated similarly between T and B cells upon activation-induced cytidine deaminase expression. Proc Natl Acad Sci U S A 102, 4506-4511.

Lang, R., Pfeffer, K., Wagner, H., and Heeg, K. (1997). A rapid method for semiquantitative analysis of the human V beta-repertoire using TaqManR PCR. J Immunol Methods 203, 181-192.

Lefrancs, The Immunoglobulin Facts Book 2001.

Lefrancs The T cell receptor Facts Book 2001.

Oprea, M., and Kepler, T. B. (1999). Genetic plasticity of V genes under somatic hypermutation: statistical analyses using a new resampling-based methodology. Genome Res 9, 1294-1304.

Pannetier, C., Even, J., and Kourilsky, P. (1995). T-cell repertoire diversity and clonal expansions in normal and clinical samples. Immunol Today 16, 176-181.

Pasqual, N., Gallagher, M., Aude-Garcia, C., Loiodice, M., Thuderoz, F., Demongeot, J., Ceredig, R., Marche, P. N., and Jouvin-Marche, E. (2002). Quantitative and qualitative changes in V-J alpha rearrangements during mouse thymocytes differentiation: implication for a limited T cell receptor alpha chain repertoire. J Exp Med 196, 1163-1173.

Pham, T., Belzer, M., Church, J. A., Kitchen, C., Wilson, C. M., Douglas, S. D., Geng, Y., Silva, M., Mitchell, R. M., and Krogstad, P. (2003). Assessment of thymic activity in human immunodeficiency virus-negative and - positive adolescents by real-time PCR quantitation of T-cell receptor rearrangement excision circles. Clin Diagn Lab Immunol 10, 323-328.

Rytkonen, M. A., Hurwitz, J. L., Thompson, S. D., and Pelkonen, J. (1996). Restricted onset of T cell receptor alpha gene rearrangement in fetal and neonatal thymocytes. Eur J Immunol 26, 1892-1896.

Van den Beemd, van Dongen et al. (2000), "Flow cytometric detection of clonality in mature T-cell malignancies by use of a Vb antibody kit", ISAC Abstract.

Wang, F., Huang, C. Y., and Kanagawa, O. (1998). Rapid deletion of rearranged T cell antigen receptor (TCR) Valpha-Jalpha segment by secondary rearrangement in the thymus: role of continuous rearrangement of TCR alpha chain gene and positive selection in the T cell repertoire formation. Proc Natl Acad Sci U S A 95, 11834-11839.

**Revendications**

1.  Méthode d'analyse *in vitro* de la diversité du répertoire des lymphocytes T et/ou B d'un individu, à partir d'ADN génomique provenant d'un échantillon biologique dudit individu, comprenant les étapes suivantes :

   A) amplification de fragments dudit ADN génomique par des réactions d'amplification en chaîne par polymérase (ACP ou PCR) multiplexes, dont l'une au moins est une PCR multi-n-plexe avec n≥2, effectuée avec une combinaison d'au moins 3 amorces, constituant au moins 2 couples d'amorces présentant les caractéristiques suivantes :

      (i) chaque couple d'amorces est constitué d'une amorce s'hybridant spécifiquement en amont et/ou dans un gène V ou D donné et d'une amorce s'hybridant spécifiquement en aval et/ou dans un gène J donné, de façon à permettre l'amplification d'au moins deux fragments caractéristiques de deux réarrangements V(D)J ou D-J distincts;
      (ii) les amorces sont thermodynamiquement compatibles ;
      (iii) les amorces sont choisies de telle façon que les fragments amplifiés avec le premier couple d'amorces peuvent être distingués des fragments amplifiés avec le second couple d'amorces ;

B) détection des produits d'amplification obtenus à l'étape A ;
C) interprétation des résultats.

**2.** Méthode selon la revendication 1, pour l'analyse de la diversité combinatoire des réarrangements V(D)J d'au moins un locus génétique choisi parmi les loci TRA, TRB, TRG, TRD, IGH, IGK et IGL.

**3.** Méthode selon la revendication 1 ou la revendication 2, dans laquelle au moins une amorce de chaque couple d'amorces est marquée.

**4.** Méthode selon l'une quelconque des revendications précédentes, dans laquelle l'étape B) comporte une étape de mesure en temps réel de l'amplification des fragments d'ADN, et dans laquelle l'étape C) est effectuée de la façon suivante :

(i) si une ou quelques courbes, en nombre inférieur à la moitié des courbes, présentent un décalage par rapport aux autres courbes, tel que les autres courbes présentent un point d'inflexion au moins 2 cycles après le point d'inflexion de la première courbe, de préférence au moins 3 ou 4 cycles après le point d'inflexion de la première courbe, ou ne montrent aucune amplification, le résultat indique la présence d'une lymphoprolifération clonale ou oligoclonale ;
(ii) si, au contraire, toutes les courbes présentent un point d'inflexion au même cycle, ou dans un décalage maximum de 2 ou 3 cycles d'amplification, le résultat permet d'écarter l'hypothèse d'une lymphoprolifération d'un clone résultant d'un des réarrangements correspondant aux fragments amplifiés.

**5.** Méthode selon la revendication 4, comportant en outre, une étape de confirmation d'une lymphoprolifération par la mesure continue de la fluorescence dans chaque tube au cours d'une montée en température entre 40°C et 95°C, l'observation d'un pic majoritaire étant indicative de la présence d'un amplicon majoritaire et donc d'une lympho-prolifération, alors que l'observation de plusieurs pics de tailles similaires indique au contraire une diversité lymphocytaire.

**6.** Méthode selon la revendication 4 ou la revendication 5, comportant en outre une étape de mesure de la diversité moléculaire des réarrangements observés, par mesure de la diversité moléculaire des amplicons, de la façon suivante :

(i) après déshybridation des amplicons à 95°C, la température des produits d'amplification est rapidement ramenée à 30°C ou en dessous ;
(ii) la fluorescence est mesurée régulièrement et de préférence en continu au cours de la réhybridation ;
(iii) une réhybridation rapide est indicative de la présence d'un amplicon majoritaire, et donc d'une lymphoprolifération clonale ou oligoclonale, alors qu'une réhybridation plus lente est indicative d'une plus grande diversité moléculaire.

**7.** Méthode selon l'une quelconque des revendications précédentes, dans laquelle l'étape B) de détection des produits d'amplification comprend une étape de séparation desdits produits suivant leur taille.

**8.** Méthode selon l'une quelconque des revendications précédentes, dans laquelle les couples d'amorces mis en oeuvre dans chaque réaction de PCR multi-n-plexe avec n≥2 sont choisis de façon à ce qu'au moins certains des produits de l'amplification par un couple d'amorces présentent des tailles différentes des produits de l'amplification par l'autre ou les autres couple(s) d'amorces.

**9.** Méthode selon l'une quelconque des revendications précédentes, dans laquelle au moins une PCR multi-n-plexe avec n≥2 est effectuée en utilisant une combinaison d'au moins 3 amorces constituant au moins 2 couples d'amorces comportant une amorce sens commune s'hybridant spécifiquement en amont et/ou dans un gène V donné, chaque couple d'amorces comportant en outre une amorce antisens s'hybridant spécifiquement en aval et/ou dans un gène J donné.

**10.** Méthode selon la revendication 9, dans laquelle plusieurs PCR multi-2-plexes sont effectuées avec des triplets d'amorces constitués chacun d'une amorce sens s'hybridant spécifiquement en amont et/ou dans un gène V donné et de deux amorces antisens s'hybridant spécifiquement en aval et/ou dans deux gènes J distincts.

**EP 2 062 982 A1**

**11.** Méthode selon l'une quelconque des revendications 1 à 8, dans laquelle au moins une réaction de PCR multi-n-plexe avec n≥2 est effectuée en utilisant une combinaison d'au moins 3 amorces constituant au moins 2 couples d'amorces comportant une amorce antisens commune s'hybridant spécifiquement en aval et/ou dans un gène J donné chaque couple d'amorces comportant en outre une amorce sens s'hybridant spécifiquement en amont et/ou dans un gène V donné.

**12.** Méthode selon l'une quelconque des revendications 1 à 10, dans laquelle au moins une réaction de PCR multi-n-plexe avec n≥2 est effectuée pour analyser certains réarrangements du locus TRB, en utilisant une combinaison d'au moins 3 amorces constituant au moins 2 couples d'amorces présentant les caractéristiques suivantes :

(i) les deux couples d'amorces comportent une amorce sens commune s'hybridant spécifiquement en amont et/ou dans un gène V donné et comportent chacun une amorce antisens s'hybridant spécifiquement en aval et/ou dans un gène J donné ;
(ii) les deux amorces antisens s'hybrident spécifiquement en aval et/ou dans deux gènes $J_y$ et $J_z$ appartenant à deux groupes distincts de gènes J du locus TRB ; et
(iii) la distance entre la région d'hybridation de l'amorce antisens spécifique du gène $J_y$ et le début dudit gène $J_y$ est supérieure à la distance entre la région d'hybridation de l'amorce antisens spécifique du gène $J_z$ et le début du premier gène J du groupe de gènes dudit gène $J_z$.

**13.** Méthode selon la revendication 12, dans laquelle au moins une réaction de PCR multi-n-plexe avec n≥2 est effectuée pour analyser certains réarrangements du locus TRB humain, en utilisant une combinaison d'au moins 3 amorces comprenant une amorce sens s'hybridant spécifiquement en amont et/ou dans un gène V donné et les amorces hTRBJ1.6 et hTRJB2.7 définies de la façon suivante :

- hTRBJ1.6 est un oligonucléotide antisens de 25 nucléotides s'hybridant entre les nucléotides 2341 et 2365 du gène J1.6 du locus TRB ; et
- hTRBJ2.7 est un oligonucléotide antisens de 22 nucléotides s'hybridant entre les nucléotides 214 et 235 du gène J2.7 du locus TRB.

**14.** Méthode selon la revendication 12 et la revendication 13, permettant l'analyse des réarrangements V(D)J du locus TRB humain en effectuant 24 PCR multi-n-plexes avec n≥2 à l'aide de combinaisons d'au moins 3 amorces, chaque combinaison d'amorces comprenant les amorces hTRBJ1.6, hTRBJ2.7 définies à la revendication 13 et au moins une amorce hTRBV choisie parmi les amorces définies dans le tableau 2 ci-dessous :

Tableau 2

| Nom du gène | Nom de l'amorce | Taille (nt) | Distance entre l'extrémité 5' de l'amorce et la fin du gène V (pb) |
|---|---|---|---|
| TRBV2 | hTRBV2up2 | 26 | 255 |
| TRBV3 | hTRBV3up2 | 23 | 57 |
| TRBV4 | hTRBV4up_ex | 23 | 100 |
| TRBV5.1, 3, 4, 5, 6, | hTRBV5up_ex1/2 | 25 | 256 |
| TRBV5.7 | hTRBV5up_ex2/2 | 25 | 256 |
| TRBV6.1,2,3, 5, 6,7,8,9 | hTRBV6up_ex1/2 | 25 | 281 |
| TRBV6.4 | hTRBV6up_ex2/2 | 23 | 279 |
| TRBV7.1,3,4,5,6,7,8 | hTRBV7up_ex1/3 | 25 | 301 |
| TRBV7.2 | hTRBV7up_ex2/3 | 25 | 301 |
| TRBV7.9 | hTRBV7up_ex3/3 | 27 | 303 |
| TRBV9 | hTRBV9up_ex | 23 | 92 |
| TRBV10.1,3 | hTRBV10.1,3up1 | 26 | 162 |
| TCRBV10.2 | hTRBV10.2up1 | 27 | 162 |

38

(suite)

| Nom du gène | Nom de l'amorce | Taille (nt) | Distance entre l'extrémité 5' de l'amorce et la fin du gène V (pb) |
|---|---|---|---|
| TRBV11 | hTRBV11up_ex | 27 | 120 |
| TRBV12.3,4,5 | hTRBV12.3,4,5up1 | 26 | 195 |
| TRBV12.1 | hTRBV12.1up1 | 27 | 196 |
| TRBV12.2 | hTRBV12.2up1 | 27 | 196 |
| TRBV13 | hTRBV13up1 | 25 | 356 |
| TRBV14 | hTRBV14up_ex | 24 | 271 |
| TRBV15 | hTRBV15up_ex | 24 | 163 |
| TRBV16 | hTRBV16up1 | 22 | 295 |
| TRBV18 | hTRBV18up1 | 22 | 46 |
| TRBV19 | hTRBV19 up 2 | 24 | 217 |
| TRBV20 | hTRBV20-1up_ex | 24 | 91 |
| TRBV24 | hTRBV24up_ex | 24 | 96 |
| TRBV25 | hTRBV25up_int | 23 | 273 |
| TRBV27 | hTRBV27up2 | 22 | 312 |
| TRBV28 | hTRBV28up_G | 24 | 71 |
| TRBV29 | hTRBV29up_G | 21 | 91 |
| TRBV30 | hTRBV30up1 | 26 | 148 |

**15.** Méthode selon la revendication 1, pour la détection *in vitro* de réarrangements incomplets D-J dans un locus génétique choisi parmi les loci TRB, et IGH.

**16.** Méthode selon la revendication 15, dans laquelle au moins une réaction de PCR multi-n-plexe avec n≥2 est effectuée pour analyser des réarrangements incomplets DJ du locus TRB humain, en utilisant une combinaison d'au moins 3 amorces constituant au moins 2 couples d'amorces présentant les caractéristiques suivantes :

(i) les deux couples d'amorces comportent une amorce sens commune s'hybridant spécifiquement en amont et/ou dans un gène D donné et comportent chacun une amorce antisens s'hybridant spécifiquement en aval et/ou dans un gène J donné ;
(ii) les deux amorces antisens s'hybrident spécifiquement en aval et/ou dans deux gènes $J_y$ et $J_z$ appartenant à deux groupes distincts de gènes J du locus TRB ; et
(iii) la distance entre la région d'hybridation de l'amorce antisens spécifique du gène $J_y$ et le début dudit gène $J_y$ est supérieure à la distance entre la région d'hybridation de l'amorce antisens spécifique du gène $J_z$ et le début du premier gène J du groupe de gènes dudit gène $J_z$.

**17.** Méthode selon la revendication 16, dans laquelle au moins une réaction de PCR multi-n-plexe avec n≥2 est effectuée pour analyser des réarrangements incomplets du locus TRB, en utilisant une combinaison d'au moins 3 amorces comprenant une amorce sens s'hybridant spécifiquement en amont et/ou dans un gène D donné et les amorces hTRBJ1.6 et hTRJB2.7 définies à la revendication 13.

**18.** Méthode selon la revendication 16 et la revendication 17, permettant l'analyse de tous les réarrangements incomplets du locus TRB humain en effectuant (i) une PCR multi-2-plexe à l'aide d'un triplet d'amorces constitué des amorces hTRBJ1.6, hTRJB2.7 et hTRDB1, et (ii) une PCR multiplexe simple à l'aide du couple d'amorces constitué des amorces hTRJB2.7 et hTRDB2, les amorces hTRDB1 et hTRDB2 étant choisies parmi les amorces définies dans le tableau 3 ci-dessous :

Tableau 3

| nom du gène | nom oligonucléotide | taille (nt) | Distance entre l'extrémité 5' de l'amorce et la fin du gène D (pb) |
|---|---|---|---|
| TRBD1 | hTRBD1up1 | 25 | 325 |
| TRBD1 | hTRBD1 up2 | 23 | 289 |
| TRBD2 | hTRBD2up1 | 26 | 322 |
| TRBD2 | hTRBD2up2 | 21 | 290 |

19. Méthode d'analyse de la diversité combinatoire des réarrangements du locus TRB chez un individu, combinant l'analyse des réarrangements V(D)J de ce locus par une méthode selon l'une quelconque des revendications 12 à 14 et l'analyse des réarrangements incomplets de ce locus par une méthode selon l'une quelconque des revendications 16 à 18.

20. Méthode selon l'une quelconque des revendications 1 à 10, permettant l'analyse des réarrangements de 95% des gènes J du locus TRA humain avec un gène V donné du même locus, dans laquelle, à l'étape A), 6 PCR multi-2-plexes ou 3 PCR multi-4-plexes sont effectuées avec des combinaisons d'amorces constituées chacune d'une amorce s'hybridant en amont et/ou dans ledit gène V et d'un ou deux couple(s) d'amorces antisens choisi(s) parmi les couples (hTRAJ56, hTRAJ41), (hTRAJ37, hTRAJ33), (hTRAJ48, hTRAJ29), (hTRAJ24, hTRAJ18), (hTRAJ53, hTRAJ11) et (hTRAJ7, hTRAJ3), lesdites amorces étant définies dans le tableau 4 ci-dessous :

Tableau 4

| nom du gène | nom oligonucléotide | taille (nt) | distance avec le début du gène J en pb |
|---|---|---|---|
| hTRAJ56 | hTRAJ56do | 24 | 883 |
| hTRAJ41 | hTRAJ41 do | 25 | 443 |
| hTRAJ37 | hTRAJ37do | 28 | 351 |
| hTRAJ33 | hTRAJ33do | 24 | 98 |
| hTRAJ48 | hTRAJ48do | 28 | 43 |
| hTRAJ29 | hTRAJ29do | 24 | 300 |
| hTRAJ24 | hTRAJ24do | 28 | 227 |
| hTRAJ18 | hTRAJ18do | 29 | 147 |
| hTRAJ53 | hTRAJ53do | 24 | 200 |
| hTRAJ11 | hTRAJ11do | 20 | 88 |
| hTRAJ7 | hTRAJ7do | 20 | 478 |
| hTRAJ3 | hTRAJ3do | 25 | 329 |

21. Méthode permettant une analyse de la diversité des réarrangements VJ du locus TRA, dans laquelle la méthode selon la revendication 20 est mise en oeuvre avec au moins trois amorces s'hybridant en amont et/ou dans trois gènes V distincts, situés chacun dans une région distincte du locus.

22. Méthode selon la revendication 20 ou la revendication 21, dans laquelle au moins une amorce s'hybridant en amont et/ou dans un gène V du locus TRA est choisie parmi les amorces définies dans le tableau 5 ci-dessous :

Tableau 5

| nom du gène | nom de l'oligonucléotide | taille (pb) | distance avec la fin du gène (pb) |
|---|---|---|---|
| TRAV1 | hTRAV1up ex | 26 | 104 |

(suite)

| nom du gène | nom de l'oligonucléotide | taille (pb) | distance avec la fin du gène (pb) |
|---|---|---|---|
| TRAV2 | hTRAV2up_ex | 24 | 70 |
| TRAV3 | hTRAV3 | 22 | 377 |
| TRAV4 | hTRAV4up ex_testAn1 | 23 | 96 |
| TRAV8 | hTRAVB-2,4,6 up | 22 | 298 |
| TRAV9 | hTRAV9-1 up ex_2/2_testAn2 | 25 | 113 |
| TRAV10 | hTRAV10 up n3 | 24 | 85 |
| TRAV12.2,3 | hTRAV12.2,3up1 | 27 | 114 |
| TRAV12.1 | hTRAV12.1 up 1 | 28 | 112 |
| TRAV14 | hTRAV14 up n2 | 22 | 69 |
| TRAV16 | hTRAV16 up n5 | 27 | 118 |
| TRAV17 | hTRAV17 up n2 | 22 | 40 |
| TRAV19 | hTRAV19 up | 24 | 144 |
| TRAV21 | hTRAV21 up | 24 | 91 |
| TRAV22 | hTRAV22 up | 21 | 42 |
| TRAV23 | hTRAV23 up n2 | 28 | 130 |
| TRAV25 | hTRAV25 up n3 | 27 | 154 |
| TRAV27 | hTRAV27 up | 27 | 138 |
| TRAV29 | hTRAV29 up | 24 | 267 |
| TRAV30 | hTRAV30 up n2 | 24 | 139 |
| TRAV35 | hTRAV35_int up | 27 | 377 |
| TRAV36 | hTRAV36_int up n2 | 27 | 304 |
| TRAV38 | hTRAV38-1,2 up n3 | 24 | 170/169 |
| TRAV40 | hTRAV40 up | 28 | 76 |
| TRAV41 | hTRAV41 int up | 28 | 368 |

**23.** Méthode selon l'une quelconque des revendications 1 à 8 et 11, permettant l'analyse des réarrangements de tous les gènes J du locus TRG humain avec au moins 2 gènes $V_x$ et $V_y$ donnés du même locus, dans laquelle, à l'étape A), au moins une PCR multi-2-plexe est effectuée avec un triplet d'amorces constitué de 2 amorces sens s'hybridant en amont et/ou dans lesdits gènes $V_x$ et $V_y$ et de l'amorce antisens hTRGJdo2 de 26 nucléotides s'hybridant entre les nucléotides 266 et 291 du gène J2 du locus TRG humain.

**24.** Méthode selon la revendication 23, dans laquelle au moins une amorce s'hybridant en amont et/ou dans un gène V du locus TRG humain est choisie parmi les amorces définies dans le tableau 6 ci-dessous :

Tableau 6

| nom du gène | nom oligonucléotide | taille (nt) | distance avec la fin du gène V en pb |
|---|---|---|---|
| TRGV1.2 | hTRGV1.2up1 | 30 | 99 |
| TRGV1.3 | hTRGV1.3up1 | 26 | 79 |
| TRGV1.4 | hTRGV1.4up1 | 28 | 158 |
| TRGV1.5 | hTRGV1.5up1 | 23 | 287 |

(suite)

| nom du gène | nom oligonucléotide | taille (nt) | distance avec la fin du gène V en pb |
|---|---|---|---|
| TRGV1.8 | hTRGV1.8up1 | 26 | 129 |
| TRGV2.9 | hTRGV2.9up1 | 27 | 178 |

25. Méthode selon l'une quelconque des revendications 1 à 10, permettant l'analyse des réarrangements de tous les gènes J du locus TRD humain avec un gène V donné du même locus, dans laquelle, à l'étape A), une PCR multi-2-plexe est effectuée avec un triplet d'amorces constitué d'une amorce s'hybridant en amont et/ou dans ledit gène V et des amorces antisens hTRDJ1do5 et hTRDJ3do2, définies comme suit :

- hTRDJ1do5 est un oligonucléotide antisens de 23 nucléotides s'hybridant entre les nucléotides 90 et 112 du gène J1 du locus TRD ; et
- hTRDJ3do2 est un oligonucléotide antisens de 26 nucléotides s'hybridant entre les nucléotides 448 et 473 du gène J3 du locus TRD.

26. Méthode selon la revendication 25, permettant l'analyse des réarrangements VJ du locus TRD en effectuant 24 PCR multi-n-plexes avec n≥2 à l'aide de combinaisons d'au moins 3 amorces, chaque combinaison d'amorces comprenant les amorces antisens hTRDJ1do5 et hTRDJ3do2 et au moins une amorce hTRDV ou hTRAV choisie parmi les amorces définies dans le tableau 7 ci-dessous :

Tableau 7

| Nom du gène | Nom de l'amorce | Taille (nt) | Distance entre l'extrémité 5' de l'amorce et la fin du gène V (pb) |
|---|---|---|---|
| TRAV9 | hTRAV9-1up ex_2/2_testAn2 | 25 | 113 |
| TRAV12.2,3 | hTRAV12.2,3up1 | 27 | 114 |
| TRAV12.1 | hTRAV12.1up1 | 28 | 112 |
| TRAV14 (TRDV4) | hTRAV14 up n2 | 22 | 69 |
| TRAV16 | hTRAV16 up n5 | 27 | 118 |
| TRAV17 | hTRAV17 up n2 | 22 | 40 |
| TRAV19 | hTRAV19 up | 24 | 144 |
| TRAV21 | hTRAV21 up | 24 | 91 |
| TRAV22 | hTRAV22up2 | 21 | 232 |
| TRAV23 (TRDV6) | hTRAV23 up n2 | 28 | 130 |
| TRAV25 | hTRAV25 up n3 | 27 | 154 |
| TRAV27 | hTRAV27 up | 27 | 138 |
| TRAV29 (TRDV5) | hTRAV29 up | 24 | 267 |
| TRAV30 | hTRAV30 up n2 | 24 | 139 |
| TRAV35 | hTRAV35_int up | 27 | 377 |
| TRAV36 (TRDV7) | hTRAV36up1 | 26 | 280 |
| TRAV38 (TRDV8) | hTRAV38-1,2 up n3 | 24 | 170/169 |
| TRAV39 | hTRAV39up1 | 26 | 352 |
| TRAV40 | hTRAV40 up | 28 | 76 |
| TRAV41 | hTRAV41_int up | 28 | 368 |
| TRDV1 | hTRDV1up1 | 25 | 259 |

(suite)

| Nom du gène | Nom de l'amorce | Taille (nt) | Distance entre l'extrémité 5' de l'amorce et la fin du gène V (pb) |
|---|---|---|---|
| **TRDV2** | hTRDV2up1 | 24 | 260 |
| **TRDV3** | hTRDV3up1 | 24 | 287 |

**27.** Méthode selon l'une quelconque des revendications 1 à 8 et 11, permettant l'analyse des réarrangements de tous les gènes J du locus IgH humain avec au moins 2 gènes $V_x$ et $V_y$ donnés du même locus, dans laquelle, à l'étape A), au moins une PCR multi-2-plexe est effectuée avec un triplet d'amorces constitué de 2 amorces sens s'hybridant en amont et/ou dans lesdits gènes $V_x$ et $V_y$ et d'une amorce antisens s'hybridant en aval et/ou dans le gène IgHJ6.

**28.** Méthode selon la revendication 27, dans laquelle l'amorce antisens est l'amorce hIgHJ6do2 s'hybridant entre la 368$^{ième}$ et la 392$^{ième}$ base à compter du début du gène IgHJ6.

**29.** Méthode selon la revendication 27 ou la revendication 28, dans laquelle au moins une amorce s'hybridant en amont et/ou dans un gène V du locus IgH humain est choisie parmi les amorces définies dans le tableau 8 ci-dessous :

Tableau 8

| nom du gène | nom oligonucléotide | taille (nt) | distance avec la fin du gène V en pb |
|---|---|---|---|
| IGHV1.2, 8, 18, 46, 69 | hIGHV1Aup1 | 25 | 172 |
| IGHV1.3, 8, 24, 45, 58, 69, f | hIGHV1Bup2 | 22 | 62 |
| IGHV2 | hIGHV2up1 | 22 | 44 |
| IGHV3.7,13,15,20,21,23, 48, 53, 64, 66, 72, 73, 74 | hIGHV3Aup1 | 24 | 315 de V3.7 |
| IGHV3.7, 9, 11, 13, 15, 20, 21, 30, 33, 43, 48, 49, 72 | hIGHV3Bup1 | 21 | 288 de V3.7 |
| IGHV4 | hIGHV4up1 | 25 | 69 |
| IGHV5 | hIGHV5up1 | 21 | 55 |
| IGHV6 | hIGHV6up1 | 23 | 371 |

**30.** Méthode selon la revendication 15, dans laquelle au moins une réaction de PCR multi-n-plexe avec n≥2 est effectuée pour analyser certains réarrangements incomplets du locus IgH humain, en utilisant une combinaison d'au moins 3 amorces constituant au moins 2 couples d'amorces comportant une amorce antisens commune s'hybridant spécifiquement en aval et/ou dans un gène J donné et comportant chacun une amorce sens s'hybridant spécifiquement en amont et/ou dans un gène D donné.

**31.** Méthode selon la revendication 30, dans laquelle l'amorce antisens commune est l'amorce hIgHJ6do2 définie à la revendication 28.

**32.** Méthode selon la revendication 30 ou la revendication 31, dans laquelle au moins une amorce s'hybridant en amont et/ou dans un gène D du locus IgH humain est choisie parmi les amorces définies dans le tableau 9 ci-dessous :

Tableau 9

| nom du gène | nom oligonucléotide | taille (pb) | Distance entre l'extrémité 5' de l'amorce et la fin du gène D (pb) |
|---|---|---|---|
| hIGHD1 | hIGHD1up1 | 23 | 44 |
| hIGHD2 | hIGHD2up1 | 22 | 67 |
| hIGHD3 | hIGHD3up1 | 20 | 102 |

(suite)

| nom du gène | nom oligonucléotide | taille (pb) | Distance entre l'extrémité 5' de l'amorce et la fin du gène D (pb) |
|---|---|---|---|
| hIGHD4 | hIGHD4up 1 | 19 | 132 |
| hIGHD5 | hIGHD5up2 | 20 | 85 |
| hIGHD6 | hIGHD6up1 | 21 | 160 |
| hIGHD7 | hIGHD7up2 | 20 | 90 |

33. Méthode d'analyse de la diversité combinatoire des réarrangements du locus IgH chez un individu, combinant l'analyse des réarrangements V(D)J de ce locus par une méthode selon l'une quelconque des revendications 27 à 29 et l'analyse des réarrangements incomplets de ce locus par une méthode selon l'une quelconque des revendications 30 à 32.

34. Méthode selon l'une quelconque des revendications précédentes, pour analyser la diversité combinatoire des réarrangements V(D)J d'au moins deux locus génétiques choisis parmi les loci TRA, TRB, TRG, TRD, IGH, IGK et IGL.

35. Méthode selon la revendication 34, pour analyser la diversité combinatoire des réarrangements V(D)J du locus TRB et la diversité combinatoire des réarrangements VJ du locus TRG ou du locus TRD.

36. Méthode selon la revendication 34 ou la revendication 35, pour analyser en outre la diversité combinatoire des réarrangements V(D)J du locus IgH.

37. Méthode selon l'une quelconque des revendications précédentes, dans laquelle les réactions de PCR multi-n-plexes sont effectuées avec une polymérase présentant les caractéristiques suivantes :

(i) elle est capable d'amplifier des fragments de plusieurs dizaines de kb ;
(ii) sa vitesse d'élongation est d'au moins 1 kb/minute ;
(iii) sa robustesse est telle qu'elle n'introduit pas plus d'une erreur par kb, en moyenne.

38. Méthode selon l'une quelconque des revendications 7 à 36, dans laquelle l'étape C) comporte une étape de traitement des données obtenues par la séparation des amplicons suivant leur taille, ledit traitement étant effectué par un ordinateur et permettant d'attribuer à chaque amplicon observé le nom du réarrangement V(D)J correspondant.

39. Méthode selon la revendication 38, dans laquelle le traitement des données intègre également l'intensité du signal de chacun des amplicons observés, pour quantifier la fréquence relative du réarrangement V(D)J correspondant.

40. Méthode selon la revendication 38 ou la revendication 39, dans laquelle l'étape B) comprend l'acquisition des données concernant la taille des amplicons et pour chacun, l'intensité du signal, et l'étape C) comprend les étapes suivantes :

(i) identification de chaque amplicon, en déterminant à quel réarrangement V(D)J il correspond, en fonction de sa taille ;
(ii) à partir de l'intensité du signal de chaque amplicon, détermination de la proportion de l'ADN génomique de départ présentant le réarrangement V(D)J correspondant ;
(iii) présentation des résultats sous forme d'un graphique tridimensionnel présentant les gènes ou les familles de gènes $V_x$ suivant un axe, les gènes $J_y$ suivant un autre axe, et la fréquence des réarrangements $V_xJ_y$ suivant le troisième axe.

41. Méthode pour déterminer *in vitro* le niveau d'immunodéficience d'un individu, comportant les étapes suivantes :

A) à partir d'un échantillon biologique dudit individu, faire une numération lymphocytaire ;
B) à partir du même échantillon ou d'un autre échantillon provenant du même individu au même moment, déterminer le niveau de diversité combinatoire du répertoire des lymphocytes dudit individu, par la mise en oeuvre d'une méthode selon l'une quelconque des revendications précédentes ;
C) combiner les données obtenues aux étapes A) et B).

**42.** Méthode selon la revendication 41, comportant en outre une étape d'interprétation de la combinaison obtenue à l'étape C), au regard d'un graphique attribuant un niveau de risque au moins aux 4 zones suivantes :

(i) numération faible (<1000 Ly/μL) et diversité combinatoire V-J faible (<40%) : risque infectieux élevé ;
(ii) numération faible (<1000 Ly/μL) mais diversité combinatoire V-J normale (>65%) : risque infectieux faible ;
(iii) numération normale (1000-3200 Ly/μL) et diversité combinatoire V-J faible (<40%) : risque infectieux moyen ;
(iv) numération normale (1000-3200 Ly/μL) et diversité combinatoire V-J normale (>65%) : le répertoire immunitaire est sain.

**43.** Méthode selon la revendication 42, dans laquelle le graphique comprend en outre les deux zones suivantes :

(v) numération au dessus de la normale (> 3200Ly/μL), et diversité combinatoire V-J faible (<40%) : risque lymphoproliphératif élevé
(vi) numération au dessus de la normale (> 3200Ly/μL), et diversité combinatoire V-J normale (>65%) : risque lymphoprolifératif moyen.

**44.** Méthode selon l'une quelconque des revendications 41 à 43 dans laquelle l'étape B) comprend la détermination du niveau de diversité combinatoire du répertoire des lymphocytes T et des lymphocytes B dudit individu.

**45.** Méthode selon la revendication 44 dans laquelle, à l'étape C), les données sont examinées à l'aide d'un graphique tridimensionnel présentant le niveau de diversité des immunoglobulines sur un axe, le niveau de diversité des TCR sur un autre axe, et la numération lymphocytaire sur un troisième axe.

**46.** Méthode de suivi de l'évolution de la diversité du répertoire des lymphocytes T et/ou B d'un individu, comportant les étapes suivantes :

A) Mesure de la diversité du répertoire des lymphocytes dudit individu, par la mise en oeuvre d'une méthode selon l'une quelconque des revendications 38 à 45, à partir de deux échantillons dudit individu prélevés à deux dates différentes ;
B) comparer les deux échantillons en évaluant :

(i) le nombre S de réarrangements observés dans les deux échantillons ;
(ii) le nombre A de réarrangements observés dans l'échantillon le plus récent mais pas dans le plus ancien ;
(iii) le nombre D de réarrangements observés dans l'échantillon le plus ancien mais pas dans le plus récent ;
(iv) le nombre Z de réarrangements qui ne sont observés dans aucun des échantillons.

**47.** Trousse pour la mise en oeuvre d'une méthode selon l'une quelconque des revendications précédentes, comprenant une combinaison d'amorces telle que définie à l'une quelconque des revendications précédentes, et des réactifs pour effectuer des PCR.

**48.** Trousse selon la revendication 47, comprenant une polymérase présentant les caractéristiques suivantes :

(i) elle est capable d'amplifier des fragments de plusieurs dizaines de kb ;
(ii) sa vitesse d'élongation est d'au moins 1 kb/minute ;
(iii) sa robustesse est telle qu'elle n'introduit pas plus d'une erreur par kb, en moyenne.

**49.** Trousse selon la revendication 47 ou la revendication 48, comprenant une plaque multipuits dans laquelle chaque puit comprend une combinaison différente d'amorces.

**50.** Trousse selon la revendication 49, dans laquelle une plaque multipuits comprend toutes les combinaisons d'amorces nécessaires à l'amplification d'au moins 50% des réarrangements V-J d'au moins un locus choisi parmi les loci TRA, TRB, TRG, TRD et IGH.

**51.** Utilisation d'une méthode ou d'une trousse selon l'une quelconque des revendications précédentes, pour étudier la mise en place et la qualité du répertoire TCR et/ou IgH d'un animal transgénique humanisé et/ou d'une culture de lymphocytes

**52.** Utilisation d'une méthode ou d'une trousse selon l'une quelconque des revendications 1 à 48, pour cribler des molécules thérapeutiques in *vitro.*

**Figure 1a**

**Figure 1b**

## Pourcentage de GC des loci TRA et B

**Figure 2a**

**Figure 2b**

**Figure 2c**

**Figure 2d**

Figure 3

**Figure 4a**

**Figure 4b**

**Figure 4c**

hTRAJ5    J18          J5 + J18
200       500          200   500

size
(bp)

8990.
8065.
7406.
6668.
4877.
2907.
1830.

1114.

471.

M1        M2          M3= M1+M2

# Figure 4d

J gene  56 +  41    37 +  33    48 + 29     24 + 18     53 +  11    7 +  3
       (883) (443)  (351) (98)  (43) (300)  (227) (147) (200) (88)  (478) (329)

> 95% of TRAJ repertoire

# Figure 4e

Figure 5

**Thymus (90% répertoire total) (duplicate)**

**Lymphopenia (duplicate)**

**Pool de 4 lignées JURKAT, MOLT4, HUT et SUP : caractérisation des réarrangements TRBV-J (simplicat)**

SUP: hTRBV9-J2.1

Jurkat: hTRBV12.3-J1.2  HUT: hTRBV13-J1.1/1.2

Molt4: hTRBV10-J2.4

Molt4: hTRBV20-J2.1

Figure 6

Figure 7

A-

B-

| Intensité | Valeur | % / Ref |
|---|---|---|
| Intensité Totale (U.A) | 15453,46 | 19,90% |
| Intensité hTRBD1,2 | 2494,39 | |

**Diversité**

| | |
|---|---|
| Nbre de réarrangements théoriques | 276 |
| Nbre de réarrangements observés | 120 |
| Nbre réarrangements avec hTRBD1,2 | 19 |
| Diversité / Théo | 43,48% |
| Diversité / Ref | 47,06% |

Lymphopénie — Non — Diversité Théorique < 40 %

Prolifération(s) Clonale(s) (en rouge dans information réarrangements)
Nombre — 0

Délétion(s) potentielle(s) (en rouge dans informations gènes)
Nombre — 2

**Homogénéité**

CV Inhomogénéité — 1,88

Int moyenne / Réarr observé — 128,78

Répertoire du patient

Rearrangement frequency

400,00
300,00
200,00
100,00
0,00

TRBJ

J2.7
J2.6
J2.5
J2.4
J2.3
J2.2
J2.1
J1.6
J1.5
J1.4
J1.3
J1.1+J1.2

TRBV

**Figure 8a**

EP 2 062 982 A1

**Répertoire patient (Surface)**

| Ordre | Nom | Valeur | Contrib / Int totale (en %) |
|---|---|---|---|
| 1 | J1.1+J1.2-V24 | 651,54 | 4,22% |
| 2 | J1.4-V30 | 651,54 | 4,22% |
| 3 | J1.1+J1.2-V29 | 565,31 | 3,66% |
| 4 | J1.1+J1.2-V28 | 464,02 | 3,00% |
| 5 | J1.6-V28 | 439,38 | 2,84% |
| 6 | J2.5-V20 | 427,06 | 2,76% |
| 7 | J1.5-V4 | 424,04 | 2,74% |
| 8 | J1.1+J1.2-V27 | 353,14 | 2,29% |
| 9 | J1.3-V30 | 320,29 | 2,07% |
| 10 | J1.1+J1.2-V6 | 302,88 | 1,96% |
| 11 | J1.1+J1.2-V30 | 288,81 | 1,87% |

**Figure 8b**

**Figure 9**

**Figure 10**

Figure 11

**ImmunTraCkeR trousse hTRB sur Thymus**

**Pool 3 lignées :**

# Figure 12a

**Figure 12b**

Figure 12c

Figure 13

Figure 14

Figure 15

Signal intentisy

treatment

time

Lymphocyte numeration

treatment

time

Diversity

treatment

time

····· Average rearrangement
—— Selected rearrangement

**Figure 16**

Human PBMC repertoire

Good reconstitution of human repertoire in humanized mouse thymus

Partial reconstitution of human repertoire in humanized mouse thymus

**Figure 17**

Sens de sélection immunitaire

Numeration

B    C

A

Diversité

## Figure 18

**Etude comparative entre différentes techniques d'immunomonitoring des répertoires immunitaires**

| | Thymus | Thymus | Jurkat (lignée monoclonal) | Pool Lignées T | T(-) |
|---|---|---|---|---|---|
| **Iotests** Donne la diversité Vb. Le test n'est pas exhaustif et ne permet de montrer que 72% des familles et 59% des membres V. | | | V12 | V2    V23 | |
| **ImmunTraCkeR** Caractérise les familles V correspondantes à une lymphoprolifération | | | | | |
| Donne la diversité V-J. ne donne pas la diversité de jonction. Identifie les clones sans besoin de séquencage. | Diversité V-J = 80% | Diversité V-J = 80% | V12-J1.2 Diversité V-J = 0,5% | V2-J1.2  V23-J2.1 V4-J2.3 Diversité V-J = 1,5% | |
| **Biomed-2** Seulement ON/OFF. ne permet pas de mesurer une diversité, ni d'assurer qu'il y a une expansion oligoclonale. | OFF | OFF | ON | ON | |

## Figure 19

ADNg 50ng/µL
**Bonne Qualité**

PCR Mix
**1**

Migration électrophorèse
**2**

Coloration
**3**

Acquisition d'images
**4**

Analyse d'images
**5**

# Figure 20

Expansion clonale

**Figure 21**

## DOCUMENTS CONSIDERES COMME PERTINENTS

| Office européen des brevets | **RAPPORT PARTIEL DE RECHERCHE EUROPEENNE** qui selon la règle 63 de la Convention sur le brevet européen est consideré, aux fins de la procédure ultérieure, comme le rapport de la recherche européenne | Numéro de la demande EP 07 29 1401 |

| Catégorie | Citation du document avec indication, en cas de besoin, des parties pertinentes | Revendication concernée | CLASSEMENT DE LA DEMANDE (IPC) |
|---|---|---|---|
| X | DONGEN VAN J J M ET AL: "DESIGN AND STANDARDIZATION OF PCR PRIMERS AND PROTOCOLS FOR DETECTION OF CLONAL IMMUNOGLOBULIN AND T-CELL RECEPTOR GENE RECOMBINATIONS IN SUSPECT LYMPHOPROLIFERATIONS: REPORT OF THE BIOMED-2 CONCERTED ACTION BMH4-CT98-3936" LEUKEMIA, MACMILLAN PRESS LTD, US, vol. 17, 2003, pages 2257-2317, XP008062303 ISSN: 0887-6924 * abrégé * * figures 4,6-10 * *Sections 1-7* | 1-3, 7-12,15, 16,19, 27,30, 33-37, 47,48 | INV. C12Q1/68 |
| Y | * page 2264, colonne 2 - page 2265, colonne 1 * ----- -/-- | 4-6 | |

DOMAINES TECHNIQUES RECHERCHES (IPC)

C12Q

## RECHERCHE INCOMPLETE

La division de la recherche estime que la présente demande de brevet, ou une ou plusieurs revendications, ne sont pas conformes aux dispositions de la CBE au point qu'une recherche significative sur l'état de la technique ne peut être effectuée, ou seulement partiellement, au regard de ces revendications.

Revendications ayant fait l'objet d'une recherche complète:

Revendications ayant fait l'objet d'une recherche incomplète:

Revendications n'ayant pas fait l'objet d'une recherche:

Raison pour la limitation de la recherche:

voir feuille supplémentaire C

| Lieu de la recherche | Date d'achèvement de la recherche | Examinateur |
|---|---|---|
| Munich | 18 avril 2008 | Schmitt-Humbert, C |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
................................................................
& : membre de la même famille, document correspondant

EPO FORM 1503 03.82 (P04E08)

**Office européen**
**des brevets**

Numero de la demande

EP 07 29 1401

**RAPPORT PARTIEL**
**DE RECHERCHE EUROPEENNE**

| | DOCUMENTS CONSIDERES COMME PERTINENTS | | CLASSEMENT DE LA DEMANDE (IPC) |
|---|---|---|---|
| Catégorie | Citation du document avec indication, en cas de besoin, des parties pertinentes | Revendication concernée | |
| D,X | WO 2005/056828 A (COMMISSARIAT ENERGIE ATOMIQUE [FR]; INSERM INST NAT DE LA SANTE ET [FR) 23 juin 2005 (2005-06-23) * revendications 1,2,5,8,9,14,19 * * page 13, ligne 20 - ligne 30 * * page 14, alinéa 2 - alinéa 3 * * page 24, ligne 19 - page 25, ligne 21 * * page 30, ligne 18 - ligne 25 * | 1-3,7,8, 37-39, 41,44-52 | |
| Y | * page 32, ligne 14 - ligne 29 * | 4-6 | |
| | ----- | | |
| X | WO 2004/033728 A (UNIV ERASMUS [NL]; VAN DONGEN JACOBUS JOHANNES MA [NL]; LANGERAK ANTHO) 22 avril 2004 (2004-04-22) | 1-3, 7-12,15, 16,19, 27,30, 33-37, 41,44, 45,47,48 | DOMAINES TECHNIQUES RECHERCHES (IPC) |
| | * figures 1-10 * * exemples 1-8 * | | |
| Y | * revendications 1-42 * | 4-6 | |
| | ----- | | |
| Y | PASQUAL N ET AL: "Quantitative and qualitative changes in V-J alpha rearrangements during mouse thymocytes differentiation: implication for a limited T cell receptor alpha chain repertoire" JOURNAL OF EXPERIMENTAL MEDICINE, TOKYO, JP, vol. 196, no. 9, 4 novembre 2002 (2002-11-04), pages 1163-1173, XP002322207 ISSN: 0022-1007 * abrégé * *Matériels et méthodes* ----- | 4-6 | |

EPO FORM 1503 03.82 (P04C11)

**Office européen**
**des brevets**

**RECHERCHE INCOMPLETE**
**FEUILLE SUPPLEMENTAIRE C**

Numéro de la demande

EP 07 29 1401

Revendications ayant fait l'objet de recherches incomplètes:
19,33-52

Revendications n'ayant pas fait l'objet de recherches:
13,14,17,18,20-26,28,29,31,32

Raison pour la limitation de la recherche:

1) La revendication 13 concerne une méthode d'analyse in vitro de la diversité du répertoire des lymphocytes T et/ou B d'un individu. Ladite méthode est basée sur l'utilisation d'une PCR multi-N-plexe avec n >= 2 effectuée avec une combinaison d'au moins trois amorces comprenant une amorce sens s'hybridant spécifiquement en amont et/ou dans un gène V donné et les amorces hTRBJ1.6 et hTRJB2.7. Les amorces dénommées hTRBJ1.6 et hTRJB2.7 sont definies par leur taille et leur position dans les gènes J1.6 et J2.7 du locus TRB.
Cette manière de definir les dites amorces ne permet pas de clairement déduire les caractéristiques structurelles, c'est-à-dire la séquence nucléotidique, desdites amorces hTRBJ1.6 et hTRJB2.7 car les séquences des gènes J1.6 et J2.7 du locus TRB ne sont pas décrites de manière claire et non ambiguë dans la demande. De plus, la présente demande utilise des paramètres non usuels pour définir lesdites amorces. D'après la description (page 31, lignes 10-22), la position d'une amorce spécifique d'un gène J est donnée en indiquant sa distance par rapport au début du gène J, c'est-à-dire la 1ère base du segment de gène J (séquence codante), après le RSS. Cette position se situe en aval du début du gène J et correspond à la distance entre la 1ère base du gène J et la première base à l'extrémité 5' de l'amorce. Cependant aucune séquence correspondant aux gènes J1.6 et J2.7 du locus TRB n'est donnée dans la demande et les séquences des amorces J1.6 et J2.7 du locus TRB ne peut donc être déduite de manière claire et non ambiguë. Par ailleurs, la nomenclature des gènes V(D)J utilisée dans la demande est la nomenclature IMGT accessible via l'adresse http://imgt.cines.fr. (cf. page 5, lignes 13-26). De cette banque de données, considérée comme étant une banque de données non usuelle, les séquences des gènes J1.6 et J2.7 du locus TRB ne peuvent être retrouvée de manière claire et non ambiguë. De plus, la demande indique page 31, ligne 23-page 32, ligne 31 de la déscription que les séquences des loci du TCR et IG peuvent être récupérées de la base de données Ensembl (accessible via l'adresse http://www.ensembl.org) en donnant pour chaque locus TCR ou IG humain considéréré les régions chromosomiques correspondantes sans aucune référence à un numéro d'accession. La banque de donnée Ensembl est considérée comme étant une banque de données usuelle, cependant la demande manque de décrire le lien entre les deux banques de données Ensembl et IMGT. L'homme du métier ne peut donc déduire de manière claire et non ambiguë les séquences des amorces hTRBJ1.6 et hTRJB2 étant donné que lesdites séquences ne peuvent pas, clairement et de manière sûre, être déterminées suivant les indications données dans la description ou les méthodes objectives couramment utilisées dans le domaine. La revendication 13 ne définit pas clairement quels sont les amorces qu'elle couvre. Ceci rend une comparaison de la revendication 13 avec l'état de la technique impossible. Il en résulte que la demande ne suffit pas à l'exigence de clarté énoncée à l'article 84 CBE.
Le défaut de clarté est tel qu'une recherche significative portant sur

**Office européen**

**des brevets**

**RECHERCHE INCOMPLETE**
**FEUILLE SUPPLEMENTAIRE C**

Numéro de la demande

EP 07 29 1401

l'ensemble de l'objet revendiqué dans la revendication 13 n'a pas pu être effectuée (règle 63 CBE).

2) Les objections soulevées ci-dessus s'appliquent mutatis mutandis aux revendications 14,17,18,20-26,28,29,31,32. Aucune recherche siginificative n'a pu être effectué pour lesdites revendications.

3) La recherche des revendications 19 et 33-52 a été limitée en conséquence.

**ANNEXE AU RAPPORT DE RECHERCHE EUROPEENNE
RELATIF A LA DEMANDE DE BREVET EUROPEEN NO.**

EP 07 29 1401

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

18-04-2008

| Document brevet cité au rapport de recherche | Date de publication | Membre(s) de la famille de brevet(s) | Date de publication |
|---|---|---|---|
| WO 2005056828    A | 23-06-2005 | CA      2548053 A1<br>EP      1704249 A1<br>FR      2863274 A1<br>JP   2007515955 T | 23-06-2005<br>27-09-2006<br>10-06-2005<br>21-06-2007 |
| WO 2004033728    A | 22-04-2004 | AU   2003300565 A1<br>CA      2501863 A1<br>CN      1965089 A<br>EP      1549764 A2<br>JP   2006501842 T | 04-05-2004<br>22-04-2004<br>16-05-2007<br>06-07-2005<br>19-01-2006 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5296351 A
- US 5418134 A

- WO 2005056828 A

**Littérature non-brevet citée dans la description**

- **AUDE-GARCIA, C. ; GALLAGHER, M. ; MARCHE, P. N. ; JOUVIN-MARCHE, E.** Preferential ADV-AJ association during recombination in the mouse T-cell receptor alpha/delta locus. *Immunogenetics,* 2001, vol. 52, 224-230
- **BAUM, P. D. ; MCCUNE, J. M.** Direct measurement of T-cell receptor repertoire diversity with AmpliCot. *Nat Methods,* 2006, vol. 3, 895-901
- **BAUM, T. P. ; HIERLE, V. ; PASQUAL, N. ; BEL-LAHCENE, F. ; CHAUME, D. ; LEFRANC, M. P. ; JOUVIN-MARCHE, E. ; MARCHE, P. N. ; DEMON-GEOT, J.** IMGT/GeneInfo: T cell receptor gamma TRG and delta TRD genes in database give access to all TR potential V(D)J recombinations. *BMC Bioinformatics,* 2006, vol. 7, 224
- **BAUM, T. P. ; PASQUAL, N. ; THUDEROZ, F. ; HIERLE, V. ; CHAUME, D. ; LEFRANC, M. P. ; JOUVIN-MARCHE, E. ; MARCHE, P. N. ; DEMONGE-OT, J.** IMGT/GeneInfo: enhancing V(D)J recombination database accessibility. *Nucleic Acids Res,* 2004, vol. 32, D51-54
- **BEREK, C. ; GRIFFITHS, G. M. ; MILSTEIN, C.** Molecular events during maturation of the immune response to oxazolone. *Nature,* 1985, vol. 316, 412-418
- **BOGUE, M. ; GILFILLAN, S. ; BENOIST, C. ; MA-THIS, D.** Regulation of N-region diversity in antigen receptors through thymocyte differentiation and thymus ontogeny. *Proc Natl Acad Sci U S A,* 1992, vol. 89, 11011-11015
- **BONARIUS, H. P. ; BAAS, F. ; REMMERSWAAL, E. B. ; VAN LIER, R. A. ; TEN BERGE, 1. J. ; TAK, P. P. ; DE VRIES, N.** Monitoring the T-cell receptor repertoire at single-clone resolution. *PLoS ONE,* 2006, vol. 1, e55
- **CABANIOLS, J. P. ; FAZILLEAU, N. ; CAS-ROUGE, A. ; KOURILSKY, P. ; KANELLOPOU-LOS, J. M.** Most alpha/beta T cell receptor diversity is due to ''terminal deoxynucleotidyl transferase. *J Exp Med,* 2001, vol. 194, 1385-1390

- **CHAUDHURI, J. ; TIAN, M., KHUONG, C. ; CHUA, K. ; PINAUD, E. ; ALT, F. W.** Transcription-targeted DNA deamination by the AID antibody diversification enzyme. *Nature,* 2003, vol. 422, 726-730
- **COCHET, M. ; PANNETIER, C. ; REGNAULT, A. ; DARCHE, S. ; LECLERC, C. ; KOURILSKY, P.** Molecular détection and in vivo analysis of the specific T cell response to a protein antigen. *Eur J Immunol,* 1992, vol. 22, 2639-2647
- **DAVIS, M. M. ; BJORKMAN, P. J.** T-cell antigen receptor genes and T-cell recognition. *Nature,* 1988, vol. 334, 395-402
- **DOUEK, D. C. ; MCFARLAND, R. D. ; KEISER, P. H. ; GAGE, E. A. ; MASSEY, J. M. ; HAYNES, B. F. ; POLIS, M. A. ; HAASE, A. T. ; FEINBERG, M. B. ; SULLIVAN, J. L. et al.** Changes in thymic function with age and during the treatment of HIV infection. *Nature,* 1998, vol. 396, 690-695
- **FUGMANN, S. D. ; LEE, A. I. ; SHOCKETT, P. E. ; VILLEY, I. J. ; SCHATZ, D. G.** The RAG proteins and V(D)J recombination: complexes, ends, and transposition. *Annu Rev Immunol,* 2000, vol. 18, 495-527
- **FUSCHIOTTI, P. ; PASQUAL, N ; HIERLE, V. ; BOREL, E. ; LONDON, J. ; MARCHE, P. N. ; JOU-VIN-MARCHE, E.** Analysis of the TCR alpha-chain rearrangement profile in human T lymphocytes. *Mol Immunol,* 2007, vol. 44, 3380-3388
- **HAMBLIN, T. J. ; DAVIS, Z. ; GARDINER, A. ; OS-CIER, D. G. ; STEVENSON, F. K.** Unmutated Ig V(H) genes are associated with a more aggressive form of chronic lymphocytic leukemia. *Blood,* 1999, vol. 94, 1848-1854
- **HUANG, C. ; KANAGAWA, O.** Ordered and coordinated rearrangement of the TCR alpha locus: role of secondary rearrangement in thymic selection. *J Immunol,* 2001, vol. 166, 2597-2601
- **JOUVIN-MARCHE, E. ; AUDE-GARCIA, C. ; CAN-DEIAS, S. ; BOREL, E. ; HACHEMI-RACHEDI, S. ; GAHERY-SEGARD, H. ; CAZENAVE, P. A. ; MARCHE, P. N.** Differential chronology of TCRADV2 gene use by alpha and delta chains of the mouse TCR. *Eur J Immunol,* 1998, vol. 28, 818-827

- **KOTANI, A. ; OKAZAKI, I. M. ; MURAMATSU, M. ; KINOSHITA, K. ; BEGUM, N. A. ; NAKAJIMA, T. ; SAITO, H. ; HONJO, T.** A target selection of somatic hypermutations is regulated similarly between T and B cells upon activation-induced cytidine deaminase expression. *Proc Natl Acad Sci U S A,* 2005, vol. 102, 4506-4511
- **LANG, R. ; PFEFFER, K. ; WAGNER, H. ; HEEG, K.** A rapid method for semiquantitative analysis of the human V beta-repertoire using TaqManR. *PCR. J Immunol Methods,* 1997, vol. 203, 181-192
- **LEFRANCS.** The Immunoglobulin Facts Book. 2001
- **LEFRANCS.** The T cell receptor Facts Book. 2001
- **OPREA, M. ; KEPLER, T. B.** Genetic plasticity of V genes under somatic hypermutation: statistical analyses using a new resampling-based methodology. *Genome Res,* 1999, vol. 9, 1294-1304
- **PANNETIER, C. ; EVEN, J. ; KOURILSKY, P.** T-cell repertoire diversity and clonal expansions in normal and clinical samples. *Immunol Today,* 1995, vol. 16, 176-181
- **PASQUAL, N. ; GALLAGHER, M. ; AUDE-GARCIA, C. ; LOIODICE, M. ; THUDEROZ, F. ; DEMONGEOT, J. ; CEREDIG, R. ; MARCHE, P. N. ; JOUVIN-MARCHE, E.** Quantitative and qualitative changes in V-J alpha rearrangements during mouse thymocytes differentiation: implication for a limited T cell receptor alpha chain repertoire. *J Exp Med,* 2002, vol. 196, 1163-1173

- **PHAM, T. ; BELZER, M. ; CHURCH, J. A. ; KITCHEN, C. ; WILSON, C. M. ; DOUGLAS, S. D. ; GENG, Y. ; SILVA, M. ; MITCHELL, R. M. ; KROGSTAD, P.** Assessment of thymic activity in human immunodeficiency virus-negative and - positive adolescents by real-time PCR quantitation of T-cell receptor rearrangement excision circles. *Clin Diagn Lab Immunol,* 2003, vol. 10, 323-328
- **RYTKONEN, M. A. ; HURWITZ, J. L. ; THOMPSON, S. D. ; PELKONEN, J.** Restricted onset of T cell receptor alpha gene rearrangement in fetal and neonatal thymocytes. *Eur J Immunol,* 1996, vol. 26, 1892-1896
- **VAN DEN BEEMD ; VAN DONGEN et al.** Flow cytometric detection of clonality in mature T-cell malignancies by use of a Vb antibody kit. *ISAC Abstract,* 2000
- **WANG, F. ; HUANG, C. Y. ; KANAGAWA, O.** Rapid deletion of rearranged T cell antigen receptor (TCR) Valpha-Jalpha segment by secondary rearrangement in the thymus: role of continuous rearrangement of TCR alpha chain gene and positive selection in the T cell repertoire formation. *Proc Natl Acad Sci U S A,* 1998, vol. 95, 11834-11839